# EUROPEAN PATENT APPLICATION

(11) **EP 4 714 952 A1**
(43) Date of publication of application: **25.03.2026**
(21) Application number: 24830731.6
(22) Date of filing: 25.06.2024
(51) Int. Cl.: C07D 471/04, C07D 498/04, A61K 31/505, A61K 31/553, A61P 11/00

(54) **COMPOUND AND USE THEREOF**

(30) Priority: 28.06.2023 CN 202310775924
(71) Applicant: Autobio Group Co., Ltd., Zhengzhou, Henan 450016 (CN); Demai Pharmaceutical Co., Ltd., Zhengzhou, Henan 451162 (CN)
(72) Inventor: WANG, Zhanguo, Zhengzhou, Henan 450016 (CN); ZHANG, Yun, Zhengzhou, Henan 450016 (CN); ZHANG, Shaoning, Zhengzhou, Henan 450016 (CN); HAN, Hongtao, Zhengzhou, Henan 450016 (CN); ZHANG, Xingshuang, Zhengzhou, Henan 450016 (CN); MENG, Qingle, Zhengzhou, Henan 450016 (CN)
(74) Representative: V.O.
(86) International application number: PCT/CN2024/101164
(87) International publication number: WO 2025/002089

(57) **Abstract**

Disclosed in the present invention are a compound and the use thereof, which belong to the field of medicinal chemistry. Further disclosed is the compound having a structure as shown in formula (I), or a deuterated compound thereof, or a stereoisomer thereof, or a pharmaceutically acceptable salt thereof. The compound has a good PDE3 and/or PDE4 inhibitory effect, and can be used for treating PDE3 and/or PDE4-related conditions.

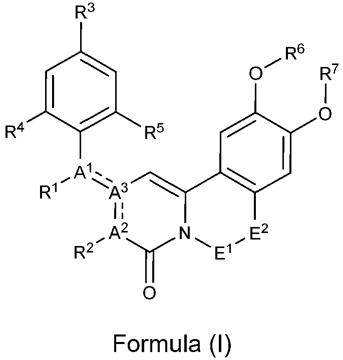

## Description

This application claims the priority of Chinese Patent Application No. 202310775924.8, filed with the China National Intellectual Property Administration on June 28, 2023, and titled with "COMPOUND AND USE THEREOF", which is hereby incorporated by reference in its entirety.

### FIELD

The present disclosure relates to the field of pharmaceutical chemistry, in particular to a compound and use thereof.

### BACKGROUND

Phosphodiesterases (PDEs) are hydrolases that hydrolyze cyclic adenosine monophosphate (cAMP) and cyclic guanosine monophosphate (cGMP), and play an important role in regulating cellular activities. There are 11 PDE families currently known. Among them, PDE3 is mainly distributed in smooth muscle cells and is involved in regulating cardiac contractility and vascular smooth muscle. Inhibition of PDE3 produces effects of smooth muscle relaxation and bronchodilation. PDE4 is primarily distributed in various immune cells, airway epithelial cells, and fibroblasts. Inhibition of PDE4 can elevate intracellular cAMP levels in inflammatory cells and immunomodulatory cells, thereby suppressing inflammatory cell functions and relaxing airway smooth muscle.

Chronic obstructive pulmonary disease (COPD) and asthma are complex inflammatory diseases characterized by airway obstruction that are common in respiratory system. At present, roflumilast is the only PDE inhibitor approved for the clinical treatment of COPD; however, its widespread clinical use is limited due to dose-dependent adverse effects. Due to the limitations of using PDE3 or PDE4 inhibitors alone, dual inhibition of PDE3/PDE4 has emerged as a new targeted therapeutic strategy for COPD and asthma. Increasing experimental evidence has demonstrated that dual-target PDE3/PDE4 inhibitors exhibit synergistic inhibitory effects, i.e. synergistic anti-inflammatory and bronchodilation effects.

Ensifentrine (RPL554) is a novel inhaled PDE3/PDE4 inhibitor for treating COPD and asthma with dual anti-inflammatory and bronchodilation effects, and is currently in Phase III clinical trials. However, data also indicate that its inhibitory effect against PDE4 is relatively modest, and the anti-inflammatory effect is not satisfied. Therefore, there is a need to develop a PDE3/PDE4 dual-target inhibitor with improved tolerability, broader therapeutic windows, and enhanced safety, or PDE3 or PDE4 inhibitors with minimized side effects.

### SUMMARY

In view of this, the technical problem to be solved by the present disclosure is to provide a compound and use thereof. The compound exhibits favorable PDE3 and/or PDE4 inhibitory effect and can be used for treating diseases associated with PDE3 and/or PDE4.

To achieve the above purpose, the present disclosure adopts the following technical solution:

The present disclosure provides a compound having a structure represented by formula (I), or a deuterated compound, stereoisomer, or pharmaceutically acceptable salt thereof: 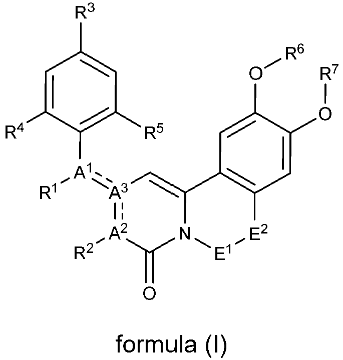 wherein, A¹ and A² are nitrogen atoms, A³ is a carbon atom.

In the case A¹ - - - A³ is a single bond and A³ - - - A² is a double bond, R² is absent.

In the case A¹ - - - A³ is a double bond and A³ - - - A² is a single bond, R¹ is absent.

R¹, R², R⁶, and R⁷ are each independently selected from the group consisting of hydrogen, substituted or unsubstituted linear or branched C₁-C₆ alkyl, substituted or unsubstituted C₂-C₆ alkenyl, substituted or unsubstituted C₂-C₆ alkynyl, and CₙH₂ₙR⁸.

n is an integer selected from 0 to 4.

R⁸ is one or more selected from the group consisting of substituted or unsubstituted 3-10 membered cycloalkyl, substituted or unsubstituted 4-10 membered heterocycloalkyl, substituted or unsubstituted 6-10 membered aryl, substituted or unsubstituted 5-10 membered heteroaryl, -OR^{a}, -OC(O)R^{a}, -OC(O)NR^{a}R^{b}, -C(O)R^{a}, - C(O)OR^{a}, -C(O)NR^{a}R^{b}, -NR^{a}R^{b}, -NR^{a}C(O)R^{b}, -NR^{a}C(O)OR^{b}, -NR^{a}C(O)NR^{b}CₙH₂ₙR^{c}, -SR^{a}, -S(O)R^{a}, -S(O)NR^{a}R^{b}, -S(O)₂R^{a}, -S(O)₂NR^{a}R^{b}, -NR^{a}S(O)₂R^{b}, -CN, -NH₂ and -CF₃.

The 3-10 membered cycloalkyl includes, but is not limited to, substituted or unsubstituted groups of cyclopropyl, cyclobutyl, cyclopentyl, cyclohexyl, cycloheptyl, and cyclooctyl.

The 4-10 membered heterocycloalkyl includes, but is not limited to, substituted or unsubstituted groups of oxetanyl, azetidinyl, pyrrolidinyl, 2-oxo-pyrrolidin-3-yl, tetrahydrofuranyl, tetrahydrothienyl, pyrazolidinyl, imidazolidinyl, thiazolidinyl, piperidinyl, tetrahydropyranyl, tetrahydrothiopyranyl, piperazinyl, morpholinyl, 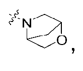 , thiomorpholinyl, 1,1-dioxo-thiomorpholin-4-yl, azepanyl, diazepanyl, homopiperazinyl, oxazepanyl, 8-aza-bicyclo[3.2.1]octyl, quinuclidinyl, 8-oxa-3-aza-bicyclo[3.2.1]octyl, 9-aza-bicyclo[3.3.1]nonyl, dihydrofuranyl, imidazolinyl, tetrahydropyridinyl, dihydropyranyl, [1,2,4]triazolo[4,3-a]pyrazinyl, pyrroloimidazolyl, 2-oxo-pyridinyl, and 3-oxo-pyridazinyl.

The 6-10 membered aryl includes, but is not limited to, substituted or unsubstituted phenyl and substituted or unsubstituted naphthyl.

The 5 to 10 membered heterocyclyl includes, but is not limited to, substituted or unsubstituted groups of pyridinyl, indolyl, quinoxalinyl, quinolinyl, isoquinolinyl, benzothiophenyl, benzofuryl, benzopyranyl, furyl, pyrrolyl, thiazolyl, oxazolyl, isoxazolyl, triazolyl, tetrazolyl, pyrazolyl, imidazolyl, thiophenyl, oxadiazolyl, benzimidazolyl, benzothiazolyl, and benzoxazolyl.

R³, R⁴ and R⁵ are independently one or more selected from the group consisting of hydrogen, halogen, and substituted or unsubstituted linear or branched C₁-C₆ alkyl.

The linear or branched C₁-C₆ alkyl is preferably selected from the group consisting of methyl, ethyl, n-propyl, i-propyl, n-butyl, i-butyl, sec-butyl, tert-butyl, n-pentyl, neopentyl, i-pentyl, hexyl and a combination thereof.

R^{a}, R^{b} and R^{c} are independently one or more selected from the group consisting of hydrogen, deuterium, oxo, thio, halogen, amino, methylimino, methoxyimino, methanesulfonyl, hydroxy, cyano, nitro, substituted or unsubstituted linear or branched C₁-C₆ alkyl, substituted or unsubstituted C₁-C₄ alkoxy, substituted or unsubstituted C₂-C₆ alkenyl, substituted or unsubstituted C₂-C₆ alkynyl, substituted or unsubstituted 3-10 membered cycloalkyl, substituted or unsubstituted 4-10 membered heterocycloalkyl, substituted or unsubstituted 6-10 membered aryl, and substituted or unsubstituted 5-10 membered heteroaryl.

The C₁-C₄ alkoxy includes, but is not limited to, methoxy, ethoxy, n-propoxy, i-propoxy, n-butoxy, i-butoxy, sec-butoxy, and tert-butoxy.

The range of the C₁-C₆ alkyl is the same as described above and is not repeated herein for brevity.

The C₂-C₆ alkenyl includes, but is not limited to, vinyl, propenyl, 2-methylpropenyl, allyl, butenyl, 2-methylbutenyl, 2-ethylbutenyl, cyclopentenyl, and cyclohexenyl.

The C₂-C₆ alkynyl includes, but is not limited to, ethynyl, propynyl, 1-butynyl, 2-butynyl, 1-pentynyl, 2-pentynyl, 4-methyl-1-butynyl, 3-methyl-1-butynyl, 3,3-dimethyl-1-propyynyl, 1-hexynyl, 2-hexynyl, 3-hexynyl, 4-methyl-1-pentynyl, 3-methyl-1-pentynyl, 4-methyl-2-pentynyl, 2,2-dimethyl-1-butynyl.

The ranges of the substituted or unsubstituted 3-10 membered cycloalkyl, 4-10 membered heterocycloalkyl, 6-10 membered aryl, and 5-10 membered heteroaryl are the same as described above and are not repeated herein for brevity.

E¹ is selected from -(CH₂)ₘ-, wherein m is 1, 2, or 3.

E² is selected from the group consisting of -O-, -NH-, -S-, 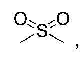 , and -CR⁹R¹⁰-.

R⁹ and R¹⁰ are independently one or more selected from the group consisting of hydrogen, deuterium, and substituted or unsubstituted linear or branched C₁-C₆ alkyl.

The preferred range of the C₁-C₆ alkyl is the same as described above and is not repeated herein for brevity.

Alternatively, R⁶ and R⁷ together with the adjacent oxygen atom and carbon atom of phenyl form a ring.

The ring is a substituted or unsubstituted 5 or 6 membered monocyclic heterocyclyl.

Optionally, the 5 or 6 membered monocyclic heterocyclyl is further fused or spiro-fused with aryl, heteroaryl, or heterocycloalkyl to form a fused ring group, a spiro ring group, or a bridged ring group.

Preferably, in the present disclosure, the above substituted 3-10 membered cycloalkyl, substituted 4-10 membered heterocycloalkyl, substituted 6-10 membered aryl, or substituted 5-10 membered heteroaryl is substituted by a substituent selected from the group consisting of deuterium, oxo, thio, halogen, hydroxy, cyano, nitro, substituted or unsubstituted linear or branched C₁-C₆ alkyl, substituted or unsubstituted C₃-C₁₀ cycloalkyl, substituted or unsubstituted C₂-C₆ alkenyl, substituted or unsubstituted C₂-C₆ alkynyl, CₙH₂ₙOR^{a}, -CₙH₂ₙOC(O)R^{a}, -CₙH₂ₙOC(O)NR^{a}R^{b}, -CₙH₂ₙC(O)R^{a}, -CₙH₂ₙC(O)OR^{a}, - CₙH₂ₙC(O)NR^{a}R^{b}, -CₙH₂ₙNR^{a}R^{b}, -CₙH₂ₙNR^{a}C(O)R^{b}, -CₙH₂ₙNR^{a}C(O)OR^{b}, - CₙH₂ₙNR^{a}C(O)NR^{b}CₙH₂ₙR^{c}, -CₙH₂ₙSR^{a}, -CₙH₂ₙS(O)R^{a}, -CₙH₂ₙS(O)NR^{a}R^{b}, -CₙH₂ₙS(O)₂R^{a}, -CₙH₂ₙS(O)₂NR^{a}R^{b}, -CₙH₂ₙNR^{a}S(O)₂R^{b}, -CₙH₂ₙCN, -CₙH₂ₙNH₂, -CₙH₂ₙCF₃ and a combination thereof.

Preferably, in the present disclosure, the above substituted linear or branched C₁-C₆ alkyl, or substituted C₁-C₄ alkoxy is substituted by a substituent selected from the group consisting of deuterium, oxo, thio, halogen, hydroxy, cyano, nitro, CₙH₂ₙOR^{a}, - CₙH₂ₙOC(O)R^{a}, -CₙH₂ₙOC(O)NR^{a}R^{b}, -CₙH₂ₙC(O)R^{a}, -CₙH₂ₙC(O)OR^{a}, -CₙH₂ₙC(O)NR^{a}R^{b}, -CₙH₂ₙNR^{a}R^{b}, -CₙH₂ₙNR^{a}C(O)R^{b}, -CₙH₂ₙNR^{a}C(O)OR^{b}, -CₙH₂ₙNR^{a}C(O)NR^{b} CₙH₂ₙR^{c}, - CₙH₂ₙSR^{a}, -CₙH₂ₙS(O)R^{a}, -CₙH₂ₙS(O)NR^{a}R^{b}, -CₙH₂ₙS(O)₂R^{a}, -CₙH₂ₙS(O)₂NR^{a}R^{b}, - CnH₂ₙNR^{a}S(O)₂R^{b}, -CₙH₂ₙCN, -CₙH₂ₙNH₂, -CₙH₂ₙCF₃ and a combination thereof.

Preferably, in the present disclosure, the substituted C₂-C₆ alkenyl is substituted by a substituent selected from the group consisting of deuterium, oxo, thio, halogen, hydroxy, cyano, nitro, substituted or unsubstituted C₁-C₆ alkyl, substituted or unsubstituted C₂-C₆ alkynyl, substituted or unsubstituted C₃-C₁₀ cycloalkyl, CₙH₂ₙOR^{a}, -CₙH₂ₙOC(O)R^{a}, -CₙH₂ₙOC(O)NR^{a}R^{b}, -CₙH₂ₙC(O)R^{a}, -CₙH₂ₙC(O)OR^{a}, -CₙH₂ₙC(O)NR^{a}R^{b}, -CₙH₂ₙNR^{a}R^{b}, - CₙH₂ₙNR^{a}C(O)R^{b}, -CₙH₂ₙNR^{a}C(O)OR^{b}, -CₙH₂ₙNR^{a}C(O)NR^{b} CₙH₂ₙR^{c}, -CₙH₂ₙSR^{a}, - CₙH₂ₙS(O)R^{a}, -CₙH_{2n\}S(O)NR^{a}R^{b}, -CₙH₂ₙS(O)₂R^{a}, -CₙH₂ₙS(O)₂NR^{a}R^{b}, -CₙH₂ₙNR^{a}S(O)₂R^{b}, -CₙH₂ₙCN, -CₙH₂ₙNH₂, -CₙH₂ₙCF₃ and a combination thereof.

Preferably, in the present disclosure, the substituted C₂-C₆ alkynyl is substituted by a substituent selected from the group consisting of deuterium, oxo, thio, halogen, hydroxy, cyano, nitro, substituted or unsubstituted C₁-C₆ alkyl, substituted or unsubstituted C₂-C₆ alkenyl, substituted or unsubstituted C₃-C₁₀ cycloalkyl, CₙH₂ₙOR^{a}, -CₙH₂ₙOC(O)R^{a}, -CₙH₂ₙOC(O)NR^{a}R^{b}, -CₙH₂ₙC(O)R^{a}, -CₙH₂ₙC(O)OR^{a}, -CₙH₂ₙC(O)NR^{a}R^{b}, -CₙH₂ₙNR^{a}R^{b}, - CₙH₂ₙNR^{a}C(O)R^{b}, -CₙH₂ₙNR^{a}C(O)OR^{b}, -CₙH₂ₙNR^{a}C(O)NR^{b} CₙH₂ₙR^{c}, -CₙH₂ₙSR^{a}, - CₙH₂ₙS(O)R^{a}, -CₙH_{2n\}S(O)NR^{a}R^{b}, -CₙH₂ₙS(O)₂R^{a}, -CₙH₂ₙS(O)₂NR^{a}R^{b}, -CₙH₂ₙNR^{a}S(O)₂R^{b}, -CₙH₂ₙCN, -CₙH₂ₙNH₂, -CₙH₂ₙCF₃ and a combination thereof.

Preferably, in the present disclosure, the R⁶ and R⁷ in formula (I) together with the adjacent oxygen atom and carbon atom of phenyl form a 5 or 6 membered monocyclic heterocyclyl selected from the group consisting of 1,3-dioxolanyl and 1,4-dioxanyl.

Preferably, the 5 or 6 membered monocyclic heterocyclyl is fused or spiro-fused with aryl selected from the group consisting of substituted or unsubstituted phenyl and substituted or unsubstituted naphthyl.

Preferably, the 5 or 6 membered monocyclic heterocyclyl is fused or spiro-fused with heteroaryl selected from the group consisting of substituted or unsubstituted pyridinyl, indolyl, quinoxalinyl, quinolinyl, isoquinolinyl, benzothiophenyl, benzofuryl, benzopyranyl, furyl, pyrrolyl, thiazolyl, oxazolyl, isoxazolyl, triazolyl, tetrazolyl, pyrazolyl, imidazolyl, thiophenyl, oxadiazolyl, benzimidazolyl, benzothiazolyl, benzoxazolyl, and a combination thereof.

Preferably, the 5 or 6 membered monocyclic heterocyclyl is fused or spiro-fused with heterocycloalkyl selected from the group consisting of substituted or unsubstituted oxetanyl, azetidinyl, azepanyl, diazepanyl, oxazepanyl, 8-aza-bicyclo[3.2.1]octyl, 8-oxa-3-aza-bicyclo[3.2.1]octyl, 9-aza-bicyclo[3.3.1]nonyl, 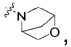 pyrrolidinyl, 2-oxo-pyrrolidin-3-yl, tetrahydrofuranyl, tetrahydrothiophenyl, pyrazolidinyl, imidazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, homopiperazinyl, quinuclidinyl, dihydrofuranyl, imidazolinyl, tetrahydropyridinyl, dihydropyranyl and a combination thereof.

Preferably, the substituted 5 or 6 membered monocyclic heterocyclyl, substituted aryl, substituted heteroaryl, or substituted heterocycloalkyl is independently substituted by a substituent selected from the group consisting of substituted or unsubstituted linear or branched C₁-C₆ alkyl, substituted or unsubstituted C₁-C₄ alkoxy, halogen, hydroxy, nitro, cyano, amino and a combination thereof.

The ranges of the linear or branched C₁-C₆ alkyl and C₁-C₄ alkoxy described above, as well as the preferred ranges of their substituents, are the same as described above and are not repeated herein for brevity.

In the present disclosure, -C(O), -S(O), and -S(O)₂ represent a double bond between the oxygen atom and the carbon atom or the sulfur atom, wherein the dash indicates the point of attachment.

The pharmaceutically acceptable salt of the structure represented by formula (I) includes, but is not limited to, hydrochloride, sulfate, citrate, benzenesulfonate, hydrobromide, hydrofluoride, phosphate, acetate, propionate, succinate, oxalate, malate, succinate, fumarate, maleate, tartrate, and trifluoroacetate, and a combination thereof.

Preferably, in the present disclosure, the compound has a structure selected from the group consisting of:

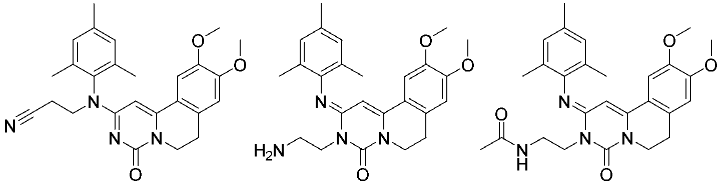

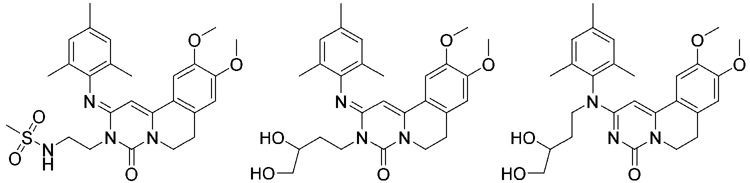

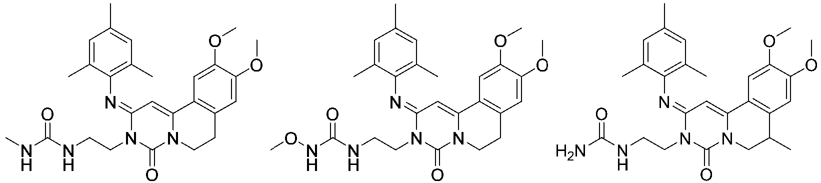

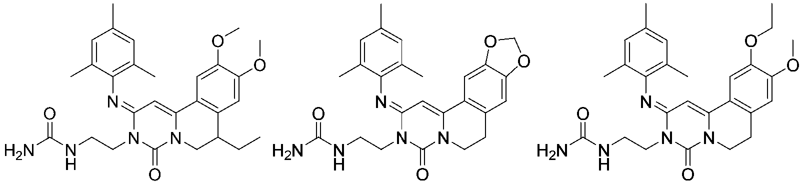

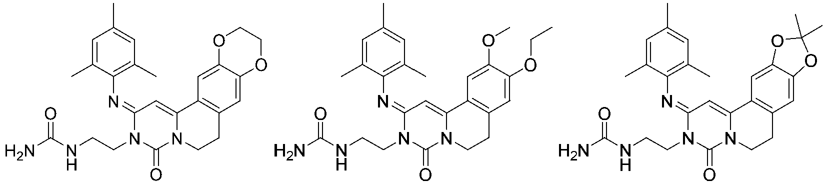

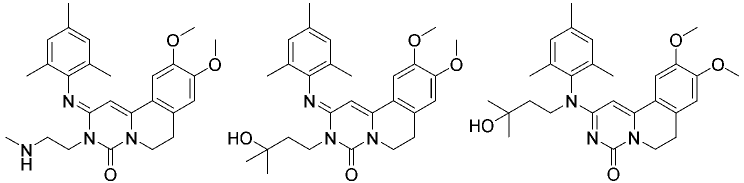

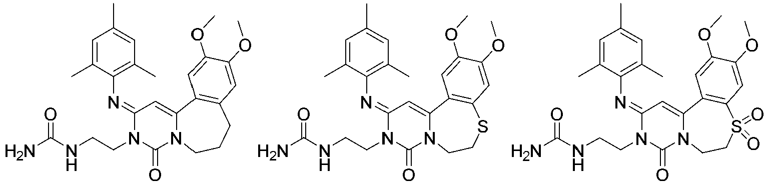

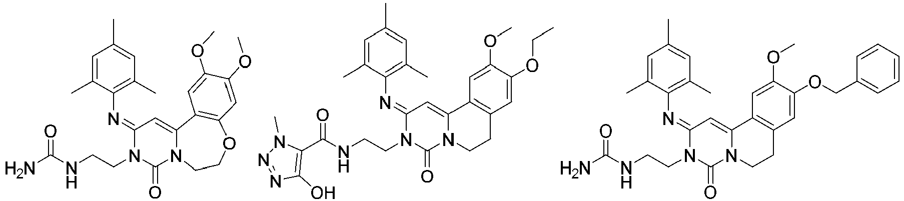

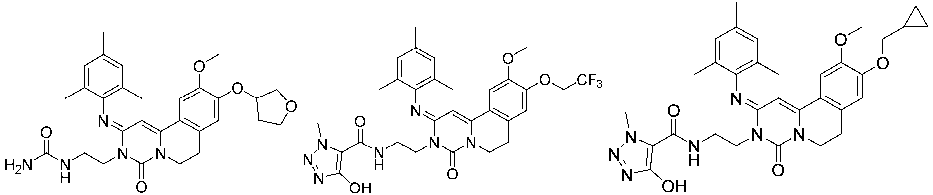

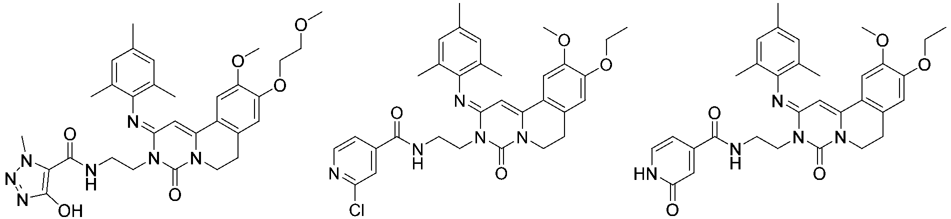

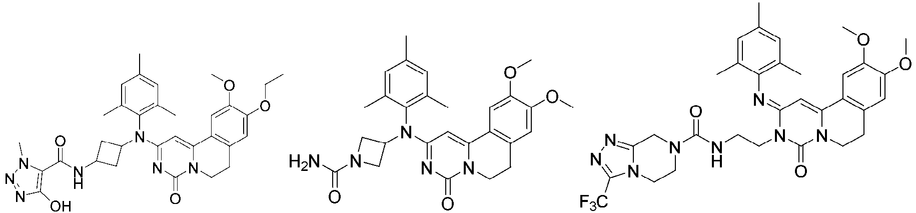

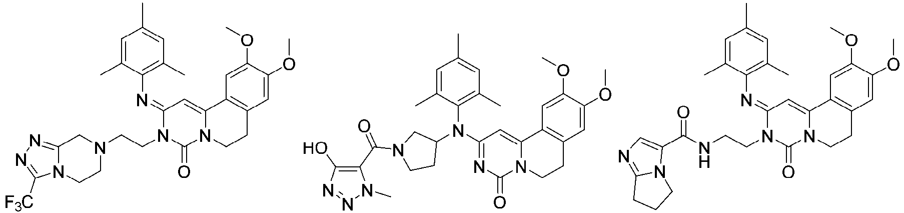

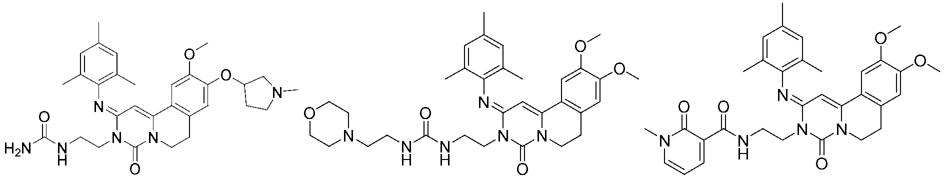

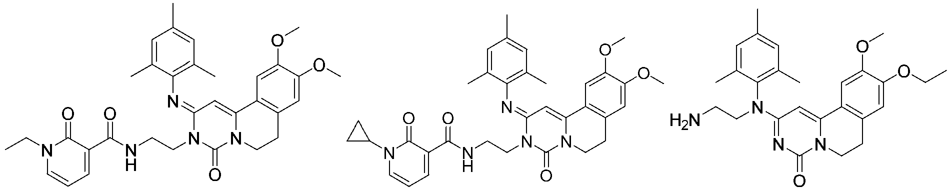

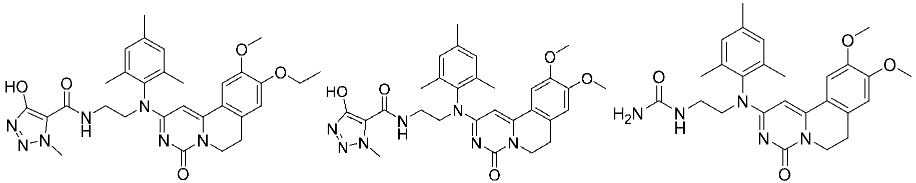

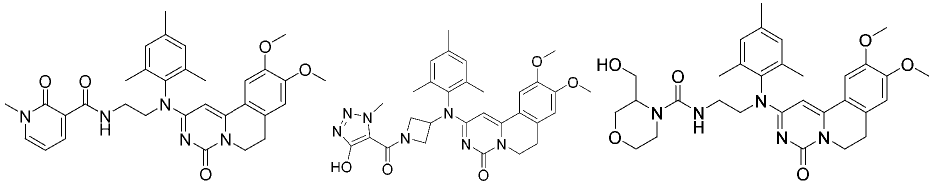

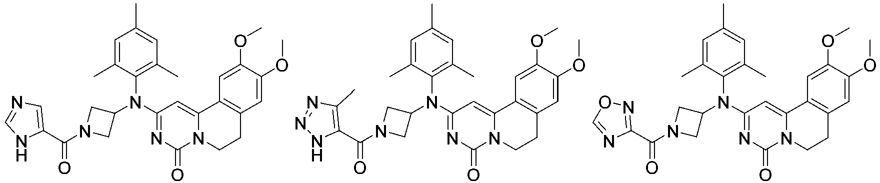

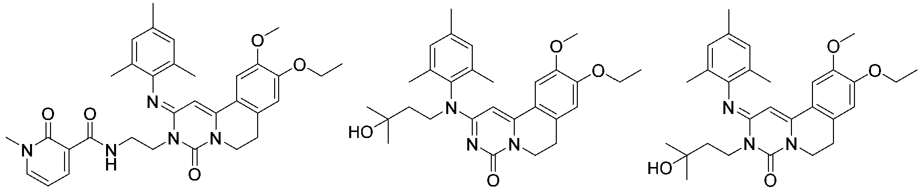

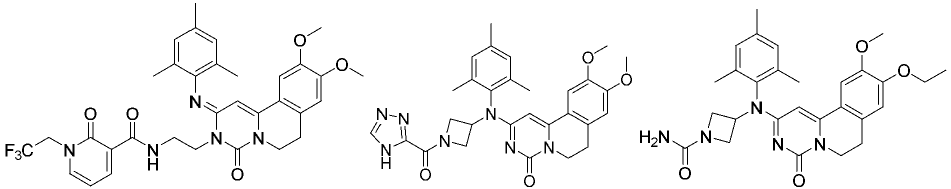

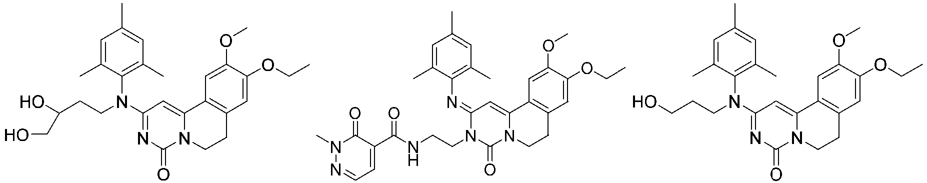

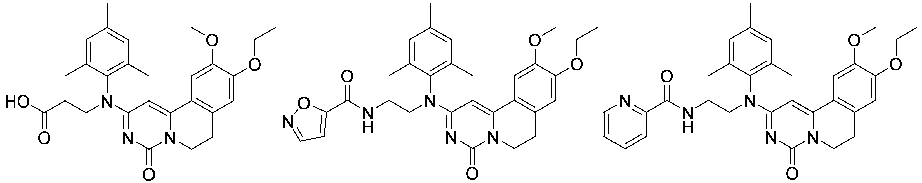

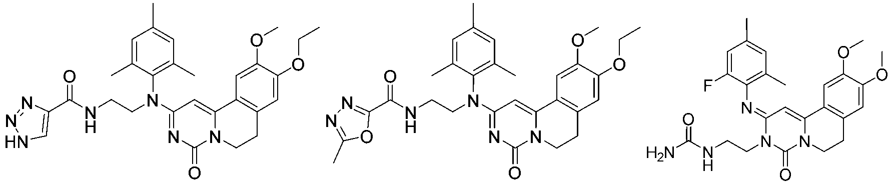

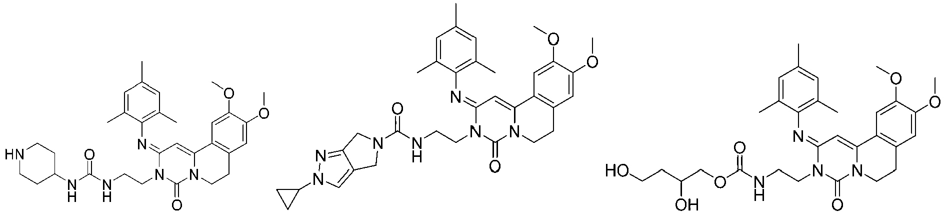

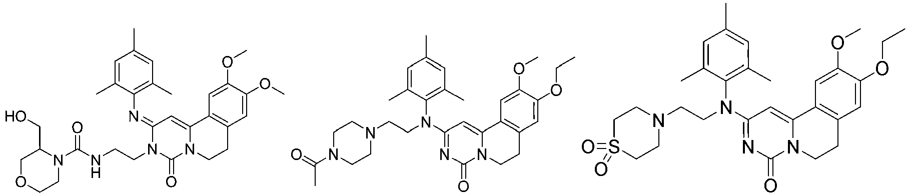

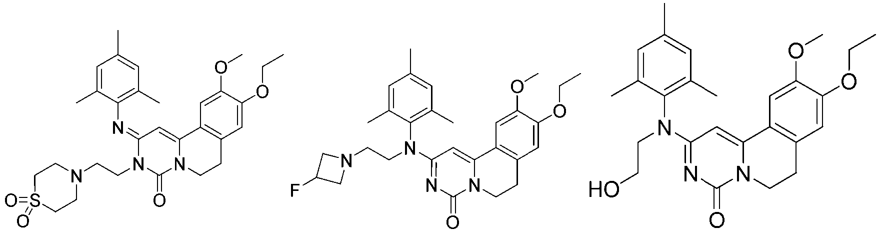

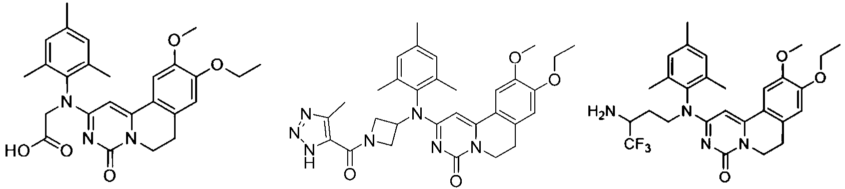

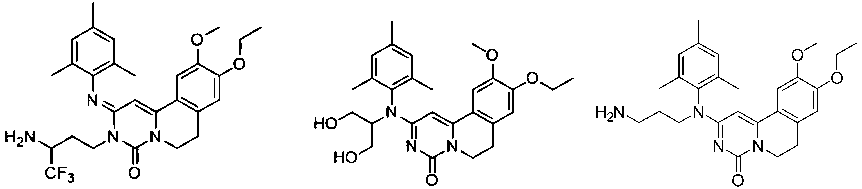

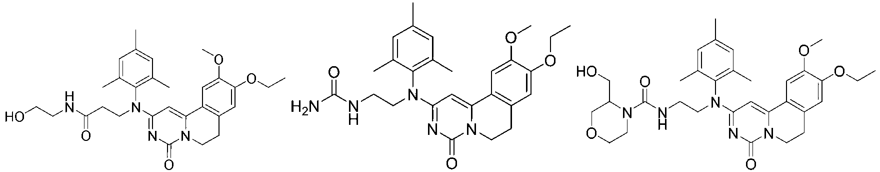

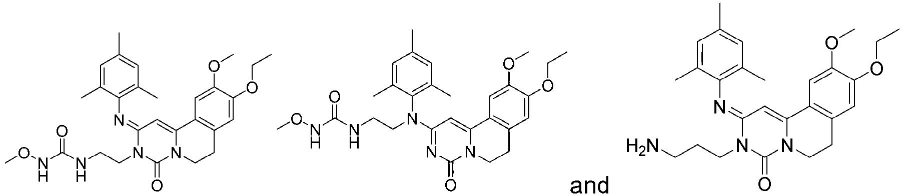

or a deuterated compound, stereoisomer, or pharmaceutically acceptable salt thereof.

The present disclosure further provides a pharmaceutical composition comprising the above compound and a pharmaceutically acceptable excipient.

The excipient may be a carrier, vehicle, diluent, or other excipient.

The present disclosure further provides use of the above compound or the above pharmaceutical composition in the manufacture of a medicament for treating a disease related to phosphodiesterase (PDE)3 and/or PDE4.

Preferably, the disease related to PDE3 and/or PDE4 is one or more selected from the group consisting of inflammation, bronchiectasis, chronic obstructive pulmonary disease, and asthma.

Compared with the prior art, the present disclosure provides a compound having a structure represented by formula (I), or a deuterated compound, stereoisomer, or pharmaceutically acceptable salt thereof. The compound exhibits good PDE3 and/or PDE4 inhibitory effect and can be used for treating a disease related to PDE3 and/or PDE4.

### DETAILED DESCRIPTION

To further illustrate the present disclosure, the compounds provided herein and their applications are described in detail below with reference to the examples.

The structures of the compounds were determined by proton nuclear magnetic resonance (¹H NMR) spectroscopy and mass spectrometry (MS), wherein:
The ¹H NMR spectra were recorded on a nuclear magnetic resonance spectrometer (Bruker Avance NEO 400). Deuterated dimethyl sulfoxide (DMSO-d₆), deuterated chloroform (CDCl₃, chloroform-d), or deuterated methanol (CD₃OD) were used as the solvent. Tetramethylsilane (TMS) was used as the internal standard.
The LC-MS instrument used was a Shimadzu liquid chromatography-mass spectrometry system (Shimadzu LC-MS 2020 (ESI)).
The HPLC instrument used was a Shimadzu high performance liquid chromatography (Shimadzu LC-2030C 3D (Plus)).

The preparative liquid chromatography was a Shimadzu LC-20AP.

The thin layer chromatography silica gel plates were GF254 silica gel plates manufactured by Yantai Xinno Chemical Co., Ltd.

The stationary phase for column chromatography was silica gel of 200 to 300 mesh supplied by Beijing Kedeli Technology Co., Ltd.

The starting materials used in the Examples of the present disclosure are known and commercially available, or may be synthesized according to known methods in the art.

Unless otherwise specified, all reactions described below were carried out under continuous magnetic stirring in a dry nitrogen or argon atmosphere, using dried solvents, and the reaction temperatures are expressed in degrees Celsius.

### Example 1: Synthesis of intermediate and compound 1

### (1) Synthesis of intermediate

### 1) Synthesis of intermediate IM-01

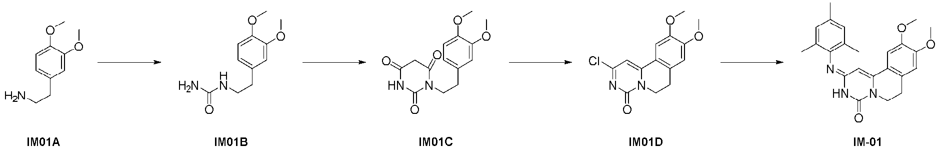

### Step 1, synthesis of compound IM01B

IM01A(6.0 g) was dissolved in water (42 mL), and concentrated hydrochloric acid (3.3 mL) and potassium cyanate (4.4 g) were added in sequence. The reaction mixture was stirred at 50 °C for 2 hours. After the reaction was completed, the mixture was cooled to room temperature and filtered, and the precipitated solid was collected. The filter cake was dried and IM01B (4.0 g) was yielded.

MS m/z (ESI): 225.1[M+1]⁺.

### Step 2, synthesis of compound IM01C

A solution of sodium ethoxide in ethanol (20 wt%, 32.5 g) was added to anhydrous ethanol (40 mL), and IM01B (4.0 g) and diethyl malonate (7.7 g) were added in sequence. The reaction mixture was heated to 80 °C for 16 hours of reaction. After the reaction was completed, ethanol was removed by concentration under reduced pressure. The residue was dissolved in water, and dilute hydrochloric acid (2 M) was added dropwise to the resulting solution in an ice bath to adjust the pH to 2. The solid was collected by filtration, the filter cake was dried, and IM01C (3.0 g) was yielded. MS m/z (ESI): 315.0[M+23]⁺.

### Step 3, synthesis of compound IM01D

Compound IM01C (10.0 g) was added to phosphorus oxychloride (100 mL). Under a nitrogen atmosphere, the reaction mixture was stirred at 85 °C for 16 hours of reaction. After the reaction was completed, the mixture was cooled to room temperature. Phosphorus oxychloride was removed by concentration under reduced pressure, and the residue was dissolved in dichloromethane (100 mL). The organic phase was washed with ice water (100 mL × 2), dried over anhydrous sodium sulfate, and concentrated, and IM01D (11.0 g) was yielded.

MS m/z (ESI): 293.1[M+1]⁺.

### Step 4, synthesis of compound IM-01

IM01D (10.0 g) was dissolved in isopropyl alcohol (100 mL), and 2,4,6-trimethylaniline (23.1 g) was added. The reaction mixture was reacted at 90 °C for 16 hours. After the reaction was completed, the mixture was concentrated to yield a crude product, and the crude product was purified by silica gel column chromatography (dichloromethane/methanol = 10/1), and IM-01 (10.0 g) was yielded.

MS m/z (ESI): 392.2[M+1]⁺.

¹H NMR (400 MHz, DMSO-d₆) δ 9.42 (br, 1H), 7.29 (s, 1H), 7.03 (s, 1H), 6.93 (s, 1H), 6.52 (s, 1H), 5.76 (s, 1H), 3.93 (t, *J* = 6.4 Hz, 2H), 3.89-3.80 (m, 6H), 2.92 (t, *J* = 6.5 Hz, 2H), 2.27 (s, 3H), 2.14 (s, 6H).

### 2) Synthesis of intermediate IM-02

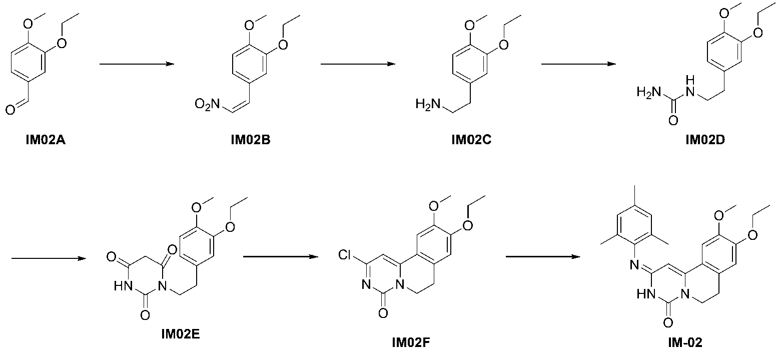

### Step 1, synthesis of compound IM02B

IM02A (21 g) and ammonium acetate (2.5 g) were added to nitromethane (250 mL). Under a nitrogen atmosphere, the reaction mixture was stirred at 100 °C for 3 hours. After the reaction was completed, the mixture was filtered, and the filtrate was concentrated under reduced pressure. The resulting residue was purified by silica gel column chromatography (petroleum ether/ethyl acetate = 4/1 to 3/2), and IM02B (24.0 g) was yielded.

### Step 2, synthesis of compound IM02C

Under a nitrogen atmosphere, a solution of lithium aluminium hydride in tetrahydrofuran (270 mL) was added to a three-necked flask. IM02B (24.0 g) was dissolved in tetrahydrofuran (250 mL) and was added dropwise to the three-necked flask at room temperature. After completion of the addition, the reaction mixture was heated to 65 °C and stirred for 3 hours. After the reaction was completed, the mixture was cooled to room temperature, and water (10 mL), 15% aqueous sodium hydroxide solution (10 mL), and water (30 mL) were added slowly in sequence. An appropriate amount of anhydrous sodium sulfate was added, and the mixture was stirred for 20 minutes. The mixture was filtered, and the filtrate was collected. The filter cake was stirred in a dichloromethane/methanol (10/1) mixed solvent for 20 minutes and filtered. The filtrates were combined and concentrated under reduced pressure. The crude product was purified by silica gel column chromatography (dichloromethane/methanol = 93/7), and IM02C (15.0 g) was yielded. MS m/z (ESI):196.0[M+1]⁺.

### Step 3, synthesis of compound IM02D

IM02C (2.2 g) was dissolved in 1,2-dichloroethane (20 mL), and trimethylsilyl isocyanate (3.9 g) was added. The reaction mixture was stirred at 80 °C for 16 hours. After the reaction was completed, the mixture was cooled to room temperature and concentrated under reduced pressure. The resulting crude product was purified by silica gel column chromatography (dichloromethane/methanol = 10/1), and IM02D (2.0 g) was yielded. MS m/z (ESI): 239.2[M+1]⁺.

### Step 4, synthesis of compound IM02E

A solution of sodium ethoxide in ethanol (20%, 14.0 g) was added to anhydrous ethanol (20 mL), and IM02D (2.0 g) and diethyl malonate (3.4 g) were added in sequence. The reaction mixture was heated to 80 °C for 16 hours of reaction. After the reaction was completed, the mixture was cooled to room temperature, and ethanol was removed by concentration under reduced pressure. The residue was dissolved in water, and hydrochloric acid (2 M) was added dropwise in an ice bath to adjust the pH until a solid precipitated. The precipitated solid was collected by filtration anddried, and IM02E (1.5 g) was yielded. MS m/z (ESI): 307.1[M+1]⁺.

### Step 5, synthesis of compound IM02F

IM02E (1.5 g) was added to phosphorus oxychloride (20 mL). Under a nitrogen atmosphere, the reaction mixture was stirred at 85 °C for 16 hours of reaction. After the reaction was completed, the mixture was cooled to room temperature. Phosphorus oxychloride was removed by concentration under reduced pressure, and the residue was dissolved in dichloromethane (50 mL). The organic phase was washed with ice water (50 mL × 2), dried over anhydrous sodium sulfate, and concentrated under reduced pressure, and IM02F (1.0 g) was yielded. MS m/z (ESI): 307.1[M+1]⁺.

### Step 6, synthesis of compound IM-02

IM02F (1.0 g) was dissolved in isopropyl alcohol (20 mL), and 2,4,6-trimethylaniline (2.2 g) was added. The reaction mixture was reacted at 90 °C for 16 hours. After the reaction was completed, the mixture was concentrated. The crude product was purified by silica gel column chromatography (dichloromethane/methanol = 10/1), and IM-02 (1.0 g) was yielded. MS m/z (ESI): 406.2[M+1]⁺.

### (2) Synthesis of compound 1

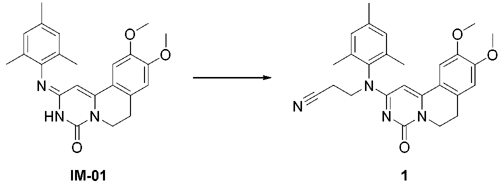

IM-01 (200 mg) was dissolved in 2-butanone (20 mL), and added with 3-bromopropionitrile (376 mg), potassium carbonate (635 mg) and potassium iodide (509 mg) in sequence. Under a nitrogen atmosphere, the reaction mixture was stirred at 85 °C for 16 hours of reaction. After the reaction was completed, the mixture was filtered, and the filtrate was concentrated under reduced pressure to yield a crude product. The crude product was purified by silica gel column chromatography (dichloromethane/methanol = 10/1), and compound 1 (120 mg) was yielded. MS m/z (ESI): 445.2[M+1]⁺.

¹H NMR (400 MHz, CDCl₃) δ 7.02 (s, 2H), 6.70 (s, 1H), 6.64 (s, 1H), 5.37 (d, *J* = 0.8 Hz, 1H), 4.16 (dt, *J* = 10.4, 6.7 Hz, 4H), 3.94-3.87 (m, 3H), 3.73 (d, J = 0.8 Hz, 3H), 2.96-2.85 (m, 4H), 2.34 (s, 3H), 2.17 (s, 6H).

### Example 2: synthesis of compound 2

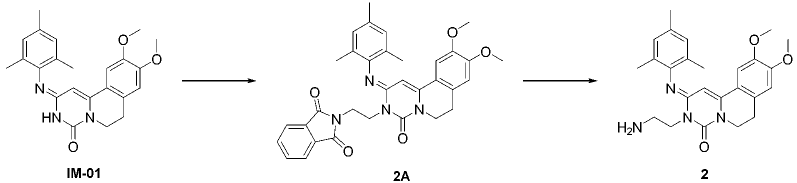

### Step 1, synthesis of compound 2A

IM-01 (4.0 g), 2-(2-bromoethyl)isodihydroindole-1,3-dione (14.3 g), potassium carbonate (12.7 g), and sodium iodide (9.2 g) were added to 2-butanone (40 mL). The reaction mixture was stirred at 85 °C for 16 hours. After the reaction was completed, the solvent was removed by rotary evaporation. The resulting crude product was purified by silica gel column chromatography (dichloromethane/methanol = 20/1), and compound 2A (0.8 g) was yielded. MS m/z (ESI): 565.4[M+1]⁺.

### Step 2, synthesis of compound 2

Compound 2A(800 mg) was dissolved in ethanol (10 mL), and hydrazine hydrate (212 mg) was added. Under a nitrogen atmosphere, the mixture was stirred at 85 °C for 16 hours. After the reaction was completed, the mixture was subjected to suction filtration. The filtrate was concentrated and purified by Flash reversed-phase column chromatography, and compound 2 (300 mg) was yielded. MS m/z (ESI): 435.2[M+1]⁺.

¹H NMR (400 MHz, DMSO-d₆) δ 8.35 (s, 1H), 6.97 (s, 1H), 6.86 (s, 2H), 6.67 (s, 1H), 5.33 (s, 1H), 4.28 (t, *J* = 6.4 Hz, 2H), 3.91 (t, *J* = 5.9 Hz, 2H), 3.80 (s, 3H), 3.62 (s, 3H), 3.04 (t, *J* = 6.4 Hz, 2H), 2.90 (t, *J* = 5.8 Hz, 2H), 2.21 (s, 3H), 1.96 (s, 6H), 1.23 (s, 2H).

### Example 3: synthesis of compound 3

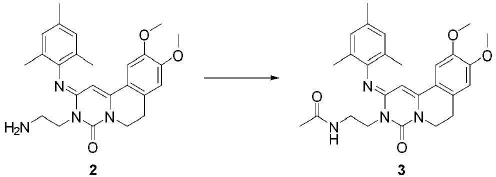

Compound 2 (40 mg) and acetic anhydride (14 mg) were dissolved in dichloromethane (5 mL), and added with triethylamine (19 mg). The reaction mixture was stirred at 25°C for 2 hours. After the reaction was completed, the reaction mixture was concentrated, and the crude product was purified by Flash reversed-phase chromatography, and compound 3 (10 mg, TFA salt) was yielded. MS m/z(ESI): 477.2[M+1]⁺.

¹H NMR (400 MHz, DMSO-d₆) δ 10.78 (s, 1H), 8.28 (s, 1H), 7.08 (s, 3H), 6.78 (s, 1H), 5.57 (s, 1H), 4.32 (s, 2H), 4.06 (s, 2H), 3.85 (s, 3H), 3.65 (s, 3H), 3.48 (d, *J =* 5.9 Hz, 2H), 2.99 (s, 2H), 2.32 (s, 3H), 2.19 (s, 6H), 1.82 (s, 3H).

### Example 4: synthesis of compound 4

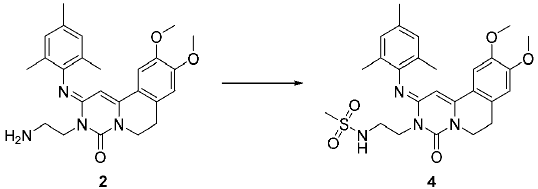

Compound 2 (40 mg) and methanesulfonic anhydride (24 mg) were dissolved in dichloromethane (5 mL), and added with triethylamine (19 mg). The reaction mixture was stirred at 25°C for 2 hours. After the reaction was completed, the reaction mixture was concentrated, and the crude product was purified by Flash reversed-phase chromatography, and compound 4 (10 mg, TFA salt) was yielded. MS m/z (ESI): 513.2[M+1]⁺.

¹H NMR (400 MHz, DMSO-d₆) δ 10.54 (s, 1H), 7.23 (t, *J* = 6.3 Hz, 1H), 7.07 (s, 3H), 6.75 (s, 1H), 5.56 (s, 1H), 4.38 (s, 2H), 4.06 (s, 2H), 3.84 (s, 3H), 3.64 (s, 4H), 3.42 (s, 2H), 2.96 (s, 2H), 2.95 (s, 3H), 2.30 (s, 4H), 2.18 (s, 5H).

### Example 5: synthesis of compound 5 and compound 6

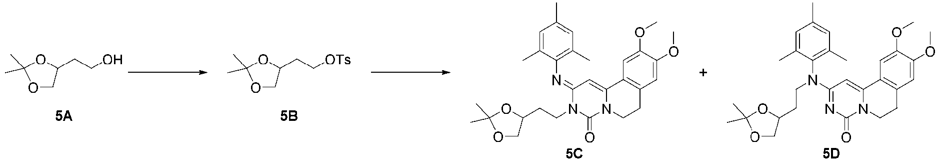

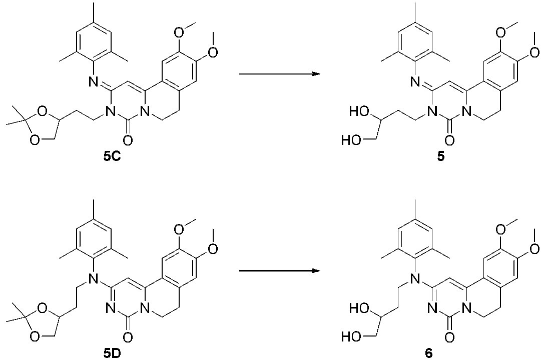

### Step 1, synthesis of compound 5B

At 0°C, compound 5A (2.0 g) and triethylamine (2.08 g) were dissolved in anhydrous dichloromethane (20 mL), and dropwise added with a solution of p-toluenesulfonyl chloride (3.1 g) in dichloromethane (30 mL). After the addition was completed, the reaction mixture was stirred at 0°C for 4 hours. After the reaction was completed, the mixture was filtered, and the filtrate was concentrated under reduced pressure. The crude product was purified by silica gel column chromatography (petroleum ether/ethyl acetate = 10/1) and compound 5B (3.0 g) was yielded. MS m/z (ESI): 301.1[M+1]⁺.

### Step 2, synthesis of compound 5C and compound 5D

Compound 5B (1.0 g) was dissolved in N,N-dimethylformamide (10 mL), and added with IM-01 (1.5 g), potassium carbonate (2.5 g), and potassium iodide (2.1 g) in sequence. The reaction mixture was heated to 80°C and stirred for 16 hours. After the reaction was completed, the reaction mixture was diluted with water (50 mL), and extracted with ethyl acetate (50 mL × 3). The combined organic phases were dried by sodium sulfate and concentrated to yield a crude product. The crude product was purified by Flash reversed-phase column chromatography, and compound 5C (0.45 g) and compound 5D (0.1 g) were yielded. 5C and 5D: MS m/z (ESI): 520.2[M+1]⁺.

### Step 3, synthesis of compound 5

Compound 5C (100 mg) was dissolved in methanol (1.0 mL), and added with dilute hydrochloric acid (2 mol/L, 1.0 mL). The reaction mixture was stirred at room temperature for 2 hours. After the reaction was completed, the mixture was concentrated to yield a crude product. The crude product was purified by Flash reversed-phase column chromatography, and compound 5 (80 mg) was yielded. MS m/z (ESI): 480.5[M+1]⁺.

¹H NMR (400 MHz, DMSO-d₆) δ 10.75 (s, 1H), 7.15-7.07 (m, 3H), 6.75 (s, 1H), 5.54 (s, 1H), 4.59-4.48 (m, 3H), 4.36-4.28 (m, 1H), 4.08 (t, *J* = 6.4 Hz, 2H), 3.85 (s, 3H), 3.70 (dd, *J* = 8.8, 3.4 Hz, 1H), 3.63 (s, 3H), 3.43-3.37 (m, 1H), 3.35-3.29 (m, 1H), 3.01 (t, *J* = 6.4 Hz, 2H), 2.33 (s, 3H), 2.20 (s, 6H), 2.05-1.95 (m, 1H), 1.87-1.76 (m, 1H).

### Step 4, synthesis of compound 6

Compound 5D (50 mg) was dissolved in methanol (0.5 mL), and added with dilute hydrochloric acid (2 mol/L, 0.5 mL). The reaction mixture was stirred at room temperature for 2 hours. After the reaction was completed, the mixture was concentrated to yield a crude product. The crude product was purified by Flash reversed-phase column chromatography, and compound 6 (40 mg) was yielded. MS m/z (ESI): 480.2[M+1]⁺.

¹H NMR (400 MHz, DMSO-d₆) δ 7.47 (s, 1H), 7.07 (d, *J =* 1.8 Hz, 2H), 7.00 - 6.92 (m, 3H), 6.72 (s, 1H), 6.57 (s, 1H), 5.23 (s, 1H), 4.90-4.86 (m, 2H), 4.64 (t, *J =* 5.6 Hz, 1H), 4.53 (t, *J* = 5.7 Hz, 1H), 4.01- 3.89 (m, 4H), 3.85 (d, *J* = 4.0 Hz, 3H), 3.80 (s, 3H), 3.76-3.67 (m, 2H), 3.58 (s, 3H), 2.91-2.86 (m, 3H), 2.30 (s, 3H), 2.26 (s, 2H), 2.10 (d, *J* = 5.3 Hz, 6H), 2.05 (s, 3H), 1.84-1.73 (m, 1H), 1.46-1.36 (m, 1H).

### Example 6: synthesis of compound 7

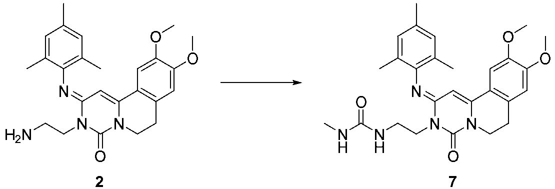

Compound 2 (40 mg) and methylcarbamoyl chloride (9 mg) were dissolved in dichloromethane (5 mL), and added with triethylamine (11 mg). The reaction mixture was stirred at 25 °C for 2 hours. After the reaction was completed, the mixture was concentrated to yield a crude product. The crude product was purified by Flash reversed-phase column chromatography, and compound 7 (10 mg) was yielded. MS m/z (ESI): 492.3[M+1]⁺.

¹H NMR (400 MHz, DMSO-d₆) δ 7.04 (s, 3H), 6.77 (s, 1H), 5.54 (s, 1H), 4.25 (s, 2H), 4.04 (s, 2H), 3.84 (s, 3H), 3.65 (s, 6H), 3.42 (s, 2H), 2.98 (s, 2H), 2.30 (s, 3H), 2.16 (s, 6H).

### Example 7: synthesis of compound 8

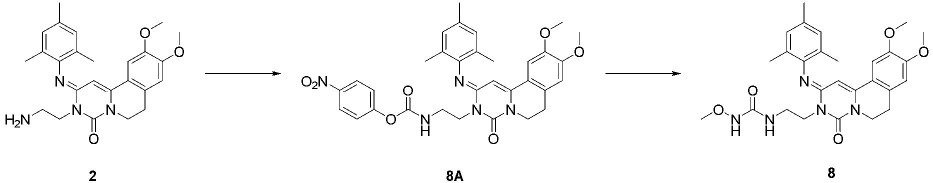

### Step 1, synthesis of compound 8A

Compound 2 (70 mg) and 4-nitrophenyl chloroformate (36 mg) were dissolved in dichloromethane (5 mL), and added with triethylamine (33 mg). The reaction mixture was stirred at 25 °C for 2 hours. After the reaction was completed, the mixture was concentrated to yield a crude product. The crude product was purified by Flash reversed-phase column chromatography, and compound 8A (60 mg) was yielded. MS m/z (ESI): 600.2[M+1]⁺.

### Step 2, synthesis of compound 8

Compound 8A (60 mg) and methoxyamine hydrochloride (10 mg) were dissolved in dichloromethane (5 mL), and added with triethylamine (20 mg). The reaction mixture was stirred at 25 °C for 16 hours. After the reaction was completed, the mixture was concentrated to yield a crude product. The crude product was purified by Flash reversed-phase column chromatography, and compound 8 (10 mg, TFA salt) was yielded. MS m/z (ESI): 508.3[M+1]⁺.

¹H NMR (400 MHz, DMSO-d₆) δ 10.75 (s, 1H), 9.35 (s, 1H), 7.26 (s, 1H), 7.08 (s, 3H), 6.78 (s, 1H), 5.57 (s, 1H), 4.35 (d, *J* = 5.6 Hz, 2H), 4.06 (s, 2H), 3.85 (s, 3H), 3.64 (s, 3H), 3.55-3.52 (m, 3H), 3.51 (d, *J* = 6.2 Hz, 2H), 2.99 (s, 2H), 2.31 (s, 3H), 2.19 (s, 6H).

### Example 8: synthesis of compound 9

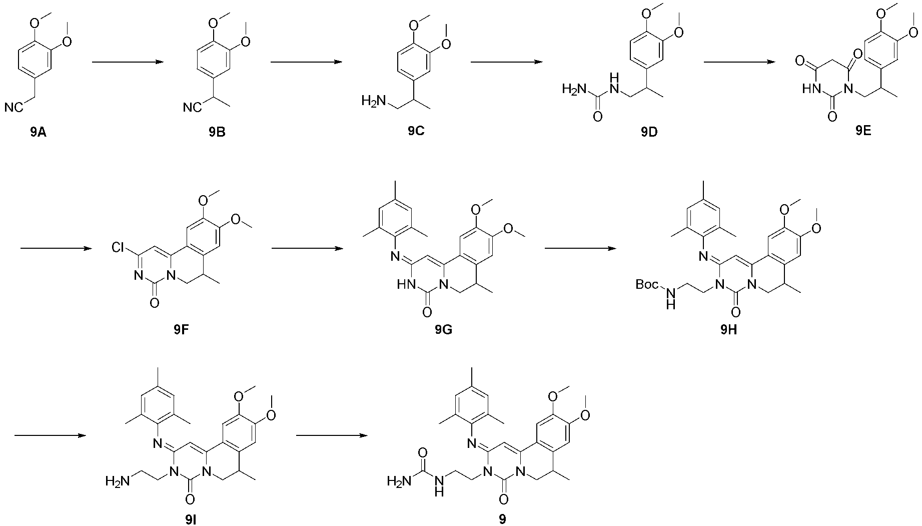

### Step 1, synthesis of compound 9B

Compound 9A (10 g) was dissolved in tetrahydrofuran (100 mL), and the solution was cooled to -78 °C under a nitrogen atmosphere, and dropwise added with a solution of lithium diisopropylamide in tetrahydrofuran (2 M, 28.2 mL). After the addition was completed, the mixture was stirred at -78 °C for 1 hour, and methyl iodide (9.61 g) was then added dropwise. The reaction mixture was stirred at room temperature for 16 hours. After the reaction was completed, saturated ammonium chloride solution (100 mL) was added dropwise to quench the reaction. The aqueous phase was extracted with ethyl acetate (150 mL × 2). The organic phases were combined, dried over anhydrous sodium sulfate and concentrated under reduced pressure to yield a crude product. The crude product was purified by silica gel column chromatography (petroleum ether/ethyl acetate = 4/1), and compound 9B (7.0 g) was yielded.

¹H NMR (400 MHz, CDCl₃) δ 6.91-6.85 (m, 3H), 3.87 (m, 7H), 1.64 (d, *J =* 7.3 Hz, 3H).

### Step 2, synthesis of compound 9C

Compound 9B (5.0 g) was dissolved in tetrahydrofuran (50 mL), and added with borane-dimethyl sulfide complex solution (2 M, 26.1 mL). The reaction mixture was stirred at 70 °C for 16 hours. After the reaction was completed, the mixture was cooled to 0 °C and methanol (5 mL) was added dropwise to quench the reaction. The reaction mixture was then concentrated and compound 9C (5.0 g) was yielded. MS m/z(ESI): 196.2[M+1]⁺.

### Step 3, synthesis of compound 9D

Compound 9C (1.0 g) was dissolved in dichloroethane (20 mL), and trimethylsilyl isocyanate (1.77 g) was added. The reaction mixture was stirred at 80 °C for 16 hours. After the reaction was completed, the mixture was cooled to room temperature, concentrated, dried under vacuum, and compound 9D (1.1 g) was yielded. MS m/z (ESI): 239.0[M+1]⁺.

### Step 4, synthesis of compound 9E

A solution of sodium ethoxide in ethanol (20%, 8.85 g) was added to anhydrous ethanol (10 mL), and added with compound 9D (1.25 g) and diethyl malonate (2.08 g) in sequence. The reaction mixture was reacted at 80 °C for 16 hours. After the reaction was completed, the mixture was cooled to room temperature and concentrated under reduced pressure to remove ethanol. The residue was dissolved in water, and hydrochloric acid (2 M) was dropwise added in an ice bath to adjust the pH to 5. The mixture was filtered through diatomaceous earth, and the filtrate was extracted with ethyl acetate (150 mL × 2). The organic phases were combined, washed with saturated saline (150 mL), dried over anhydrous sodium sulfate, filtered, and concentrated under reduced pressure. The resulting residue was purified by Flash silica gel chromatography (dichloromethane/methanol = 20/1) and compound 9E (1.2 g) was yielded. MS m/z (ESI): 306.9[M+1]⁺.

### Step 5, synthesis of compound 9F

Under a nitrogen atmosphere, compound 9E (1.1 g) was added to phosphorus oxychloride (10 mL). The reaction mixture was heated to 80 °C and stirred for 12 hours. After the reaction was completed, the mixture was cooled to room temperature and concentrated under reduced pressure to remove phosphorus oxychloride. The resulting residue was dissolved in dichloromethane (50 mL), washed with water (50 mL × 2), dried over anhydrous sodium sulfate, and concentrated, and compound 9F (1.0 g) was yielded. MS m/z (ESI): 307.0[M+1]⁺.

### Step 6, synthesis of compound 9G

Compound 9F (1.0 g) was dissolved in isopropyl alcohol (50 mL), and added with 2,4,6-trimethylaniline (2.23 g). The reaction mixture was reacted at 90 °C for 16 hours. After the reaction was completed, the mixture was concentrated to yield a crude product. The crude product was purified by silica gel column chromatography (dichloromethane/methanol = 10/1), and compound 9G (0.8 g) was yielded. MS m/z (ESI): 406.0[M+1]⁺.

### Step 7, synthesis of compound 9H

Compound 9G (300 mg) was added to toluene (10 mL), and added with tert-butyl (2-hydroxyethyl)carbamate (173 mg) and cyanomethylene tri-n-butylphosphorane (515 mg). Under a nitrogen atmosphere, the mixture was stirred at 120 °C for 4 hours. After the reaction was completed, the mixture was concentrated to yield a crude product. The crude product was purified by silica gel column chromatography (dichloromethane/methanol = 10/1) and compound 9H (210 mg) was yielded. MS m/z (ESI): 549.5[M+1 ]⁺.

### Step 8, synthesis of compound 9l

Compound 9H (100 mg) was added to 4M hydrochloric acid/dioxane solution (5 mL) and the mixture was stirred at 25 °C for 1 hour. After the reaction was completed, the mixture was concentrated to yield compound 9l (90 mg). MS m/z (ESI): 449.1[M+1]⁺.

### Step 9, synthesis of compound 9

Compound 9I (100 mg) was added to dichloromethane (5 mL), and added with phenyl carbamate (46 mg) and triethylamine (68 mg) in sequence. The reaction mixture was stirred at 25 °C for 16 hours. After the reaction was completed, the mixture was concentrated to yield a crude product. The crude product was purified by Flash reversed-phase chromatography, and compound 9 (40 mg) was yielded. MS m/z (ESI): 492.0[M+1]⁺.

¹H NMR (400 MHz, DMSO-d₆) δ 7.18-6.99 (m, 3H), 6.78 (s, 1H), 6.54 (s, 1H), 6.01 (s, 2H), 5.62 (s, 1H), 4.34-4.06 (m, 3H), 3.96-3.76 (m, 4H), 3.64 (s, 3H), 3.51-3.32 (m, 2H), 3.29-3.15 (m, 1H), 2.32 (s, 3H), 2.22 (s, 6H), 1.22 (d, *J* = 7.0 Hz, 3H).

### Example 9: synthesis of compound 10

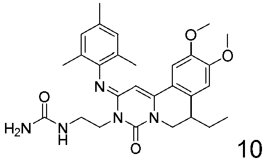

Compound 10 (25 mg) was yielded by referring to the synthetic method described in Example 8. MS m/z (ESI): 506.1[M+1]⁺.

¹H NMR (400 MHz, DMSO-d₆) δ 7.12 (s, 1H), 7.07 (d, *J* = 4.8 Hz, 2H), 6.77 (s, 1H), 6.52 (s, 1H), 5.98 (s, 2H), 5.61 (s, 1H), 4.64-4.60 (m, 1H), 4.27 (s, 2H), 3.86 (s, 3H), 3.65-3.59 (m, 4H), 3.41 (s, 2H), 2.98 (s, 1H), 2.32 (s, 3H), 2.23 (s, 3H), 2.20 (s, 3H), 1.48 (m, 2H), 0.91 (t, *J* = 7.3 Hz, 3H).

### Example 10: synthesis of compound 11

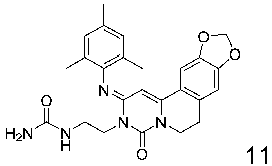

Compound 11 (60 mg) was yielded by referring to the synthetic method of intermediate IM-01 and Example 8. MS m/z (ESI): 462.1 [M+1 ]⁺.

¹H NMR (400 MHz, DMSO-d₆) δ 11.54 (s, 1H), 7.19 (s, 1H), 7.07 (d, *J* = 14.5 Hz, 3H), 6.58 (s, 1H), 6.25-5.95 (m, 4H), 5.65 (s, 1H), 4.26 (t, *J* = 6.8 Hz, 2H), 4.06 (t, *J* = 6.2 Hz, 2H), 3.39 (d, *J* = 5.9 Hz, 2H), 2.97 (t, *J* = 6.0 Hz, 2H), 2.33 (s, 3H), 2.19 (s, 6H).

### Example 11: synthesis of compound 12

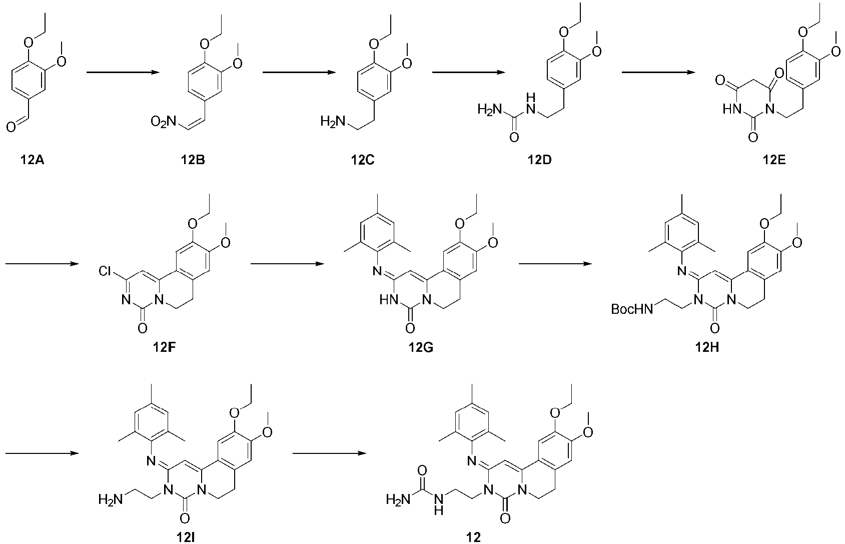

### Step 1, synthesis of compound 12B

Compound 12A (5.2 g) and ammonium acetate (0.6 g) were added to nitromethane (50 mL). Under a nitrogen atmosphere, the reaction mixture was stirred at 100 °C for 3 hours. After the reaction was completed, the mixture was filtered, and the filtrate was concentrated under reduced pressure. The residue was purified by silica gel column chromatography (petroleum ether/ethyl acetate = 4/1 to 3/2), and compound 12B (6 g) was yielded.

¹H NMR (400 MHz, DMSO-d₆) δ 8.21 (d, *J* = 13.5 Hz, 1H), 8.06 (d, *J* = 13.5 Hz, 1H), 7.49 (d, *J* = 1.9 Hz, 1H), 7.40 (dd, *J* = 8.3, 1.8 Hz, 1H), 7.04 (d, *J* = 8.4 Hz, 1H), 4.09 (q, *J* = 7.0 Hz, 2H), 3.82 (s, 3H), 1.35 (t, *J* = 7.0 Hz, 3H).

### Step 2, synthesis of compound 12C

Under a nitrogen atmosphere, a solution of lithium aluminum hydride in tetrahydrofuran (60 mL, 1 mol/L) was added to a three-necked flask. A solution of compound 12B (6.0 g) dissolved in tetrahydrofuran (60 mL) was slowly added dropwise to the three-necked flask at room temperature. After the addition was completed, the reaction mixture was heated to 65 °C and stirred for 3 hours. After the reaction was completed, the mixture was cooled to room temperature and then water (6 mL), 15% aqueous sodium hydroxide solution (6 mL), and water (18 mL) were slowly added in sequence. An appropriate amount of anhydrous sodium sulfate was added, and the mixture was stirred for 20 minutes. The mixture was filtered, and the filtrate was collected. The filter cake was stirred in a dichloromethane/methanol (10/1) mixture for 20 minutes and filtered. The filtrates were combined and concentrated under reduced pressure, and the resulting crude product was purified by silica gel column chromatography (dichloromethane/methanol = 93/7), and compound 12C (3.8 g) was yielded. MS m/z (ESI): 196.0[M+1]⁺.

### Step 3, synthesis of compound 12D

Compound 12C (2.2 g) was dissolved in dichloroethane (20 mL), and added with trimethylsilyl isocyanate (3.9 g). The reaction mixture was stirred at 80 °C for 16 hours. After the reaction was completed, the mixture was cooled to room temperature and concentrated under reduced pressure. The resulting crude product was purified by silica gel column chromatography (dichloromethane/methanol = 10/1), and compound 12D (2.0 g) was yielded. MS m/z (ESI): 239.1[M+1]⁺.

### Step 4, synthesis of compound 12E

A solution of sodium ethoxide in ethanol (20%, 14.0 g) was added to anhydrous ethanol (20 mL), and added with compound 12D (2.0 g) and diethyl malonate (3.4 g) in sequence. The reaction mixture was reacted at 80°C for 16 hours. After the reaction was completed, the mixture was cooled to room temperature and concentrated under reduced pressure to remove ethanol. The residue was dissolved with water, and hydrochloric acid (2 M) was added dropwise in an ice bath to adjust the pH to 2 until solid precipitated. The precipitated solid was collected by filtration and dried, and compound 12E (1.5 g) was yielded. MS m/z (ESI): 307.1[M+1]⁺.

### Step 5, synthesis of compound 12F

Compound 12E (1.5 g) was added to phosphorus oxychloride (20 mL). Under a nitrogen atmosphere, the reaction mixture was stirred at 85°C for 16 hours of reaction. After the reaction was completed, the mixture was cooled to room temperature and concentrated under reduced pressure to remove phosphorus oxychloride. The resulting residue was dissolved in dichloromethane (50 mL). The organic phase was washed with ice water (50 mL × 2), dried by anhydrous sodium sulfate and concentrated, and compound 12F (1.0 g) was yielded. MS m/z (ESI): 307.1[M+1 ]⁺.

### Step 6, synthesis of compound 12G

Compound 12F (1.0 g) was dissolved in isopropyl alcohol (20 mL), and 2,4,6-trimethylaniline (2.2 g) was added. The reaction solution was reacted at 90 °C for 16 hours. After the reaction was completed, the reaction solution was concentrated. The resulting crude product was purified by silica gel column chromatography (dichloromethane/methanol = 10/1), and compound 12G (1.0 g) was yielded. MS m/z (ESI): 406.1[M+1]⁺.

### Step 7, synthesis of compound 12H

Compound 12G (1.0 g) was added to toluene (10 mL), and added with tert-butyl (2-hydroxyethyl)carbamate (0.6 g) and cyanomethylene tri-n-butylphosphorane (1.8 g). Under a nitrogen atmosphere, the reaction mixture was stirred at 120 °C for 2 hours. After the reaction was completed, the reaction mixture was concentrated. The resulting crude product was purified by silica gel column chromatography (dichloromethane/methanol = 10/1), and compound 12H (0.5 g) was yielded. MS m/z (ESI): 549.1[M+1 ]⁺.

### Step 8, synthesis of compound 12l

Compound 12H (200 mg) was added to a 4M hydrochloric acid/dioxane solution (5 mL). The reaction mixture was stirred at 25°C for 1 hour. After the reaction was completed, the reaction mixture was concentrated, and compound 121 (160 mg) was yielded. MS m/z(ESI): 449.1[M+1]⁺.

### Step 9, synthesis of compound 12

Compound 121 (200 mg), phenyl carbamate (92 mg), and triethylamine (226 mg) were added to dichloromethane (5 mL). The reaction mixture was stirred at 25 °C for 16 hours. After the reaction was completed, the reaction mixture was concentrated. The resulting crude product was purified by Flash reversed-phase chromatography, and compound 12 (20 mg) was yielded. MS m/z(ESI): 492.1[M+1]⁺.

¹H NMR (400 MHz, DMSO-d₆) δ 6.96 (s, 1H), 6.85 (s, 2H), 6.66 (s, 1H), 6.10 (t, *J =* 5.8 Hz, 1H), 5.45 (s, 2H), 5.29 (s, 1H), 4.18 (t, *J* = 6.7 Hz, 2H), 3.92 (t, *J* = 6.0 Hz, 2H), 3.87-3.82 (m, 2H), 3.80 (s, 3H), 3.35 (d, *J* = 6.6 Hz, 2H), 2.90 (t, *J* = 5.9 Hz, 2H), 2.22 (s, 3H), 1.97 (s, 6H), 1.22 (t, *J* = 6.9 Hz, 3H).

### Example 12: synthesis of compound 13

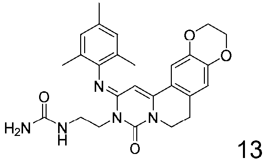

Compound 13 (150 mg) was yielded by referring to the synthesis method of Example 11. MS m/z(ESI): 476.2[M+1]⁺.

¹H NMR (400 MHz, DMSO-d₆) δ 11.57 (s, 1H), 7.10 (s, 2H), 7.05 (s, 1H), 6.96 (s, 1H), 6.60 (s, 1H), 6.06 (s, 2H), 5.61 (s, 1H), 4.33-4.30 (m, 2H), 4.29-4.22 (m, 4H), 4.06 (t, *J* = 6.3 Hz, 2H), 3.39 (d, *J* = 5.8 Hz, 2H), 2.94 (t, *J* = 6.2 Hz, 2H), 2.33 (s, 3H), 2.19 (s, 6H).

### Example 13: synthesis of compound 14

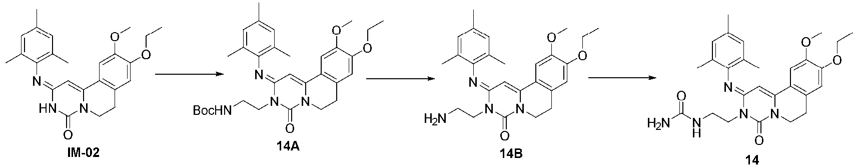

Compound 14 (20 mg) was yielded by referring to the synthesis method of Example 11. MS m/z(ESI): 492.1[M+1]⁺.

¹H NMR (400 MHz, DMSO-d₆) δ 6.94 (s, 1H), 6.85 (s, 2H), 6.66 (s, 1H), 6.10 (t, *J* = 5.9 Hz, 1H), 5.44 (s, 2H), 5.31 (s, 1H), 4.18 (t, *J* = 6.8 Hz, 2H), 4.06 (q, *J* = 6.9 Hz, 2H), 3.91 (t, *J* = 6.0 Hz, 2H), 3.62 (s, 3H), 3.39-3.34 (m, 2H), 2.89 (t, *J* = 6.0 Hz, 2H), 2.22 (s, 3H), 1.97 (s, 6H), 1.33 (t, *J* = 7.0 Hz, 3H).

### Example 14: synthesis of compound 15

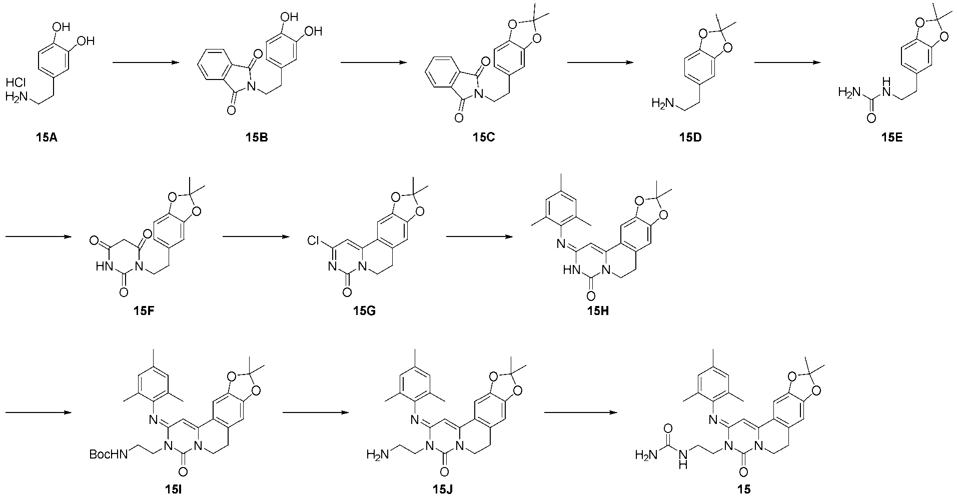

### Step 1, synthesis of compound 15B

Compound 15A (10.0 g) and ethyl 1,3-dioxindole-2-carboxylate (11.6 g) were dissolved in methanol (10 mL), and slowly added with triethylamine (2.2 g). The reaction mixture was stirred at 25 °C for 12 hours. After the reaction was completed, the reaction mixture was concentrated. The crude product was purified by Flash reversed-phase chromatography, and compound 15B (9.0 g) was yielded. MS m/z (ESI): 284.2[M+1]⁺.

### Step 2, synthesis of compound 15C

Compound 15B (10.0 g) and 2,2-dimethoxypropane (14.7 g) were dissolved in toluene (100 mL). Under nitrogen protection, the reaction was carried out at 120 °C for 5 minutes. Then, p-toluenesulfonic acid (0.3 g) was slowly added, and the mixture was stirred at 120 °C for 2 hours. The reaction mixture was cooled to room temperature and concentrated. The crude product was purified by silica gel column chromatography (petroleum ether/ethyl acetate = 3/1), and compound 15C (8.0 g) was yielded. MS m/z (ESI): 324.1[M+1]⁺.

### Step 3, synthesis of compound 15D

Compound 15C (8.0 g) was dissolved in a mixed solvent of dichloromethane/methanol (v/v = 1/1, 100 mL), and dropwise added with hydrazine hydrate (12.4 g). After the addition was completed, the reaction mixture was stirred at 25°C for 72 hours. After the reaction was completed, the mixture was filtered, and the filtrate was concentrated to yield compound 15D (5.0 g). MS m/z(ESI): 194.1[M+1]⁺.

### Step 4, synthesis of compound 15E

Compound 15D (5.0 g) was dissolved in dichloroethane (50 mL), and added with trimethylsilyl isocyanate (8.9 g). The reaction mixture was stirred at 80°C for 16 hours. After the reaction was completed, the mixture was cooled to room temperature and concentrated under reduced pressure. The crude product was purified by silica gel column chromatography (dichloromethane/methanol = 10/1), and compound 15E (4.0 g) was yielded. MS m/z (ESI): 237.1[M+1]⁺.

### Step 5, synthesis of compound 15F

A solution of sodium ethoxide in ethanol (20%, 28.7 g) was added to anhydrous ethanol (50 mL), and added with compound 15E (4.0 g) and diethyl malonate (6.8 g) in sequence. The reaction mixture was reacted at 80 °C for 16 hours. After the reaction was completed, the mixture was cooled to room temperature and concentrated under reduced pressure to remove ethanol. The residue was dissolved with water, and hydrochloric acid (2 M) was added dropwise in an ice bath to adjust the pH until solid precipitated. The precipitated solid was collected by filtration and dried, and compound 15F (2.5 g) was yielded. MS m/z (ESI): 305.1[M+1]⁺.

### Step 6, synthesis of compound 15G

Compound 15F (2.4 g) was added to phosphorus oxychloride (20 mL). Under a nitrogen atmosphere, the reaction mixture was stirred at 85°C for 16 hours. After the reaction was completed, the mixture was cooled to room temperature and concentrated under reduced pressure to remove phosphorus oxychloride. The resulting residue was dissolved in dichloromethane (50 mL). The organic phase was washed with ice water (50 mL × 2), dried over anhydrous sodium sulfate, and concentrated, and compound 15G (2.4 g) was yielded. MS m/z (ESI): 305.0[M+1]⁺.

### Step 7, synthesis of compound 15H

Compound 15G (1.0 g) was dissolved in isopropyl alcohol (20 mL), and added with 2,4,6-trimethylaniline (2.2 g). The reaction mixture was reacted at 90°C for 16 hours. After the reaction was completed, the mixture was concentrated. The resulting crude product was purified by silica gel column chromatography (dichloromethane/methanol = 10/1) and compound 15H (0.8 g) was yielded. MS m/z (ESI): 404.2[M+1]⁺.

### Step 8, synthesis of compound 15l

Compound 15H (500 mg) was added to toluene (10 mL), and added with tert-butyl (2-hydroxyethyl)carbamate (300 mg) and cyanomethylene tri-n-butylphosphorane (897 mg). Under a nitrogen atmosphere, the reaction mixture was stirred at 120 °C for 2 hours. After the reaction was completed, the reaction mixture was concentrated. The resulting crude product was purified by silica gel column chromatography (dichloromethane/methanol = 10/1), and compound 151 (400 mg) was yielded. MS m/z (ESI): 547.2[M+1]⁺.

### Step 9, synthesis of compound 15J

Compound 151 (200 mg) was added to a 4M hydrochloric acid/dioxane solution (5 mL). The reaction mixture was stirred at 25°C for 1 hour. After the reaction was completed, the reaction mixture was concentrated, and compound 15J (200 mg) was yielded. MS m/z (ESI): 447.2[M+1]⁺.

### Step 10, synthesis of compound 15

Compound 15J (150 mg), phenyl carbamate (69 mg), and triethylamine (170 mg) were added to dichloromethane (5 mL). The reaction mixture was stirred at 25 °C for 16 hours. After the reaction was completed, the reaction mixture was concentrated. The resulting crude product was purified by Flash reversed-phase chromatography, and compound 15 (30 mg) was yielded. MS m/z (ESI): 490.2[M+1]⁺.

¹H NMR (400 MHz, DMSO-d₆) δ 6.84 (d, *J* = 6.2 Hz, 3H), 6.66 (s, 1H), 6.10 (t, *J* = 5.8 Hz, 1H), 5.44 (s, 2H), 5.26 (s, 1H), 4.17 (t, *J* = 6.8 Hz, 2H), 3.89 (t, *J* = 6.0 Hz, 2H), 3.35 (d, *J* = 7.0 Hz, 2H), 2.85 (t, *J* = 6.0 Hz, 2H), 2.23 (s, 3H), 1.95 (s, 6H), 1.61 (s, 6H).

### Example 15: synthesis of compound 16

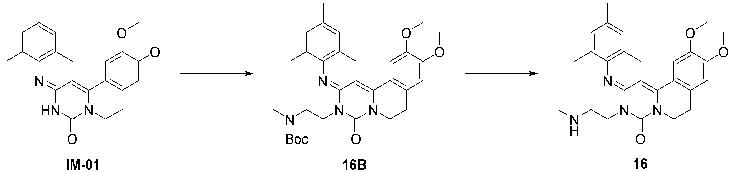

### Step 1, synthesis of compound 16B

Compound IM-01 (500 mg) was added to toluene (10 mL), and added with tert-butyl (2-hydroxyethyl)(methyl)carbamate (338 mg) and cyanomethylene tri-n-butylphosphorane (1.50 g). Under a nitrogen atmosphere, the reaction mixture was stirred at 120 °C for 4 hours. After the reaction was completed, the reaction mixture was concentrated to yield a crude product. The crude product was purified by silica gel column chromatography (dichloromethane/methanol = 10/1), and compound 16B (250 mg) was yielded. MS m/z (ESI): 549.1[M+1]⁺.

### Step 2, synthesis of compound 16

Compound 16B (200 mg) was added to a 4M hydrochloric acid/dioxane solution (2 mL). The reaction mixture was stirred at 25°C for 1 hour. After the reaction was completed, the reaction mixture was concentrated, and compound 16 (170 mg) was yielded. MS m/z (ESI): 449.0[M+1]⁺.

¹H NMR (400 MHz, DMSO-d₆) δ 7.05 (d, *J* = 3.3 Hz, 3H), 6.76 (s, 1H), 5.54 (s, 1H), 4.57-4.51 (m, 2H), 4.03 (t, *J* = 6.3 Hz, 2H), 3.83 (s, 3H), 3.63 (s, 3H), 3.35 -3.29 (m, 2H), 2.98 (t, *J* = 6.3 Hz, 2H), 2.66 (s, 3H), 2.29 (s, 3H), 2.13 (s, 6H).

### Example 16: synthesis of compound 17 and compound 18

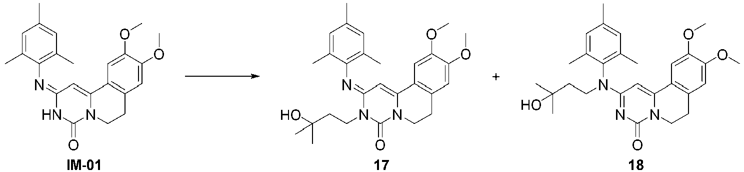

IM-01 (200 mg), 4-bromo-2-methylbutan-2-ol (470 mg), potassium carbonate (635 mg), and potassium iodide (509 mg) were added to 2-butanone (10 mL). The reaction mixture was stirred at 85°C for 16 hours. After the reaction was completed, the solvent was removed under reduced pressure. The crude product was purified by silica gel column chromatography (dichloromethane/methanol = 20/1), and compound 17 (15 mg) and compound 18 (60 mg) were yielded.

Compound 17, MS m/z (ESI): 478.3[M+1]⁺.

¹H NMR (400 MHz, DMSO-d₆) δ 6.95 (s, 1H), 6.85 (s, 2H), 6.66 (s, 1H), 5.32 (s, 1H), 4.36 (s, 1H), 4.27-4.17 (m, 2H), 3.91 (t, J = 5.8 Hz, 2H), 3.80 (s, 3H), 3.61 (s, 3H), 2.90 (t, J = 5.8 Hz, 2H), 2.22 (s, 3H), 1.96 (s, 6H), 1.85-1.75 (m, 2H), 1.17 (s, 6H).

Compound 18, MS m/z (ESI): 478.2[M+1]⁺.

¹H NMR (400 MHz, CDCl3) δ 6.99 (s, 2H), 6.69 (s, 1H), 6.63 (s, 1H), 5.32 (s, 1H), 4.14 (t, J = 6.4 Hz, 2H), 4.01-3.95 (m, 2H), 3.90 (s, 3H), 3.72 (s, 3H), 2.89 (t, J = 6.3 Hz, 2H), 2.33 (s, 3H), 2.16 (s, 6H), 1.97-1.89 (m, 2H), 1.25 (s, 6H).

### Example 17: synthesis of compound 19

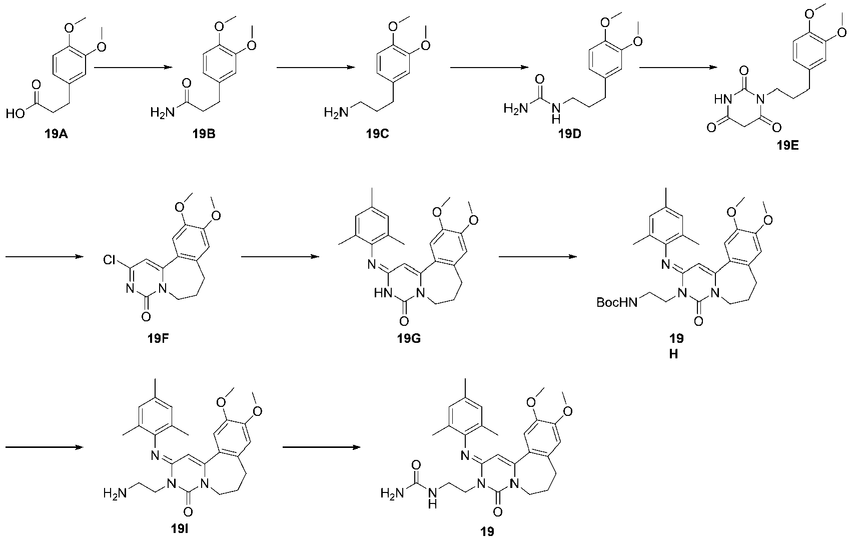

### Step 1, synthesis of compound 19B

Compound 19A (10.0 g) was dissolved in tetrahydrofuran (175 mL), and added with triethylamine (7.3 mL). The resulting mixture was cooled to -10°C, and ethyl chloroformate (5.0 mL) was added dropwise. After the reaction mixture was stirred at - 10°C for 20 minutes, a solution of concentrated aqueous ammonia (105 mL) in tetrahydrofuran (105 mL) was added. The reaction mixture was further stirred at -10°C for 30 minutes and then was stirred at room temperature for 90 minutes. After the reaction was completed, the mixture was concentrated under reduced pressure, and the residue was extracted with dichloromethane (100 mL × 3). The organic phases were combined, washed with saturated aqueous sodium bicarbonate solution and saline, dried over anhydrous magnesium sulfate, filtered, and concentrated under reduced pressure and compound 19B (9.0 g) was yielded. MS m/z (ESI): 210.1[M+1]⁺.

### Step 2, synthesis of compound 19C

Under a nitrogen atmosphere, lithium aluminum hydride (1.81 g) was added into a three-necked flask, and anhydrous tetrahydrofuran (80 mL) was slowly added. Compound 19B (5.0 g) was dissolved in anhydrous tetrahydrofuran (180 mL), and the solution was added dropwise into the three-necked flask at 0°C. After the addition was completed, the reaction mixture was heated to 65°C and stirred for 2 hours. After the reaction was completed, the mixture was cooled to room temperature, and then slowly added with water (1.8 mL), 15% aqueous sodium hydroxide solution (1.8 mL), and water (5.4 mL) in sequence. An appropriate amount of anhydrous sodium sulfate was added and the mixture was stirred for 20 minutes. The mixture was filtered to collect the filtrate. The filter cake was stirred in a dichloromethane/methanol (10/1) mixed solvent for 20 minutes and was filtered again. The filtrates were combined and concentrated under reduced pressure. The resulting crude product was purified by silica gel column chromatography (dichloromethane/methanol = 24/1), and compound 19C (3.5 g) was yielded. MS m/z (ESI): 196.1[M+1]⁺.

### Step 3, synthesis of compound 19D

Compound 19C (2.0 g) was dissolved in 1,2-dichloroethane (30 mL), and trimethylsilyl isocyanate (2.94 g) was added. The reaction mixture was stirred at 85°C for 16 hours. After the reaction was completed, the mixture was cooled to room temperature and was concentrated under reduced pressure. The resulting crude product was purified by silica gel column chromatography (dichloromethane/methanol = 20/1), and compound 19D (2.1 g) was yielded. MS m/z(ESI): 239.1 [M+1 ]⁺.

### Step 4, synthesis of compound 19E

A solution of sodium ethoxide in ethanol (20%, 14.0 g) was added to anhydrous ethanol (20 mL), and added with compound 19D (2.0 g) and diethyl malonate (3.4 g) in sequence. The reaction mixture was reacted at 80°C for 16 hours under a nitrogen atmosphere. After the reaction was completed, the mixture was cooled to room temperature and was concentrated under reduced pressure to remove ethanol. The residue was dissolved in water, and hydrochloric acid (2 M) was added dropwise in an ice bath to adjust the pH until a solid precipitated. The precipitated solid was collected by filtration, and dried, and compound 19E (1.5 g) was yielded. MS m/z (ESI): 307.2[M+1]⁺.

### Step 5, synthesis of compound 19F

Compound 19E (1.4 g) was added to phosphorus oxychloride (20 mL). Under a nitrogen atmosphere, the reaction mixture was stirred at 80°C for 16 hours. After the reaction was completed, the mixture was cooled to room temperature. Phosphorus oxychloride was removed under reduced pressure, and the resulting residue was dissolved in dichloromethane (50 mL). The organic phase was washed with ice water (50 mL × 2), dried over anhydrous sodium sulfate, and concentrated under reduced pressure, and compound 19F (1.4 g) was yielded. MS m/z (ESI): 307.1[M+1]⁺.

### Step 6, synthesis of compound 19G

Compound 19F (1.4 g) was dissolved in isopropyl alcohol (20 mL), and 2,4,6-trimethylaniline (1.85 g) was added. The reaction mixture was reacted at 90°C for 16 hours under a nitrogen atmosphere. After the reaction was completed, the mixture was concentrated. The resulting crude product was purified by silica gel column chromatography (dichloromethane/methanol = 14/1), and compound 19G (160 mg) was yielded. MS m/z (ESI): 406.1[M+1]⁺.

### Step 7, synthesis of compound 19H

Compound 19G (140 mg) was added to toluene (5 mL), and added with tert-butyl (2-hydroxyethyl)carbamate (84 mg) and cyanomethylene tri-n-butylphosphorane (250 mg). Under a nitrogen atmosphere, the reaction mixture was stirred at 120°C for 16 hours. After the reaction was completed, the mixture was concentrated. The resulting crude product was purified by silica gel column chromatography (dichloromethane/methanol = 24/1), and compound 19H (140 mg) was yielded. MS m/z (ESI): 549.3[M+1]⁺.

### Step 8, synthesis of compound 19l

At room temperature, compound 19H (140 mg) was dissolved in a solution of hydrogen chloride in 1,4-dioxane (4 M, 2 mL). The reaction mixture was stirred at room temperature for 4 hours. After the reaction was completed, the reaction mixture was concentrated under reduced pressure, and compound 191 (125 mg) was yielded. MS m/z (ESI): 449.1[M+1]⁺.

### Step 9, synthesis of compound 19

Compound 191 (100 mg), phenyl carbamate (46 mg), and 39rimethylamine (113 mg) were added to dichloromethane (2.5 mL). The mixture was stirred at 25°C for 4 hours. After the reaction was completed, the mixture was concentrated under reduced pressure. The resulting crude product was purified by Flash reversed-phase chromatography, and compound 19 (20 mg) was yielded. MS m/z (ESI): 492.3[M+1 ]⁺.

¹H NMR (400 MHz, DMSO-d₆) δ 6.93 (s, 1H), 6.81 (s, 2H), 6.68 (d, J = 1.5 Hz, 1H), 6.09 (t, J = 5.3 Hz, 1H), 5.45 (s, 2H), 4.93 (d, J = 1.6 Hz, 1H), 4.19 (t, J = 6.2 Hz, 2H), 3.78 (d, J = 1.5 Hz, 3H), 3.66 (d, J = 1.6 Hz, 3H), 3.37 (s, 2H), 3.32 (d, J = 2.1 Hz, 4H), 2.67 (s, 2H), 2.18 (s, 3H), 1.98 (s, 6H).

### Example 18: synthesis of compound 20

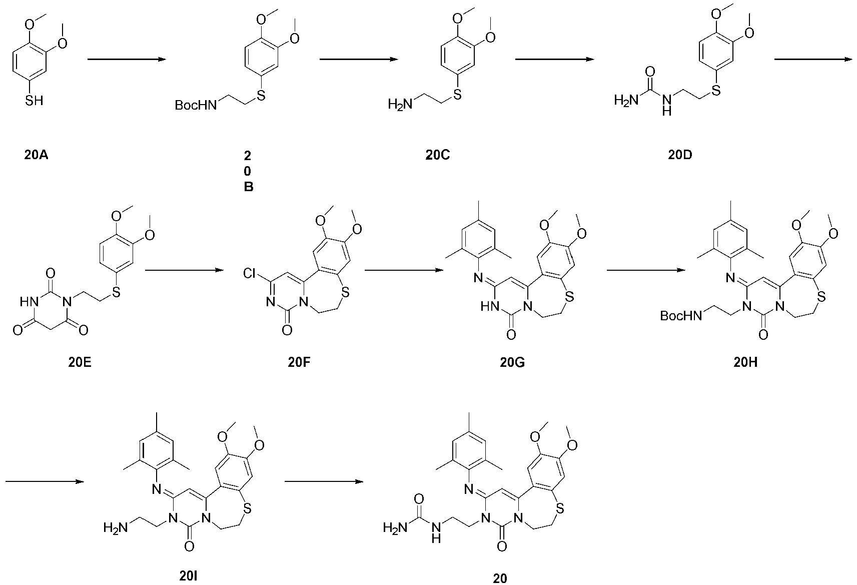

### Step 1, synthesis of compound 20B

Sodium hydride (0.85 g) was added by portion to a solution of compound 20A (4 g) in tetrahydrofuran (130 mL) at 0°C. After the addition was completed, the mixture was stirred at room temperature for 30 minutes, and tert-butyl (2-bromoethyl)carbamate (8.4 g) was added. The reaction mixture was heated to 40°C and reacted for 3 hours. After the reaction was completed, the mixture was poured into ice water and extracted with ethyl acetate (100 mL × 3). The organic phases were combined, washed with saturated saline, dried over anhydrous sodium sulfate, and concentrated to yield a crude product. The crude product was purified by silica gel column chromatography (petroleum ether/ethyl acetate = 4/1), and compound 20B (7.9 g) was yielded. MS m/z (ESI): 314.0 [M+1]⁺.

### Step 2, synthesis of compound 20C

Compound 20B (7.9 g) was dissolved in dichloromethane (80 mL), and trifluoroacetic acid (26 mL) was added dropwise slowly. After the addition was completed, the mixture was stirred at room temperature for 1 hour. After the reaction was completed, the reaction mixture was concentrated, and compound 20C (9.5 g) was yielded. MS m/z (ESI): 213.7 [M+1]⁺.

### Step 3, synthesis of compound 20D

Compound 20C (9.0 g) was dissolved in 1,2-dichloroethane (250 mL), trimethylsilyl isocyanate (16.6 g) was added, and the system was replaced with nitrogen. The reaction mixture was heated to 90°C and reacted for 4 hours. After the reaction was completed, the mixture was cooled to room temperature and concentrated to yield a crude product. The crude product was slurried with methyl tert-butyl ether and compound 20D (3.7 g) was yielded. MS m/z (ESI): 256.9 [M+1]⁺.

### Step 4, synthesis of compound 20E

Compound 20D (3.0 g) was dissolved in ethanol (80 mL), and added with diethyl malonate (4.7 g) and sodium ethoxide (4.0 g). Under a nitrogen atmosphere, the mixture was heated under reflux for 18 hours of reaction. After the reaction was completed, the reaction mixture was cooled to room temperature, concentrated, and dissolved with water, and the pH was adjusted to 5 with dilute hydrochloric acid. The mixture was filtered, and the filtrate was extracted with ethyl acetate. The organic phases were combined, washed with saturated saline, dried over anhydrous sodium sulfate, and concentrated, and compound 20E (1.0 g) was yielded. MS m/z (ESI): 324.9 [M+1]⁺.

### Step 5, synthesis of compound 20F

Compound 20E (700 mg) was dissolved in phosphorus oxychloride (37 mL) and reacted at 70°C for 2 hours. After the reaction was completed, the reaction mixture was cooled to room temperature, dropwise added into ice water to quench the reaction, and extracted with ethyl acetate. The organic phases were combined, washed with saturated saline, dried over anhydrous sodium sulfate, and evaporated to dryness to yield a crude product. The crude material was purified by silica gel plate chromatography (petroleum ether/ethyl acetate = 1/1), and compound 20F (230 mg) was yielded. MS m/z (ESI): 324.8 [M+1]⁺.

### Step 6, synthesis of compound 20G

Compound 20F (220 mg) and 2,4,6-trimethylaniline (458 mg) were dissolved in propylene glycol (7 mL) and reacted at 90°C for 10 hours. After the reaction was completed, the mixture was cooled to room temperature and concentrated to yield a crude product. The crude product was purified by silica gel column chromatography (dichloromethane/methanol = 10/1), and compound 20G (211 mg) was yielded. MS m/z (ESI): 424.2 [M+1]⁺.

### Step 7, synthesis of compound 20H

Compound 20G (210 mg), tert-butyl (2-hydroxyethyl)carbamate (104 mg), and cyanomethylene tri-n-butylphosphorane (359 mg) were dissolved in toluene (5 mL). after the reaction mixture was replaced with nitrogen, heated to 120°C and reacted for 4 hours. The reaction mixture was cooled to room temperature and concentrated to yield a crude product. The crude product was purified by silica gel column chromatography (dichloromethane/methanol = 10/1), and compound 20H (235 mg) was yielded. MS m/z (ESI): 567.2 [M+1]⁺.

### Step 8, synthesis of compound 20l

Compound 20H (235 mg) was dissolved in a solution of hydrochloride in 1,4-dioxane (4 M, 2.5 mL) and reacted at 25°C for 2 hours. After the reaction was completed, the reaction mixture was concentrated, and compound 201 (239 mg) was yielded. MS m/z (ESI): 467.2 [M+1]⁺.

### Step 9, synthesis of compound 20

Compound 20l (200 mg) was dissolved in dichloromethane (3 mL), phenyl carbamate (59 mg) and triethylamine (130 mg) were added. The reaction mixture was replaced with nitrogen, and reacted at 25°C for 3 hours. After the reaction was completed, the mixture was concentrated to yield a crude product. The crude product was purified by silica gel column chromatography (dichloromethane/methanol = 10/1), and compound 20 (150 mg) was yielded. MS m/z (ESI): 509.9 [M+1]⁺.

¹H NMR (400 MHz, DMSO-d₆) δ 7.11 (s, 1H), 6.85 (s, 1H), 6.80 (s, 1H), 6.76 (s, 1H), 6.11 (t, J = 5.7 Hz, 1H), 5.46 (s, 2H), 4.94 (s, 1H), 4.64-4.57 (m, 1H), 4.20 (q, J = 6.5 Hz, 2H), 3.79 (s, 3H), 3.71 (s, 3H), 3.36 (q, J = 6.4 Hz, 2H), 3.24-2.96 (m, 4H), 2.17 (s, 3H), 2.05 (s, 3H), 1.90 (s, 3H).

### Example 19: synthesis of compound 21

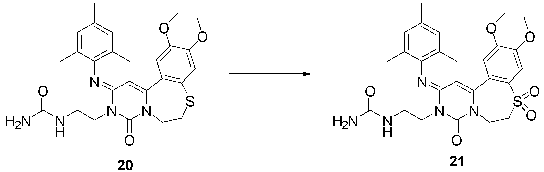

Compound 20 (50 mg) was dissolved in dichloromethane (1 mL), and m-chloroperoxybenzoic acid (101 mg) was added at 0°C. The mixture was heated to room temperature and stirred for 1 hour. After the reaction was completed, the mixture was concentrated to yield a crude product. The crude product was purified by Flash reversed-phase column chromatography, and compound 21 (10 mg) was yielded. MS m/z(ESI): 541.9 [M+1]⁺.

¹H NMR (400 MHz, DMSO-d₆) δ 7.41 (s, 1H), 6.97 (s, 2H), 6.88 (s, 1H), 6.35 (s, 1H), 5.75 (s, 2H), 5.43 (s, 1H), 4.71 (d, J = 9.6 Hz, 1H), 4.31 (s, 1H), 4.21 (s, 2H), 3.90 (s, 3H), 3.84 (s, 3H), 3.37 (s, 2H), 3.17 (s, 2H), 2.25 - 2.13 (m, 6H), 1.96 (s, 3H).

### Example 20: synthesis of compound 22

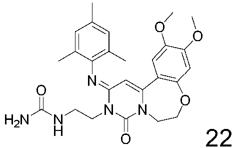

Compound 22 (57 mg) was yielded referring to the synthetic method described in Example 19. MS m/z (ESI): 494.3[M+1]⁺.

¹H NMR (400 MHz, DMSO-d₆) δ 7.06 (s, 2H), 6.85 (s, 1H), 6.68 (s, 1H), 6.57 (s, 1H), 6.03 (s, 2H), 5.42 (s, 1H), 4.42 (d, J = 5.0 Hz, 2H), 4.28-4.24 (m, 2H), 4.22 (d, J = 4.9 Hz, 2H), 3.82 (s, 3H), 3.67 (s, 3H), 3.40 (d, J = 6.1 Hz, 2H), 2.29 (s, 3H), 2.20 (s, 6H).

### Example 21: synthesis of compound 23

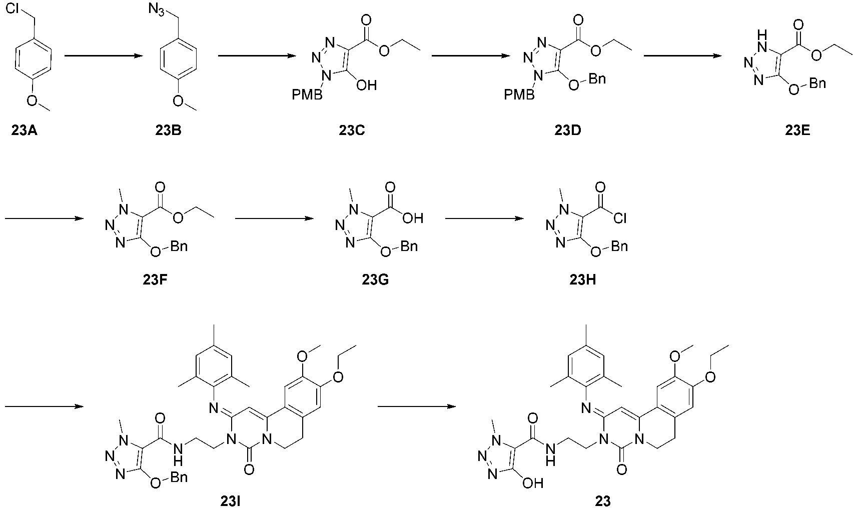

### Step 1, synthesis of compound 23B

Compound 23A (10.0 g) was dissolved in N,N-dimethylformamide (100 mL), and sodium azide (4.6 g) was added slowly. The reaction mixture was stirred at 25°C for 16 hours. After the reaction was completed, water (200 mL) was added, and the mixture was extracted with ethyl acetate (100 mL × 3). The organic phases were combined, washed with saturated saline (50 mL), dried over anhydrous sodium sulfate, and concentrated, and compound 23B (11 g) was yielded.

### Step 2, synthesis of compound 23C

A solution of sodium ethoxide in ethanol (20%, 104 g) was added to absolute ethanol (100 mL), and added with compound 23B (10.0 g) and diethyl malonate (25.0 g). The reaction mixture was heated to 80°C and reacted for 16 hours. After the reaction was completed, the mixture was cooled to room temperature and concentrated under reduced pressure to remove ethanol. The residue was dissolved in water, and dilute hydrochloric acid (2 M) was added dropwise in ice bath to adjust pH to 2. The resulting precipitated solid was filtered, and dried, and compound 23C (12 g) was yielded. MS m/z (ESI): 278.1[M+1]⁺.

### Step 3, synthesis of compound 23D

Compound 23C (12.0 g) was added in N,N-dimethylformamide (120 mL), and added with benzyl bromide (11.1 g) and potassium carbonate (12.0 g). The reaction mixture was reacted under stirring at 25°C for 16 hours. After the reaction was completed, water (200 mL) was added, and the mixture was extracted with ethyl acetate (100 mL × 3). The organic phases were combined, washed with saturated saline (50 mL), dried over anhydrous sodium sulfate, and concentrated, and compound 23D (10 g) was yielded. MS m/z (ESI): 368.1[M+1]⁺.

### Step 4, synthesis of compound 23E

Compound 23D (10.0 g) was added slowly into trifluoroacetic acid /trifluoromethanesulfonic acid (V/V = 4/1, 25 mL) and stirred at 90°C for 2 hours. After the reaction was completed, the reaction mixture was cooled to room temperature and concentrated under reduced pressure to remove the solvent. The crude product was purified by Flash reversed-phase column chromatography, and compound 23E (3.0 g) was yielded. MS m/z (ESI): 248.1[M+1]⁺.

### Step 5, synthesis of compound 23F

Compound 23E (3.0 g) was dissolved in acetonitrile (30 mL), and added with iodomethane (2.6 g) and potassium carbonate (3.3 g). The reaction mixture was stirred at 25°C for 16 hours. After the reaction was completed, the mixture was filtered. The filtrate was concentrated, and compound 23F (2.6 g) was yielded. MS m/z (ESI): 262.1[M+1]⁺.

### Step 6, synthesis of compound 23G

Compound 23F (2.6 g) was added to a solution of sodium hydroxide in ethanol (5 M, 20 mL). The reaction mixture was stirred at 25°C for 2 hours. After the reaction was completed, the mixture was concentrated under reduced pressure and diluted with water. The pH was adjusted to 2 with 1 M dilute hydrochloric acid. The resulting solution was purified by Flash reversed-phase column chromatography, and compound 23G (800 mg) was yielded. MS m/z (ESI): 234.1[M+1]⁺.

### Step 7, synthesis of compound 23H

Compound 23G (50 mg) and a small amount of N,N-dimethylformamide were dissolved in dichloromethane (5 mL), and oxalyl chloride (32 mg) was added dropwise at 0°C. The reaction mixture was reacted under stirring at 25°C for 2 hours. After the reaction was completed, the mixture was concentrated, and compound 23H (60 mg) was yielded.

### Step 8, synthesis of compound 23l

Compound 23H (60 mg), IM-02 (107 mg), and triethylamine (48 mg) were added to dichloromethane (5 mL), and the mixture was stirred at 25°C for 16 hours. After the reaction was completed, the reaction mixture was concentrated to yield a crude product. The crude product was purified by silica gel column chromatography (dichloromethane/methanol = 10/1), and compound 231 (70 mg) was yielded. MS m/z (ESI): 664.3[M+1]⁺.

### Step 9, synthesis of compound 23

Compound 23l (70 mg), palladium on carbon (50 mg), and palladium hydroxide on carbon (50 mg) were added to methanol (10 mL). Under a hydrogen atmosphere, the mixture was reacted under stirring at 30°C for 2 hours. After the reaction was completed, the mixture was filtered, and the filtrate was concentrated. The resulting crude product was purified by Flash reversed-phase chromatography, and compound 23 (5 mg) was yielded. MS m/z (ESI): 574.3[M+1]⁺.

¹H NMR (400 MHz, DMSO-d₆) δ 14.65 (s, 1H), 8.32 (s, 1H), 7.22 (s, 1H), 7.10 (s, 2H), 6.97 (s, 1H), 6.77 (s, 1H), 5.57 (s, 1H), 4.41 (s, 2H), 4.12 (d, J = 6.5 Hz, 2H), 4.01 (s, 1H), 3.90 (s, 2H), 3.68 (s, 1H), 3.64 (s, 2H), 2.95 (s, 1H), 2.31 (s, 3H), 2.16 (s, 5H), 1.35 (t, J = 6.9 Hz, 3H).

### Example 22: synthesis of compound 24

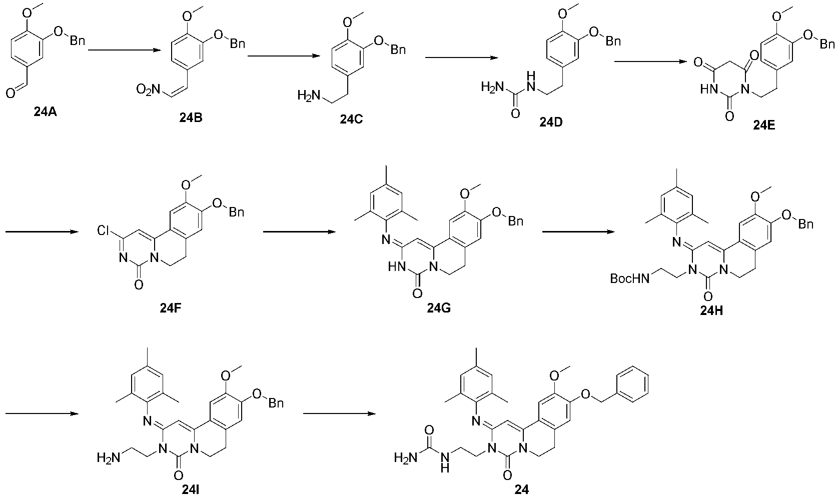

Compound 24 (15 mg) was yielded referring to the synthetic method described in Example 11. MS m/z (ESI): 554.2[M+1]⁺.

¹H NMR (400 MHz, DMSO-d₆) δ 7.46 - 7.33 (m, 5H), 7.08 (s, 1H), 6.86 (s, 2H), 6.70 (s, 1H), 6.10 (t, J = 5.6 Hz, 1H), 5.44 (s, 2H), 5.33 (s, 1H), 5.14 (s, 2H), 4.18 (t, J = 6.6 Hz, 2H), 3.92 (t, J = 5.7 Hz, 2H), 3.63 (s, 3H), 3.35 (d, J = 6.4 Hz, 2H), 2.88 (s, 2H), 2.22 (s, 3H), 1.97 (s, 6H).

### Example 23: synthesis of compound 25

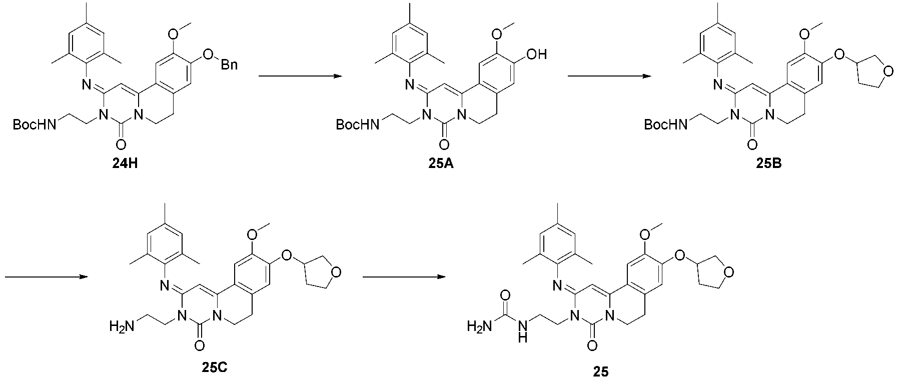

### Step 1, synthesis of compound 25A

Compound 24H (600 mg) was dissolved in methanol (10 mL), and palladium on carbon (100 mg) was subsequently added. Under a hydrogen atmosphere, the reaction mixture was stirred at room temperature for 16 hours. After the reaction was completed, the mixture was filtered, and the filtrate was concentrated under reduced pressure to yield a crude product. The crude product was purified by silica gel column chromatography (dichloromethane/methanol = 20/1), and compound 25A (500 mg) was yielded. MS m/z (ESI): 521.3[M+1]⁺.

### Step 2, synthesis of compound 25B

Compound 25A (200 mg), 3-hydroxytetrahydrofuran (51 mg), and cyanomethylene tri-n-butylphosphorane (278 mg) were dissolved in toluene (5 mL). Under a nitrogen atmosphere, the reaction mixture was refluxed and stirred at 120°C for 6 hours. After the reaction was completed, the reaction solution was cooled to room temperature and concentrated under reduced pressure to yield a crude product. The crude product was purified by silica gel column chromatography (dichloromethane/methanol = 24/1), and compound 25B (100 mg) was yielded. MS m/z (ESI): 591.3[M+1]⁺.

### Step 3, synthesis of compound 25C

Compound 25B (100 mg) was added to a hydrochloric acid/dioxane solution (4 M, 2 mL). The reaction mixture was stirred at 25°C for 4 hours. After the reaction was completed, the mixture was concentrated under reduced pressure, and compound 25C (90 mg) was yielded. MS m/z (ESI): 491.2[M+1]⁺.

### Step 4, synthesis of compound 25

Compound 25C (90 mg), phenyl carbamate (38 mg), and triethylamine (93 mg) were added to dichloromethane (2 mL). The reaction mixture was stirred at 25°C for 4 hours. After the reaction was completed, the mixture was concentrated under reduced pressure. The resulting crude product was purified by Flash reversed-phase chromatography, and compound 25 (20 mg) was yielded. MS m/z (ESI): 534.3[M+1]⁺.

¹H NMR (400 MHz, DMSO-d₆) δ 6.94 (s, 1H), 6.85 (s, 2H), 6.68 (s, 1H), 6.10 (t, J = 5.5 Hz, 1H), 5.45 (s, 2H), 5.32 (s, 1H), 5.06 (s, 1H), 4.18 (t, J = 6.6 Hz, 2H), 3.94-3.89 (m, 2H), 3.88-3.70 (m, 4H), 3.62 (s, 3H), 3.35 (d, J = 6.3 Hz, 2H), 2.90 (t, J = 5.6 Hz, 2H), 2.28-2.18 (m, 4H), 1.97 (s, 6H), 1.93 (s, 1H).

### Example 24: synthesis of compound 26

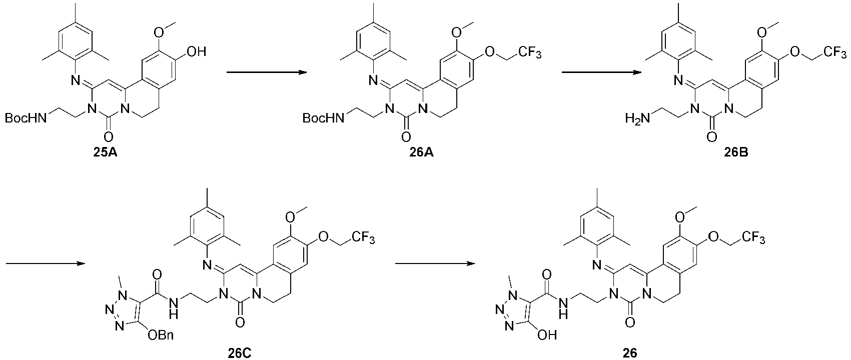

### Step 1, synthesis of compound 26A

Compound 25A (800 mg), and 2,2,2-trifluoroethyl trifluoromethanesulfonate (537 mg) were dissolved in acetonitrile (10 mL), and added with cesium carbonate (601 mg). Under a nitrogen atmosphere, the reaction mixture was stirred at 80°C for 10 hours. After the reaction was completed, the reaction mixture was cooled to room temperature, diluted with water (50 mL), and extracted with ethyl acetate (50 mL × 3). The organic phases were combined, washed with saturated saline and concentrated under reduced pressure to yield a crude product. The crude product was purified by silica gel column chromatography (petroleum ether/ethyl acetate = 1/1), and compound 26A (180 mg) was yielded. MS m/z(ESI): 603.3[M+1]⁺.

### Step 2, synthesis of compound 26B

Compound 26A (180 mg) was added to a hydrochloric acid/dioxane solution (4 M, 4 mL). The reaction mixture was stirred at 25°C for 1 hour. After the reaction was completed, the mixture was concentrated under reduced pressure, and compound 26B (150 mg) was yielded. MS m/z (ESI): 503.2[M+1]⁺.

### Step 3, synthesis of compound 26C

Compound 26B (100 mg) was dissolved in dichloromethane (2 mL), and added with triethylamine (88 mg) and compound 23H (44 mg). The reaction mixture was stirred at room temperature for 4 hours. After the reaction was completed, the mixture was concentrated under reduced pressure to yield a crude product. The crude product was purified by silica gel column chromatography (dichloromethane/methanol = 20/1), and compound 26C (70 mg) was yielded. MS m/z (ESI): 718.3[M+1]⁺.

### Step 4, synthesis of compound 26

Compound 26C (67 mg) was dissolved in methanol (1 mL), and added with palladium on carbon (30 mg) and palladium hydroxide on carbon (30 mg). Under a hydrogen atmosphere, the reaction mixture was heated to 30°C and stirred for 4 hours. After the reaction was completed, the reaction mixture was filtered, and the filtrate was concentrated under reduced pressure to yield a crude product. The crude product was purified by Flash reversed-phase chromatography, and compound 26 (27.5 mg) was yielded. MS m/z (ESI): 628.3[M+1]⁺.

¹H NMR (400 MHz, DMSO-d₆) δ 14.66 (s, 1H), 10.66 (s, 1H), 8.31 (s, 1H), 7.23 (s, 1H), 7.10 (s, 2H), 6.87 (s, 1H), 5.63 (s, 1H), 4.86 (q, J = 8.5 Hz, 2H), 4.42 (s, 2H), 4.02 (s, 2H), 3.90 (s, 3H), 3.69 (s, 5H), 2.95 (s, 2H), 2.31 (s, 3H), 2.14 (s, 6H).

### Example 25: synthesis of compound 27

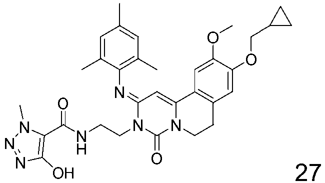

Compound 27 (6.5 mg) was yielded referring to the synthetic method described in Example 24. MS m/z(ESI): 600.3[M+1]⁺.

¹H NMR (400 MHz, DMSO-d₆) δ 14.67 (s, 1H), 10.58 (s, 1H), 8.31 (s, 1H), 7.12 (s, 2H), 7.05 (s, 1H), 6.77 (s, 1H), 5.56 (s, 1H), 4.42 (s, 2H), 4.01 (s, 2H), 3.91 (d, J = 6.8 Hz, 5H), 3.70 (d, J = 5.8 Hz, 2H), 3.66 (s, 3H), 2.94 (t, J = 6.1 Hz, 2H), 2.32 (s, 3H), 2.15 (s, 6H), 1.29-1.22 (m, 1H), 0.62-0.53 (m, 2H), 0.35-0.28 (m, 2H).

### Example 26: synthesis of compound 28

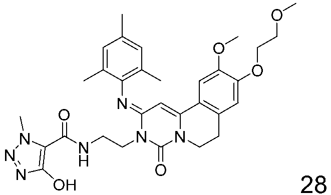

Compound 28 (12 mg) was yielded referring to the synthetic method described in Example 24. MS m/z (ESI): 604.3[M+1]⁺.

¹H NMR (400 MHz, DMSO-d₆) δ 14.67 (s, 1H), 10.58 (s, 1H), 8.31 (s, 1H), 7.11 (d, J = 6.6 Hz, 3H), 6.79 (s, 1H), 5.58 (s, 1H), 4.42 (s, 2H), 4.24-4.16 (m, 2H), 4.02 (s, 2H), 3.91 (s, 3H), 3.74-3.63 (m, 7H), 3.30 (s, 3H), 2.95 (s, 2H), 2.32 (s, 3H), 2.15 (s, 6H).

### Example 27: synthesis of compound 29

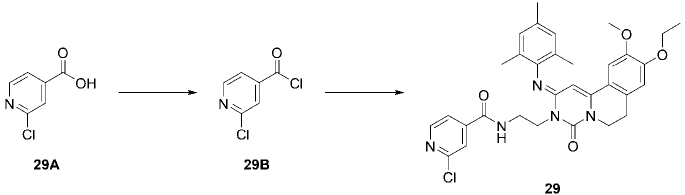

### Step 1, synthesis of compound 29B

Compound 29A (200 mg) was dissolved in thionyl chloride (5 mL). Under a nitrogen atmosphere, the mixture was heated to 80°C and reacted for 2 hours. After the reaction was completed, the resulting mixture was evaporated to dryness, and compound 29B (215 mg) was yielded.

### Step 2, synthesis of compound 29

Compound 29B (215 mg) was dissolved in dichloromethane (10 mL), and added with compound 14B (548 mg) and triethylamine (370 mg). After nitrogen displacement, the mixture was reacted at 25°C for 18 hours. After the reaction was completed, the reaction solution was concentrated to yield a crude product. The crude product was purified by silica gel column chromatography (petroleum ether/ethyl acetate = 1/4), and compound 29 (476 mg) was yielded. MS m/z (ESI): 588.2 [M+1]⁺.

¹H NMR (400 MHz, DMSO-d₆) δ 9.01 (t, J = 5.8 Hz, 1H), 8.54 (d, J = 5.1 Hz, 1H), 7.81 (s, 1H), 7.72 (d, J = 5.1 Hz, 1H), 6.94 (s, 1H), 6.84 (s, 2H), 6.67 (s, 1H), 5.33 (s, 1H), 4.36 (t, J = 5.8 Hz, 2H), 4.05 (p, J = 6.3 Hz, 2H), 3.89 (t, J = 5.9 Hz, 2H), 3.78-3.67 (m, 2H), 3.62 (s, 3H), 2.86 (d, J = 6.0 Hz, 2H), 2.21 (s, 3H), 1.95 (s, 6H), 1.33 (t, J = 6.9 Hz, 3H).

### Example 28: synthesis of compound 30

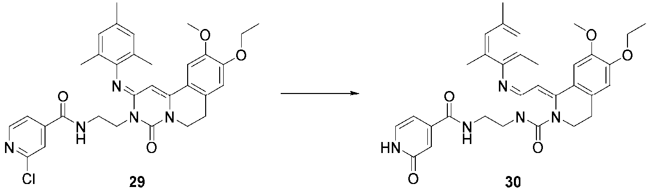

Compound 29 (200 mg), (E)-benzaldehyde oxime (54 mg), RockPhos-Pd-G3 (86 mg) and cesium carbonate (244 mg) were dissolved in toluene (5 mL). Under a nitrogen atmosphere, the mixture was heated to 80°C and reacted for 18 hours. After the reaction was completed, the reaction mixture was cooled to room temperature, and filtered, and the filtrate was concentrated to yield a crude product. The crude product was purified by Flash reversed-phase chromatography, and compound 30 (14 mg) was yielded. MS m/z(ESI): 570.0[M+1]⁺.

¹H NMR (400 MHz, DMSO-d₆) δ 10.61 (s, 1H), 8.81 (s, 1H), 7.45 (d, J = 6.7 Hz, 1H), 7.12 (s, 2H), 7.07 (s, 1H), 6.78 (s, 1H), 6.63 (s, 1H), 6.38 (d, J = 6.6 Hz, 1H), 5.59 (s, 1H), 4.47 (s, 2H), 4.15-4.09 (m, 2H), 4.02 (s, 2H), 3.69 (d, J = 5.1 Hz, 2H), 3.65 (s, 3H), 2.95 (s, 2H), 2.32 (s, 3H), 2.19 (s, 6H), 1.35 (t, J = 6.9 Hz, 3H).

### Example 29: synthesis of compound 31

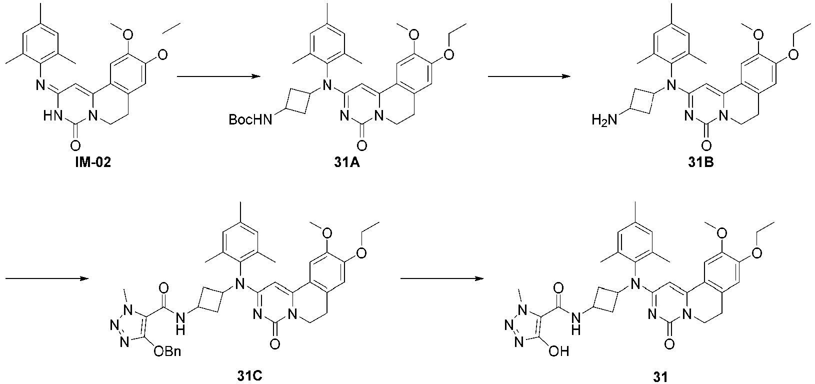

### Step 1, synthesis of compound 31A

IM-02 (500 mg) was added to toluene (5 mL), and added with tert-butyl (3-hydroxycyclobutyl)carbamate (346 mg) and cyanomethylene tri-n-butylphosphorane (890 mg). Under a nitrogen atmosphere, the reaction mixture was stirred at 120°C for 16 hours. After the reaction was completed, the mixture was concentrated. The resulting crude product was purified by silica gel column chromatography (dichloromethane/methanol = 24/1), and compound 31A (200 mg) was yielded. MS m/z (ESI): 575.4[M+1]⁺.

### Step 2, synthesis of compound 31B

Compound 31A (200 mg) was added to a hydrochloric acid/dioxane solution (4 M, 4 mL). The mixture was stirred at 25°C for 2 hours. After the reaction was completed, the reaction mixture was concentrated under reduced pressure to yield a crude product, which was purified by Flash reversed-phase chromatography, and compound 31B (120 mg) was yielded. MS m/z (ESI): 475.3[M+1]⁺.

### Step 3, synthesis of compound 31C

Compound 31B (120 mg) was dissolved in dichloromethane (2 mL), and added with triethylamine (128 mg) and compound 23H (64 mg). The mixture was stirred at room temperature for 4 hours. After the reaction was completed, the mixture was concentrated under reduced pressure to yield a crude product, which was purified by silica gel column chromatography (dichloromethane/methanol = 20/1), and compound 31C (120 mg) was yielded. MS m/z (ESI): 690.3[M+1]⁺.

### Step 4, synthesis of compound 31

Compound 31C (90 mg) was dissolved in methanol (2 mL), and added with palladium on carbon (18 mg) and palladium hydroxide on carbon (18 mg). Under a hydrogen atmosphere, the mixture was heated to 30°C and stirred for 14 hours. After the reaction was completed, the mixture was filtered, and the filtrate was concentrated under reduced pressure. The crude product was purified by Flash reversed-phase chromatography, and compound 31 (7 mg) was yielded. MS m/z (ESI): 600.3[M+1]⁺.

¹H NMR (400 MHz, DMSO-d₆) δ 7.80 (d, J = 8.3 Hz, 1H), 6.94 (s, 1H), 6.86 (s, 2H), 6.64 (s, 1H), 5.32 (s, 1H), 5.25-5.15 (m, 1H), 4.25 (dd, J = 15.9, 8.0 Hz, 1H), 4.09-4.03 (m, 2H), 3.95-3.88 (m, 3H), 3.83 (s, 2H), 3.61 (s, 3H), 3.01-2.94 (m, 2H), 2.89 (t, J = 6.0 Hz, 2H), 2.82-2.75 (m, 2H), 2.22 (s, 3H), 1.98 (d, J = 3.9 Hz, 6H), 1.33 (t, J = 7.0 Hz, 3H).

### Example 30: synthesis of compound 32

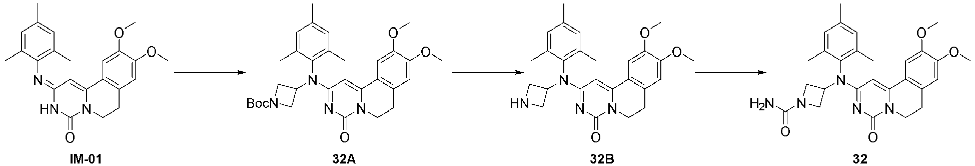

### Step 1, synthesis of compound 32A

IM-01 (200 mg) was added to toluene (5 mL), and added with tert-butyl 3-hydroxyazetidine-1-carboxylate (133 mg) and cyanomethylene tri-n-butylphosphorane (370 mg). Under a nitrogen atmosphere, the mixture was stirred at 120°C for 4 hours. After the reaction was completed, the mixture was concentrated under reduced pressure to yield a crude product, which was purified by silica gel column chromatography (dichloromethane/methanol = 10/1), and compound 32A (100 mg) was yielded. MS m/z (ESI): 547.2[M+1]⁺.

### Step 2, synthesis of compound 32B

Compound 32A (100 mg) was added to dichloromethane (2 mL), and added with trifluoroacetic acid (1 mL). The mixture was stirred at 25°C for 1 hour. After the reaction was completed, the reaction mixture was concentrated under reduced pressure. The residue was dried under vacuum, and compound 32B (70 mg) was yielded. MS m/z (ESI): 447.0[M+1]⁺.

### Step 3, synthesis of compound 32

Compound 32B (70 mg) was added to dichloromethane (2 mL), and added with phenyl carbamate (32 mg) and triethylamine (79 mg) in sequence. The mixture was stirred at 25°C for 16 hours. After the reaction was completed, the mixture was concentrated under reduced pressure. The crude product was purified by Flash reversed-phase chromatography, and compound 32 (30 mg) was yielded. MS m/z (ESI): 490.2[M+1]⁺.

¹H NMR (400 MHz, DMSO-d₆) δ 7.45-6.22 (m, 4H), 5.98-5.81 (m, 2H), 5.24 (s, 1H), 4.99-4.80 (m, 1H), 4.27-3.70 (m, 8H), 3.59 (s, 2H), 3.57-3.45 (m, 2H), 2.88 (t, J = 6.2 Hz, 2H), 2.34-2.26 (m, 3H), 2.13-1.99 (m, 6H).

### Example 31: synthesis of compound 33

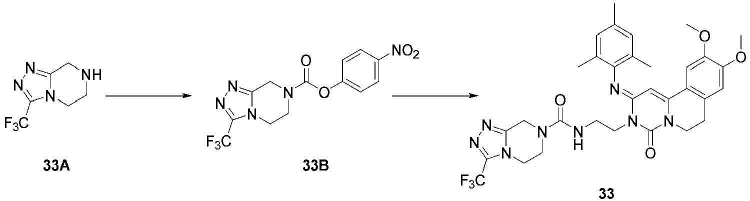

### Step 1, synthesis of compound 33A

p-Nitrophenyl chloroformate (1.26 g) and N,N-diisopropylethylamine (3.36 g) were dissolved in anhydrous dichloromethane (20 mL), and added with compound 33A (1.0 g). Under a nitrogen atmosphere, the mixture was stirred at room temperature for 14 hours. After the reaction was completed, the mixture was concentrated under reduced pressure to yield a crude product. The crude product was purified by silica gel column chromatography (dichloromethane/methanol = 24/1), and compound 33B (0.62 g) was yielded. MS m/z(ESI): 358.0[M+1]⁺.

### Step 2, synthesis of compound 33

Compound 2 (100 mg) and compound 33B (90 mg) were dissolved in acetonitrile (2 mL), and added with triethylamine (116 mg). Under a nitrogen atmosphere, the mixture was stirred at 80°C for 12 hours. After the reaction was completed, the mixture was concentrated under reduced pressure to yield a crude product. The crude product was purified by Flash reversed-phase chromatography, and compound 33 (25 mg) was yielded. MS m/z(ESI): 653.0[M+1]⁺.

¹H NMR (400 MHz, DMSO-d₆) δ 10.69 (s, 1H), 7.32 (s, 1H), 7.11 (d, J = 7.1 Hz, 3H), 6.79 (s, 1H), 5.60 (s, 1H), 4.78 (s, 2H), 4.36 (s, 2H), 4.17 (t, J = 5.0 Hz, 2H), 3.88 - 3.79 (m, 7H), 3.65 (s, 3H), 3.50 (d, J = 5.2 Hz, 2H), 2.93 (t, J = 6.2 Hz, 2H), 2.33 (s, 3H), 2.20 (s, 6H).

### Example 32: synthesis of compound 34

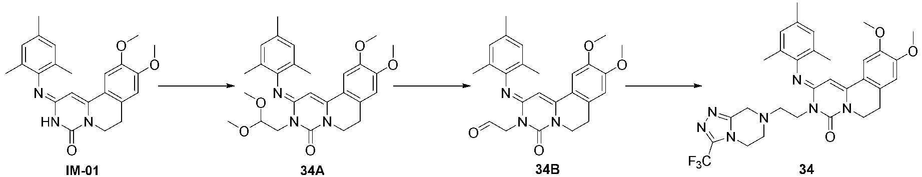

### Step 1, synthesis of compound 34A

IM-01 (2.0 g) was dissolved in N,N-dimethylformamide (20 mL), and added with 2-bromo-1,1-dimethoxyethane (6.0 g), potassium carbonate (6.3 g) and sodium iodide (4.59 g) in sequence. Under a nitrogen atmosphere, the mixture was stirred at 83°C for 72 hours. After the reaction was completed, the mixture was concentrated under reduced pressure. The resulting residue was diluted with water (70 mL) and extracted with ethyl acetate (70 mL × 3). The organic phases were combined, dried over anhydrous sodium sulfate, filtered to remove the drying agent, and concentrated under reduced pressure. The resulting residue was purified by silica gel column chromatography (petroleum ether/ethyl acetate = 4/1 to 3/2), and compound 34A (150 mg) was yielded. MS m/z(ESI): 480.2[M+1]⁺.

### Step 2, synthesis of compound 34B

Compound 34A (130 mg) and concentrated hydrochloric acid (0.3 mL, 12 mol/L) were dissolved in 1,4-dioxane (1 mL). Under a nitrogen atmosphere, the reaction mixture was stirred at 25°C for 10 hours. After the reaction was completed, saturated aqueous sodium carbonate solution was added to adjust the pH to 7-8. The reaction solution was diluted with water (3 mL), and extracted with ethyl acetate (5 mL × 3). The organic phases were combined, dried over anhydrous sodium sulfate, filtered, and concentrated under reduced pressure, and compound 34B (100 mg) was yielded. MS m/z(ESI): 434.1[M+1]⁺.

### Step 3, synthesis of compound 34

Compound 34B (100 mg) and 3-(trifluoromethyl)-5,6,7,8-tetrahydro-[1,2,4]triazolo[4,3-a]pyrazine (89 mg) were dissolved in ethanol (2 mL). Under a nitrogen atmosphere, the reaction mixture was stirred at 78°C for 12 hours. After the reaction was completed, the mixture was cooled to room temperature, added with sodium borohydride (44 mg, 1.15 mmol) and methanol (0.5 mL), heated to 78°C, and stirred for 12 hours. The reaction mixture was quenched with water and concentrated under reduced pressure. The residue was dissolved in ethanol (1 mL), and added with palladium on carbon (10 mg). Under a hydrogen atmosphere, the mixture was stirred at room temperature for 10 hours. After the reaction was completed, the mixture was filtered and was concentrated under reduced pressure to yield a crude product. The crude product was purified by Flash reversed-phase chromatography, and compound 34 (15 mg) was yielded. MS m/z(ESI): 610.2[M+1]⁺.

¹H NMR (400 MHz, DMSO-d₆) δ 6.96 (s, 1H), 6.85 (s, 2H), 6.66 (s, 1H), 5.32 (s, 1H), 4.35 (t, J = 6.5 Hz, 2H), 4.15 (t, J = 5.3 Hz, 2H), 4.00 (s, 2H), 3.91 (t, J = 6.0 Hz, 2H), 3.80 (s, 3H), 3.61 (s, 3H), 3.08 (t, J = 5.4 Hz, 2H), 2.97 (t, J = 6.6 Hz, 2H), 2.89 (t, J = 5.9 Hz, 2H), 2.22 (s, 3H), 1.94 (s, 6H).

### Example 33: synthesis of compound 35

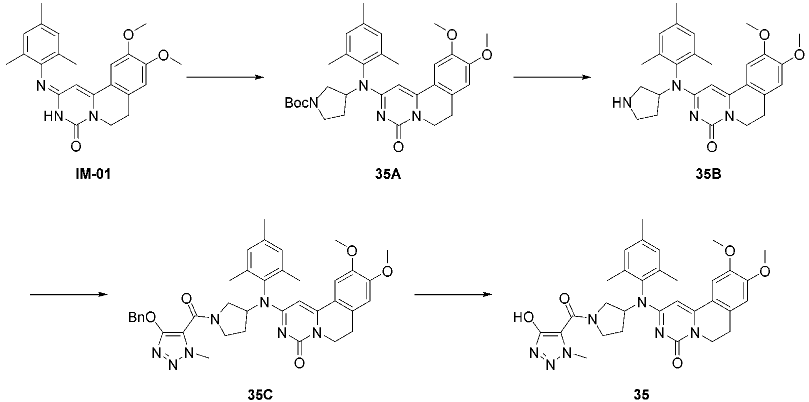

### Step 1, synthesis of compound 35A

IM-01 (1.0 g) and tert-butyl 3-hydroxypyrrolidin-1-carboxylate (590 mg) were dissolved in toluene (10 mL), and added with cyanomethylene tri-n-butylphosphorane (1.9 g). The reaction mixture was heated to 120°C and reacted for 10 hours under a nitrogen atmosphere. After the reaction was completed, the mixture was concentrated under reduced pressure. The crude product was purified by silica gel chromatography (petroleum ether/ethyl acetate = 3/2), and compound 35A (0.5 g) was yielded. MS m/z (ESI): 561.3[M+1]⁺.

### Step 2, synthesis of compound 35B

Compound 35A (500 mg) was added to a solution of hydrochloric acid in 1,4-dioxane (4 M, 5 mL). The reaction mixture was stirred at 25°C for 1 hour. After the reaction was completed, the mixture was dried by rotary evaporation, and compound 35B (0.42 g) was yielded. MS m/z (ESI): 461.3[M+1]⁺.

### Step 3, synthesis of compound 35C

Compound 35B (110 mg) and compound 23H (50 mg) were dissolved in dichloromethane (3 mL), and added with N,N-diisopropylethylamine (51 mg). The reaction mixture was reacted at room temperature for 3 hours. After the reaction was completed, the mixture was concentrated under reduced pressure. The crude residue was purified by preparative thin-layer chromatography (dichloromethane/methanol = 60/1), and compound 35C (80 mg) was yielded. MS m/z (ESI): 676.2[M+1]⁺.

### Step 4, synthesis of compound 35

Compound 35C (80 mg) was dissolved in methanol (20 mL), and added with palladium hydroxide on carbon (8 mg) and palladium on carbon (8 mg). The reaction mixture was reacted at 30°C for 72 hours under a hydrogen atmosphere. After the reaction was completed, the mixture was filtered and concentrated under reduced pressure. The crude product was purified by preparative thin-layer chromatography (dichloromethane/methanol = 30/1), and compound 35 (17 mg) was yielded. MS m/z (ESI): 586.3[M+1]⁺.

¹H NMR (400 MHz, DMSO-d₆) δ 6.96 (d, J = 3.5 Hz, 1H), 6.85 (s, 2H), 6.64 (d, J = 7.9 Hz, 1H), 6.13-6.02 (m, 1H), 5.33 (d, J = 10.3 Hz, 1H), 4.38-4.04 (m, 1H), 4.01-3.52 (m, 15H), 2.90 (q, J = 5.9 Hz, 2H), 2.81-2.72 (m, 1H), 2.21 (s, 4H), 2.03-1.85 (m, 6H).

### Example 34: synthesis of compound 36

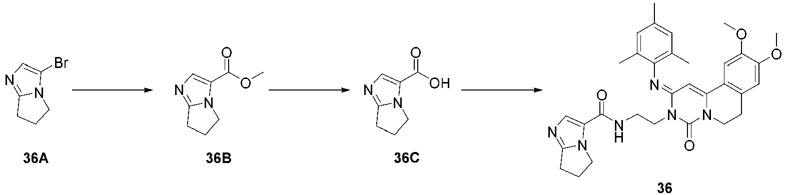

### Step 1, synthesis of compound 36B

Compound 36A (100 mg), 1,1'-binaphthyl-2,2'-bis(diphenylphosphine) (73 mg), bis(acetonitrile)dichloropalladium (23 mg) and N,N-diisopropylethylamine (104 mg) were added to a methanol/acetonitrile mixed solvent (1/1, 5 mL). The reaction mixture was stirred at 100°C for 16 hours under a carbon monoxide atmosphere (1 MPa). After the reaction was completed, the reaction mixture was cooled to room temperature and concentrated under reduced pressure. The resulting crude product was purified by silica gel column chromatography (dichloromethane/methanol = 10/1), and compound 36B (100 mg) was yielded. MS m/z(ESI): 167.1[M+1]⁺.

### Step 2, synthesis of compound 36C

Compound 36B (100 mg) and lithium hydroxide (75 mg) were dissolved in tetrahydrofuran (2 mL) and water (2 mL). The reaction mixture was stirred at 25°C for 1 hour. After the reaction was completed, the pH of the mixture was adjusted to 2 by 1 M dilute hydrochloric acid. The resulting solution was purified by Flash reversed-phase chromatography, and compound 36C (40 mg) was yielded. MS m/z(ESI): 153.2[M+1]⁺.

### Step 3, synthesis of compound 36

Compound 36C (10 mg) was dissolved in N,N-dimethylformamide (2 mL), and added with HATU (26 mg) and diisopropylethylamine (22 mg). The mixture was stirred at room temperature for 0.5 hours, and added with compound 2 (25 mg). The reaction mixture was stirred for 3 hours at room temperature. After the reaction was completed, the reaction mixture was diluted with ethyl acetate to 100 mL and was washed with water (30 mL × 3). The organic phase was dried by anhydrous sodium sulfate, filtered, and concentrated under reduced pressure. The crude residue was purified by preparative thin-layer chromatography (dichloromethane/methanol = 25/1), and compound 36 (15 mg) was yielded. MS m/z(ESI): 569.2[M+1]⁺.

¹H NMR (400 MHz, DMSO-d₆) δ 8.38 (s, 1H), 7.54 (s, 1H), 6.97 (s, 1H), 6.87 (s, 2H), 6.68 (s, 1H), 5.35 (s, 1H), 4.32 (t, J = 6.7 Hz, 2H), 4.08 (t, J = 7.1 Hz, 2H), 3.92 (t, J = 6.2 Hz, 2H), 3.81 (s, 3H), 3.68-3.54 (m, 5H), 2.93-2.87 (m, 2H), 2.75 (t, J = 7.6 Hz, 2H), 2.49-2.41 (m, 2H), 2.22 (s, 3H), 1.97 (s, 6H).

### Example 35: synthesis of compound 37

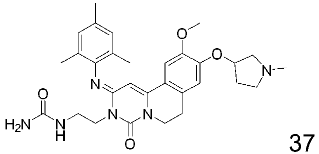

Compound 37 (20 mg) was yielded referring to the synthetic method described in Example 23. MS m/z(ESI): 547.3[M+1]⁺.

¹H NMR (400 MHz, DMSO-d₆) δ 6.86 (d, J = 5.1 Hz, 3H), 6.66 (s, 1H), 6.10 (t, J = 5.7 Hz, 1H), 5.44 (s, 2H), 5.31 (s, 1H), 4.88 (s, 1H), 4.18 (t, J = 6.6 Hz, 2H), 3.91 (d, J = 6.1 Hz, 2H), 3.61 (s, 3H), 3.39-3.33 (m, 2H), 2.89 (t, J = 5.9 Hz, 2H), 2.77 (m, 1H), 2.68-2.54 (m, 2H), 2.37-2.27 (m, 2H), 2.23 (d, J = 9.9 Hz, 6H), 1.97 (s, 6H), 1.79-1.69 (m, 1H).

### Example 36: synthesis of compound 38

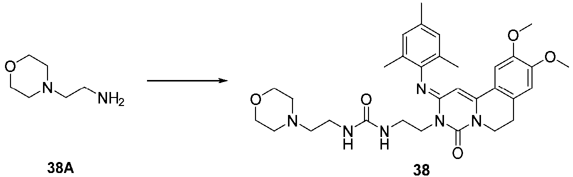

Compound 38A (15 mg), N,N'-carbonyldiimidazole (22 mg), and triethylamine (46 mg) were dissolved in dichloromethane (5 mL). The reaction mixture was stirred at 25°C for 2 hours, and added with compound 2 (50 mg). The mixture was stirred for 16 hours at 25°C. After the reaction was completed, the mixture was concentrated under reduced pressure. The resulting crude product was purified by Flash reversed-phase chromatography, and compound 38 (5 mg) was yielded. MS m/z(ESI): 591.2[M+1]⁺.

¹H NMR (400 MHz, DMSO-d₆) δ 10.92 (s, 1H), 9.74 (s, 1H), 7.08 (s, 3H), 6.78 (s, 3H), 5.58 (s, 1H), 4.31 (s, 2H), 4.06 (s, 2H), 3.93-3.84 (m, 5H), 3.65 (s, 3H), 3.57 (s, 2H), 3.45 (d, J = 6.3 Hz, 4H), 3.37-3.29 (m, 2H), 3.11 (s, 2H), 3.09-2.96 (m, 4H).

### Example 37: synthesis of compound 39

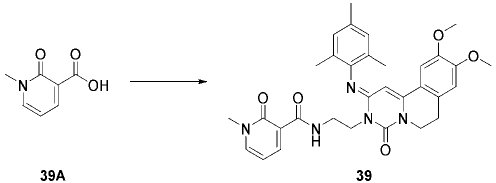

Compound 39A (79 mg), compound 2 (150 mg), N,N,N',N'-tetramethylchloroformamidinium hexafluorophosphate (145 mg) and N-methylimidazole (85 mg) were dissolved in N,N-dimethylformamide (2 mL). The reaction mixture was stirred at 25°C for 16 hours. After the reaction was completed, the reaction mixture was purified by Flash reversed-phase chromatography, and compound 39 (30 mg) was yielded. MS m/z(ESI): 570.7[M+1]⁺.

¹H NMR (400 MHz, DMSO-d₆) δ 8.28 (dd, J = 7.3, 2.1 Hz, 1H), 8.04 (dd, J = 6.5, 2.1 Hz, 1H), 7.08 (s, 2H), 7.04 (s, 1H), 6.76 (s, 1H), 6.50 (t, J = 6.9 Hz, 1H), 5.53 (s, 1H), 4.40 (t, J = 6.2 Hz, 2H), 4.02 (s, 2H), 3.85 (s, 3H), 3.79 (t, J = 6.3 Hz, 2H), 3.64 (s, 3H), 3.56 (s, 3H), 2.97 (s, 2H), 2.30 (s, 3H), 2.13 (s, 6H).

### Example 38: synthesis of compound 40

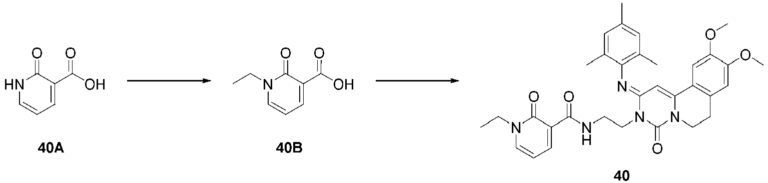

### Step 1, synthesis of compound 40B

Potassium hydroxide (1.6 g) was dissolved in water (3 mL) to prepare a solution. Compound 40A (2.0 g) was dissolved in methanol (20 mL), and added with the prepared aqueous potassium hydroxide solution. After the reaction mixture was stirred for 10 minutes, iodoethane (2.47 g) was added dropwise. Under a sealing condition, the reaction mixture was heated to70 °C with continuous stirring for 20 hours. After the reaction was completed, the mixture was concentrated under reduced pressure, diluted with water, adjusted to pH 2-3 with 1 M diluted hydrochloric acid, and extracted with ethyl acetate. The organic phases were combined, dried over anhydrous sodium sulfate, filtered, and concentrated under reduced pressure. The crude product was purified by silica gel column chromatography (dichloromethane/methanol = 10/1) to obtain compound 40B (0.35 g) in 12% yield. MS m/z(ESI): 168.0[M+1]⁺.

### Step 2, synthesis of compound 40

Compound 40B (46 mg), compound 2 (80 mg), N,N,N',N'-tetramethylchloroformamidinium hexafluorophosphate (78 mg), and N-methylimidazole (45 mg) were dissolved in N,N-dimethylformamide (1.5 mL). The reaction mixture was stirred at 25 °C for 12 hours. After the reaction was completed, the reaction mixture was purified by Flash reversed-phase column chromatography, and compound 40 (15 mg) was yielded. MS m/z(ESI): 584.3[M+1]⁺.

¹H NMR (400 MHz, DMSO-d₆) δ 9.91 (t, J = 5.6 Hz, 1H), 8.29 (d, J = 7.2 Hz, 1H), 8.05 (d, J = 6.5 Hz, 1H), 6.96 (s, 1H), 6.84 (s, 2H), 6.67 (s, 1H), 6.51 (t, J = 6.8 Hz, 1H), 5.32 (s, 1H), 4.34 (t, J = 6.1 Hz, 2H), 4.02 (q, J = 6.9 Hz, 2H), 3.89 (t, J = 5.5 Hz, 2H), 3.80 (s, 3H), 3.76-3.68 (m, 2H), 3.62 (s, 3H), 2.89 (t, J = 5.3 Hz, 2H), 2.21 (s, 3H), 1.93 (s, 6H), 1.24 (t, J = 7.0 Hz, 3H).

### Example 39: synthesis of compound 41

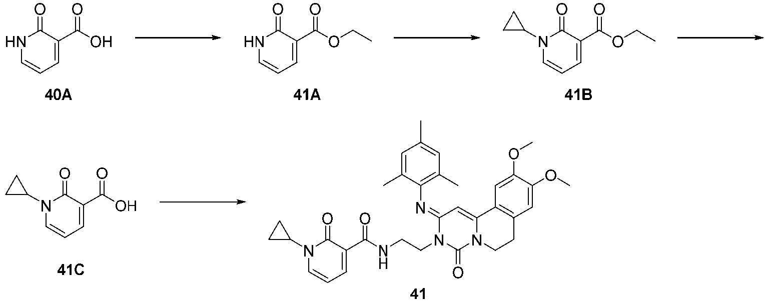

### Step 1, synthesis of compound 41A

Compound 40A (1.4 g) was dissolved in anhydrous ethanol (50 mL), and added with thionyl chloride (1 mL). The reaction mixture was reacted at 90 °C for 1 hour. After the reaction was completed, the mixture was concentrated under reduced pressure. The resulting crude product was purified by Flash reversed-phase chromatography, and compound 41A (0.3 g) was yielded. MS m/z(ESI): 168.1[M+1]⁺.

### Step 2, synthesis of compound 41B

Compound 41A (300 mg) was dissolved in toluene (15 mL), and cyclopropylboronic acid (308 mg), copper acetate (326 mg), sodium bis(trimethylsilyl)amide (1645 mg), and pyridine (710 mg) were added. The reaction mixture was reacted at 100 °C for 48 hours under a nitrogen atmosphere. After the reaction was completed, the mixture was concentrated under reduced pressure. The residue was diluted with water (20 mL) and extracted with ethyl acetate (30 mL × 3). The organic phases were combined, washed with saturated saline, dried over anhydrous sodium sulfate, and filtered, and the filtrate was dried by rotary evaporation to yield compound 41B (0.3 g). MS m/z(ESI): 207.9[M+1]⁺.

### Step 3, synthesis of compound 41C

Compound 41B (300 mg) was dissolved in ethanol (10 mL), and added with water (2 mL) and sodium hydroxide (116 mg) in sequence. The reaction mixture was reacted at room temperature for 1 hour. After the reaction was completed, the mixture was diluted with water (10 mL) and extracted with methyl tert-butyl ether. The aqueous phase was adjusted to pH 2-3 with 1 M aqueous hydrochloric acid solution, and was extracted again with ethyl acetate (30 mL × 3). The organic phases were combined and concentrated, and the residue was purified by Flash reversed-phase chromatography and lyophilized, and compound 41C (30 mg) was yielded. MS m/z(ESI): 179.9[M+1]⁺.

### Step 4, synthesis of compound 41

Compound 41C (30 mg) was dissolved in N,N-dimethylformamide (2 mL), and added with N,N,N',N'-tetramethylchloroformamidinium hexafluorophosphate (70 mg), N-methylimidazole (41 mg), and compound 2 (109 mg) in sequence. The reaction mixture was stirred at 25 °C for 16 hours under a nitrogen atmosphere. After the reaction was completed, the mixture was purified by Flash reversed-phase chromatography and lyophilized, and compound 41 (9 mg) was yielded. MS m/z(ESI): 596.3[M+1]⁺.

¹H NMR (400 MHz, DMSO-d₆) δ 10.78 (s, 1H), 10.09 (s, 1H), 8.27 (dd, J = 7.2, 2.1 Hz, 1H), 7.95 (dd, J = 6.7, 2.0 Hz, 1H), 7.10 (d, J = 12.1 Hz, 3H), 6.78 (s, 1H), 6.44 (t, J = 7.0 Hz, 1H), 5.57 (s, 1H), 4.40 (t, J = 6.6 Hz, 2H), 4.06 (s, 2H), 3.85 (s, 3H), 3.78 (d, J = 6.4 Hz, 2H), 3.64 (s, 3H), 3.43 (td, J = 7.5, 3.7 Hz, 1H), 2.99 (t, J = 6.5 Hz, 2H), 2.32 (s, 3H), 2.17 (s, 6H), 1.05 (q, J = 7.3 Hz, 2H), 0.94 - 0.87 (m, 2H).

### Example 40: synthesis of compound 42

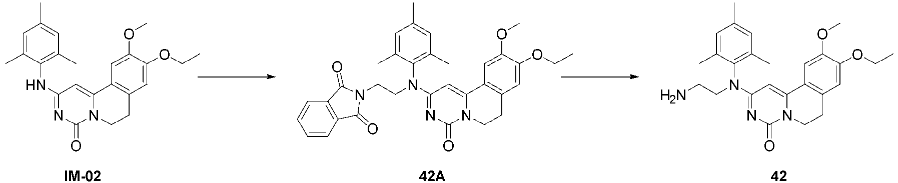

### Step 1, synthesis of compound 42A

IM-02 (1.0 g), 2-(2-bromoethyl)isoindoline-1,3-dione (3.5 g), potassium carbonate (3.1 g), and potassium iodide (2.5 g) were added to 2-butanone (20 mL). The mixture was stirred at 85 °C for 16 hours. After the reaction was completed, the mixture was concentrated under reduced pressure. The crude product was purified by silica gel column chromatography (dichloromethane/methanol = 20/1), and compound 42A (200 mg) was yielded. MS m/z(ESI): 579.3[M+1]⁺.

### Step 2, synthesis of compound 42

Compound 42A (120 mg) was dissolved in ethanol (10 mL), and hydrazine hydrate (31 mg) was added. The reaction mixture was stirred at 85 °C under a nitrogen atmosphere for 16 hours. After the reaction was completed, the mixture was filtered and concentrated under reduced pressure to yield a crude product. The crude product was purified by Flash reversed-phase chromatography, and compound 42 (90 mg) was yielded. MS m/z(ESI): 449.3[M+1]⁺.

¹H NMR (400 MHz, DMSO-d₆) δ 7.99 (s, 2H), 7.10 (s, 2H), 6.98 (s, 1H), 6.60 (s, 1H), 5.27 (s, 1H), 4.07 (d, J = 7.0 Hz, 2H), 3.98 - 3.90 (m, 4H), 3.59 (s, 3H), 3.10 (d, J = 6.6 Hz, 2H), 2.88 (t, J = 6.3 Hz, 2H), 2.31 (s, 3H), 2.11 (s, 6H), 1.33 (t, J = 6.9 Hz, 3H).

### Example 41: synthesis of compound 43

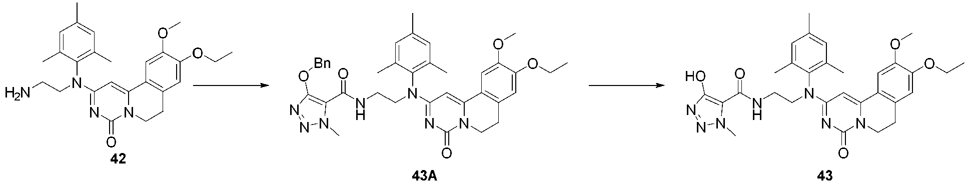

### Step 1, synthesis of compound 43A

Compound 42 (50 mg) and compound 23H (45 mg) were dissolved in dichloromethane (5 mL), and added with triethylamine (36 mg). The reaction mixture was stirred at 25 °C for 16 hours. After the reaction was completed, the mixture was concentrated under reduced pressure, and the resulting crude product was purified by Flash reversed-phase chromatography, and compound 43A (40 mg) was yielded. MS m/z (ESI): 664.3 [M+1]⁺.

### Step 2, synthesis of compound 43

Compound 43A (40 mg), palladium on carbon (40 mg), and palladium hydroxide on carbon (40 mg) were added to methanol (10 mL). Under a hydrogen atmosphere, the mixture was stirred at 30 °C for 2 hours. After the reaction was completed, the reaction mixture was filtered, and the filtrate was concentrated under reduced pressure. The crude product was purified by Flash reversed-phase chromatography, and compound 43 (15 mg) was yielded. MS m/z (ESI): 574.2 [M+1]⁺.

¹H NMR (400 MHz, CDCl₃) δ 6.99 (s, 2H), 6.70 (s, 1H), 6.65 (s, 1H), 5.42 (s, 1H), 4.23-4.20 (m, 2H), 4.14-4.12 (m, 2H), 4.10 (s, 3H), 4.09-4.06 (m, 2H), 3.74-3.72 (m, 5H), 2.92 (t, J = 6.3 Hz, 2H), 2.33 (s, 3H), 2.15 (s, 6H), 1.48 (t, J = 7.0 Hz, 3H).

### Example 42: synthesis of compound 44

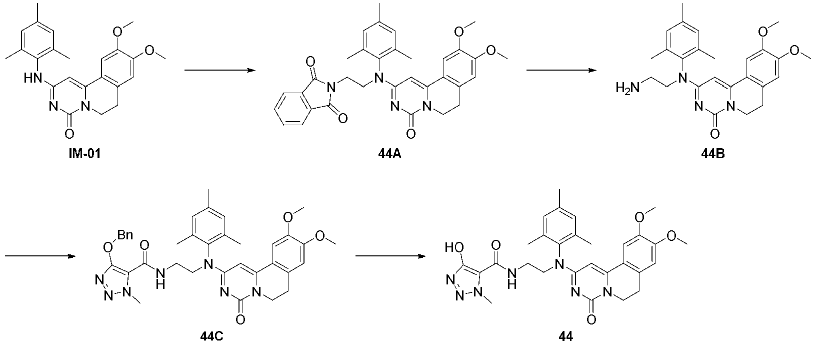

Compound 44 (10mg) was yielded by referring to the synthesis method described in Example 40 and Example 41. MS m/z(ESI): 560.2[M+1]⁺.

¹H NMR (400 MHz, CDCl₃) δ 7.00 (s, 2H), 6.72 (s, 1H), 6.63 (s, 1H), 5.41 (s, 1H), 4.20-4.24 (m, 2H), 4.11 (s, 3H), 4.10-4.07 (m, 2H), 3.92 (s, 3H), 3.75 (d, J = 4.8 Hz, 2H), 3.72 (s, 3H), 2.95 (s, 2H), 2.33 (s, 3H), 2.16 (s, 6H).

### Example 43: synthesis of compound 45

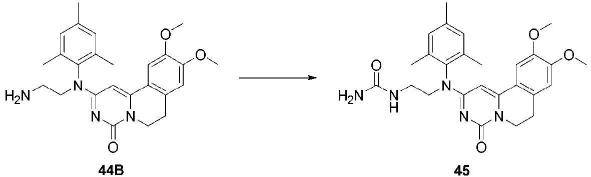

Compound 44B (50 mg) and phenyl carbamate (16 mg) were dissolved in dichloromethane (5 mL), and added with triethylamine (23 mg). The reaction mixture was stirred at 25 °C for 2 hours. After the reaction was completed, the mixture was concentrated. The resulting crude product was purified by Flash reversed-phase chromatography, and compound 45 (10 mg) was yielded. MS m/z(ESI): 478.2[M+1]⁺.

¹H NMR (400 MHz, CDCl₃) δ 7.01 (s, 2H), 6.72 (s, 1H), 6.62 (s, 1H), 5.36 (s, 1H), 4.22-4.13 (m, 2H), 3.92 (s, 5H), 3.72 (s, 3H), 3.52 (t, J = 6.6 Hz, 2H), 2.94 (t, J = 6.3 Hz, 2H), 2.34 (s, 3H), 2.15 (s, 6H).

### Example 44: synthesis of compound 46

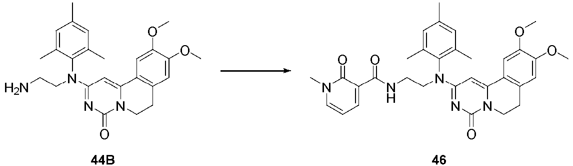

Compound 44B (50 mg), 1-methyl-2-oxo-1,2-dihydropyridine-3-carboxylic acid (26 mg), N,N,N',N'-tetramethylchloroformamidinium hexafluorophosphate (48 mg), and N-methylimidazole (28 mg) were added to N,N-dimethylformamide (2 mL). The reaction mixture was stirred at 25 °C for 16 hours. After the reaction was completed, the mixture was purified by Flash reversed-phase chromatography, and compound 46 (5 mg) was yielded. MS m/z(ESI): 570.2[M+1]⁺.

¹H NMR (400 MHz, CDCl₃) δ 9.91 (s, 1H), 8.43 (d, J = 5.4 Hz, 1H), 7.50 (d, J = 6.6 Hz, 1H), 6.96 (s, 2H), 6.69 (s, 1H), 6.64 (s, 1H), 6.34 (t, J = 6.9 Hz, 1H), 5.38 (s, 1H), 4.18 (t, J = 6.3 Hz, 2H), 4.12-4.06 (m, 2H), 3.90 (s, 3H), 3.80 (dd, J = 15.0, 5.9 Hz, 2H), 3.72 (s, 3H), 3.62 (s, 3H), 2.90 (t, J = 6.4 Hz, 2H), 2.31 (s, 3H), 2.18 (s, 6H).

### Example 45: synthesis of compound 47

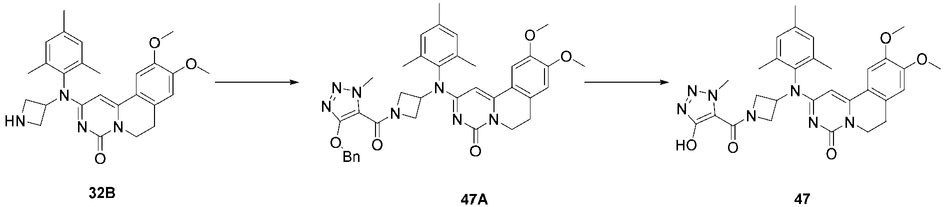

### Step 1, synthesis of compound 47A

4-Benzyloxy-1-methyl-1H-1,2,3-triazole-5-carboxylic acid (50 mg) was dissolved in N,N-dimethylformamide (4 mL), and added with HATU (170 mg) and diisopropylethylamine (93 mg). The mixture was stirred for 0.5 hour. Compound 32B (80 mg) was then added, and the reaction mixture was stirred for 3 hours. After the reaction was completed, the reaction mixture was diluted with ethyl acetate to 50 mL, washed with water (30 mL × 3), dried over anhydrous sodium sulfate and filtered, and the filtrate was concentrated to yield a crude product. The crude product was purified by preparative thin-layer chromatography, and compound 47A (100 mg) was yielded. MS m/z(ESI): 662.3[M+1]⁺.

### Step 2, synthesis of compound 47

Compound 47A (100 mg), palladium hydroxide on carbon (50 mg), and palladium on carbon (50 mg) were added to methanol (10 mL), and the mixture was stirred at room temperature for 1 hour under a hydrogen atmosphere. After the reaction was completed, the mixture was filtered, and the filtrate was concentrated. The crude residue was purified by Flash reversed-phase chromatography, and compound 47 (4.7 mg) was yielded. MS m/z(ESI): 572.2[M+1]⁺.

¹H NMR (400 MHz, DMSO-d₆) δ 7.57 (s, 1H), 7.26 (s, 1H), 7.10 (d, J = 5.1 Hz, 2H), 7.06 (s, 1H), 5.94 (s, 1H), 4.83 (d, J = 9.4 Hz, 1H), 4.57 (t, J = 11.0 Hz, 1H), 4.43 (dd, J = 11.8, 7.5 Hz, 1H), 4.11 (t, J = 6.6 Hz, 2H), 3.87 (d, J = 11.3 Hz, 6H), 3.75 (s, 3H), 3.59 (dd, J = 13.9, 7.3 Hz, 1H), 3.41 - 3.35 (m, 1H), 2.35-2.24 (m, 9H).

### Example 46: synthesis of compound 48

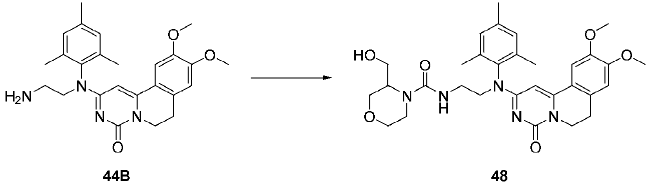

Morpholin-3-methanol (16 mg), N,N'-carbonyldiimidazole (19 mg), and triethylamine (58 mg) were dissolved in dichloromethane (5 mL), stirred at 25 °C for 2 hours, and added with compound 44B (50 mg). The reaction mixture was stirred at 25 °C for 16 hours. After the reaction was completed, the mixture was concentrated. The resulting crude product was purified by Flash reversed-phase chromatography, and compound 48 (5 mg) was yielded. MS m/z(ESI): 578.3[M+1]⁺.

¹H NMR (400 MHz, CDCl₃) δ 7.01 (s, 2H), 6.71 (s, 1H), 6.63 (s, 1H), 5.41 (s, 1H), 4.25-4.06 (m, 5H), 3.92 (s, 3H), 3.90 (s, 1H), 3.87 (d, J = 5.6 Hz, 2H), 3.75 (d, J = 6.1 Hz, 1H), 3.72 (s, 3H), 3.52-3.47 (m, 5H), 3.12 - 3.02 (m, 1H), 2.92 (t, J = 6.3 Hz, 2H), 2.34 (s, 3H), 2.14-2.17 (m, 6H).

### Example 47: synthesis of compound 49

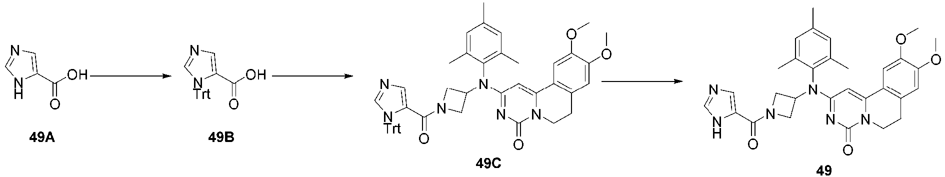

### Step 1, synthesis of compound 49B

Triphenylmethyl chloride (1368 mg) and 1H-imidazole-5-carboxylic acid 49A (500 mg) were dissolved in N,N-dimethylformamide (10 mL), and added with pyridine (0.5 mL). Under a nitrogen atmosphere, the mixture was heated and stirred at 50 °C for 6 hours. After the reaction was completed, water (30 mL) was added to the mixture. The resulting solid was collected by filtration and washed with ethyl acetate (10 mL × 3), and compound 49B (400 mg) was yielded. MS m/z(ESI): 709.2[2M+1]⁺.

### Step 2, synthesis of compound 49C

Compound 49B (90 mg) was dissolved in N,N-dimethylformamide (10 mL), and added with HATU (220 mg) and diisopropylethylamine (119 mg) in sequence. After the mixture was stirred for 15 minutes, compound 32B (100 mg) was added. The mixture was stirred for 3 hours. After the reaction was completed, the reaction mixture was diluted with water (20 mL) and extracted with ethyl acetate (15 mL × 3). The organic phases were combined, dried over anhydrous sodium sulfate, filtered and concentrated under reduced pressure. The crude product was purified by silica gel column chromatography (dichloromethane/methanol = 100/1 to 100/3), and compound 49C (115 mg) was yielded. MS m/z(ESI): 783.4[M+1]⁺.

### Step 3, synthesis of compound 49

Compound 49C (100 mg) was dissolved in a solution of trifluoroacetic acid in dichloromethane (5%, 10 mL). The mixture was stirred at room temperature for 1 hour. After the reaction was completed, the reaction mixture was concentrated, and the crude product was purified by thin-layer chromatography and dried, and compound 49 (18 mg) was yielded. MS m/z(ESI): 541.0[M+1]⁺.

¹H NMR (400 MHz, DMSO-d₆) δ 8.57 (s, 1H), 8.41-8.02 (m, 1H), 7.74 (t, J = 6.2 Hz, 1H), 7.30 (s, 1H), 7.10 (s, 3H), 6.08 (s, 1H), 4.83-4.73 (m, 1H), 4.57 (t, J = 11.0 Hz, 1H), 4.46-4.37 (m, 1H), 4.18-4.04 (m, 2H), 3.87 (s, 3H), 3.76 (s, 3H), 3.70-3.59 (m, 1H), 3.40-3.28 (m, 1H), 3.01 (t, J = 6.6 Hz, 2H), 2.40-2.09 (m, 9H).

### Example 48: synthesis of compound 50

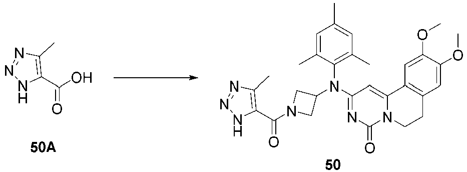

Compound 50A (19 mg) was dissolved in N,N-dimethylformamide (1 mL), and added with HATU (138 mg) and diisopropylethylamine (75 mg). The mixture was stirred for 0.5 hour, and added with compound 32B (65 mg). The reaction mixture was stirred for 4 hours. After the reaction was completed, the mixture was purified by Flash reversed-phase chromatography, and compound 50 (10 mg) was yielded. MS m/z(ESI): 556.3[M+1]⁺.

¹H NMR (400 MHz, DMSO-d₆) δ 7.11 (s, 2H), 6.98 (s, 1H), 6.62-6.24 (m,1 H), 5.27 (s, 1H), 5.03-4.93 (m, 1H), 4.82-4.70 (m, 1H), 4.29-4.18 (m, 2H), 3.95 (t, J = 6.4 Hz, 2H), 3.90-3.85 (m, 1H), 3.81 (s, 3H), 3.73-3.68 (m, 1H), 3.59 (s, 3H), 2.89 (t, J = 6.3 Hz, 2H), 2.41 (d, J = 5.8 Hz, 3H), 2.34-2.28 (m, 3H), 2.12-2.02 (m, 6H).

### Example 49: synthesis of compound 51

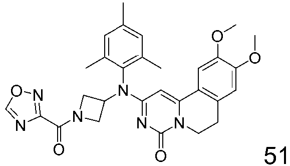

Compound 51 (20mg) was yielded by referring to the synthesis method described in Example 48. MS m/z(ESI): 543.3[M+1]⁺.

¹H NMR (400 MHz, DMSO-d₆) δ 9.82 (s, 1H), 9.20 (t, J = 5.9 Hz, 1H), 7.31 (s, 1H), 7.10 (d, J = 3.2 Hz, 3H), 6.09 (s, 1H), 4.83 (d, J = 9.5 Hz, 1H), 4.60 (t, J = 11.1 Hz, 1H), 4.40 (dd, J = 11.8, 6.9 Hz, 1H), 4.12 (dd, J = 11.7, 5.8 Hz, 2H), 3.87 (s, 3H), 3.76 (s, 3H), 3.72-3.66 (m, 1H), 3.41 (dd, J = 7.9, 5.1 Hz, 1H), 3.01 (t, J = 6.2 Hz, 2H), 2.30 (s, 6H), 2.24 (s, 3H).

### Example 50: synthesis of compound 52

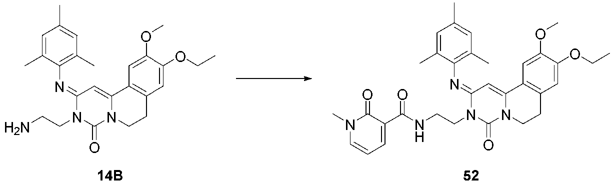

Compound 14B (100 mg), 1-methyl-2-oxo-1,2-dihydropyridine-3-carboxylic acid (39 mg), N,N,N',N'-tetramethylchloroformamidinium hexafluorophosphate (71 mg), and N-methylimidazole (42 mg) were added to N,N-dimethylformamide (2 mL). The reaction mixture was stirred at 25°C for 16 hours. After the reaction was completed, the mixture was purified by Flash reversed-phase chromatography, and compound 52 (10 mg) was yielded. MS m/z(ESI): 584.3[M+1]⁺.

¹H NMR (400 MHz, CDCl₃) δ 10.32 (s, 1H), 8.43 - 8.32 (m, 1H), 7.58 (dd, J = 6.5, 1.9 Hz, 1H), 7.01 (s, 2H), 6.73 (s, 1H), 6.69 (s, 1H), 6.36 (t, J = 6.9 Hz, 1H), 5.61 (s, 1H), 4.71 (d, J = 6.0 Hz, 2H), 4.16 (q, J = 6.8 Hz, 4H), 3.93 (d, J = 6.1 Hz, 2H), 3.74 (s, 3H), 3.65 (s, 3H), 2.98 (t, J = 6.2 Hz, 2H), 2.34 (s, 3H), 2.25 (s, 6H), 1.49 (t, J = 7.0 Hz, 3H).

### Example 51: synthesis of compound 53 and compound 54

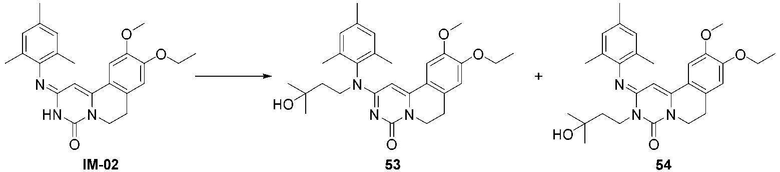

IM-02 (200 mg), 4-bromo-2-methylbutan-2-ol (412 mg), potassium carbonate (635 mg), and potassium iodide (491 mg) were added to 2-butanone (10 mL). The reaction mixture was stirred at 85°C for 16 hours. After the reaction was completed, the mixture was concentrated under reduced pressure. The crude product was purified by silica gel chromatography (dichloromethane/methanol = 20/1), and compound 53 (5 mg) and compound 54 (15 mg) were yielded.

### Compound 53: MS m/z(ESI): 492.3[M+1]⁺.

¹H NMR (400 MHz, CDCl₃) δ 6.99 (s, 2H), 6.68 (s, 1H), 6.64 (s, 1H), 5.32 (s, 1H), 4.15-4.09 (m, 4H), 4.01-3.97 (m, 2H), 3.71 (s, 3H), 2.87 (t, J = 6.2 Hz, 2H), 2.34 (s, 3H), 2.16 (s, 6H), 1.96-1.89 (m, 2H), 1.47 (t, J = 7.0 Hz, 3H), 1.26 (s, 6H).

Compound 54: MS m/z(ESI): 492.2[M+1]⁺.

¹H NMR (400 MHz, DMSO-d₆) δ 6.94 (s, 1H), 6.85 (s, 2H), 6.66 (s, 1H), 5.31 (s, 1H), 4.36 (s, 1H), 4.27-4.16 (m, 2H), 4.06 (q, J = 6.9 Hz, 2H), 3.91 (t, J = 6.0 Hz, 2H), 3.62 (s, 3H), 2.88 (t, J = 5.9 Hz, 2H), 2.22 (s, 3H), 1.96 (s, 6H), 1.85-1.76 (m, 2H), 1.33 (t, J = 6.9 Hz, 3H), 1.17 (s, 6H).

### Example 52: synthesis of compound 55

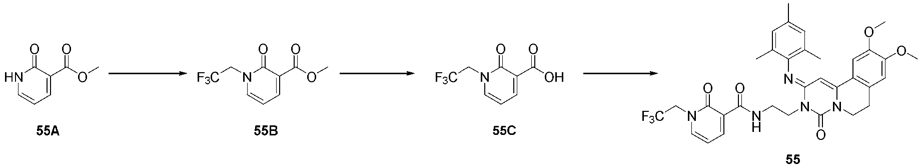

### Step 1, synthesis of compound 55B

Compound 55A (2.0 g), 2,2,2-trifluoroethyl trifluoromethanesulfonate (3.36 g), and cesium carbonate (8.54 g) were dissolved in N,N-dimethylformamide (10 mL). The reaction mixture was stirred at room temperature for 10 hours. After the reaction was completed, water (1 mL) was added to quench the reaction. The resulting mixture was purified by Flash reversed-phase chromatography, and compound 55B (2.0 g) was yielded. MS m/z (ESI): 236.1[M+1] ⁺.

### Step 2, synthesis of compound 55C

Compound 55B (500 mg) was dissolved in a solution of tetrahydrofuran/water (V/V = 1/1, 10 mL), and added with lithium hydroxide (152 mg). The reaction mixture was reacted at 25°C for 6 hours. After the reaction was completed, the mixture was concentrated under reduced pressure, and extracted with ether (3 mL), and the pH was adjusted to 2-3 with 1 M diluted hydrochloric acid. The precipitated solid was filtered, collected, and vacuum-dried, and compound 55C (220 mg) was yielded. MS m/z (ESI): 222.2[M+1] ⁺.

### Step 3, synthesis of compound 55

Compound 55C (51 mg) was dissolved in N,N-dimethylformamide (2 mL), and added with HATU (102 mg) and diisopropylethylamine (53 mg) in sequence. The reaction mixture was stirred at room temperature for 15 minutes, and compound 2 (90 mg) was added. The reaction mixture was stirred for 3 hours at room temperature. After the reaction was completed, water (0.1 mL) was added to quench the reaction. The resulting mixture was purified by reversed-phase chromatography, and compound 55 (22.45 mg) was yielded. MS m/z (ESI): 638.2[M+1] ⁺.

¹H NMR (400 MHz, CDCl3) δ 9.61 (s, 1H), 8.55 (s, 1H), 7.46 (d, J = 6.8 Hz, 1H), 6.85 (s, 2H), 6.71 (s, 1H), 6.66 (s, 1H), 6.43 (t, J = 6.8 Hz, 1H), 5.46 (s, 1H), 4.68 (q, J = 8.5 Hz, 2H), 4.57 (s, 2H), 4.03 (s, 2H), 3.98 - 3.84 (m, 5H), 3.76 (s, 3H), 2.89 (s, 2H), 2.27 (s, 3H), 2.21 - 1.83 (m, 6H).

### Example 53: synthesis of compound 56

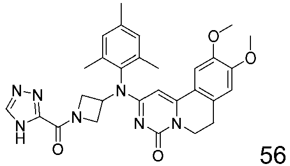

Compound 56 (18mg) was yielded by referring to the synthesis method described in Example 48. MS m/z(ESI): 542.2 [M+1]⁺.

¹H NMR (400 MHz, DMSO-d₆) δ 8.30 (s, 1H), 7.76 (s, 1H), 7.29 (s, 1H), 7.13 (s, 2H), 7.09 (s, 1H), 6.05 (s, 1H), 4.86-4.75 (m, 1H), 4.58-4.42 (m, 2H), 4.18-4.02 (m, 2H), 3.87 (s, 3H), 3.76 (s, 3H), 3.67-3.59 (m, 1H), 3.28 (s, 1H), 3.01 (t, J = 6.9 Hz, 2H), 2.34 (s, 3H), 2.30 (s, 3H), 2.26 (s, 3H).

### Example 54: synthesis of compound 57

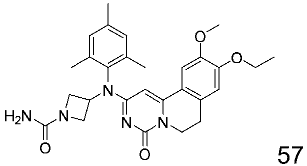

Compound 57 (30mg) was yielded by referring to the synthesis method described in Example 30. MS m/z(ESI): 504.3[M+1]⁺.

¹H NMR (400 MHz, DMSO-d₆) δ 8.15 (s, 3H), 7.33 (s, 1H), 7.15 (s, 2H), 7.10 (s, 1H), 6.12 (s, 1H), 4.81-4.79 (m, 1H), 4.64-4.47 (m, 2H), 4.18-4.09 (m, 4H), 3.76 (s, 3H), 3.38-3.35 (m, 1H), 3.01 (t, J = 6.4 Hz, 2H), 2.75 (d, J = 11.0 Hz, 1H), 2.33-2.29 (m, 6H), 2.18 (s, 3H), 1.36 (t, J = 6.9 Hz, 3H).

### Example 55: synthesis of compound 58

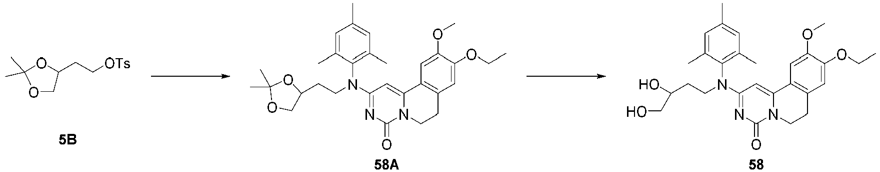

### Step 1, synthesis of compound 58A

Under a nitrogen atmosphere, IM-02 (500 mg) was dissolved in acetonitrile (30 mL), and compound 5B (667 mg), potassium carbonate (1.2 g), and potassium iodide (1.02 g) were added in sequence. The mixture was reacted at 80°C for 72 hours. After the reaction was completed, the reaction mixture was diluted with water (80 mL), and extracted with ethyl acetate (50 mL × 3). The organic phases were combined, washed with saline, dried over anhydrous sodium sulfate, and concentrated under reduced pressure. The resulting crude product was purified by Flash reversed-phase chromatography, and compound 58A (180 mg) was yielded. MS m/z(ESI): 534.3[M+1 ]⁺.

### Step 2, synthesis of compound 58

Compound 58A (100 mg) was dissolved in water (5 mL), and added with a solution of 2 M hydrogen chloride in methanol (5 mL). The reaction mixture was stirred at room temperature for 2 hours. After the reaction was completed, saturated sodium bicarbonate was added to adjust the pH to 7-9. The precipitated solid was filtered to yield a crude product. The crude product was purified by reversed-phase chromatography, and compound 58 (38 mg) was yielded. MS m/z(ESI): 494.2[M+1]⁺.

¹H NMR (400 MHz, Chloroform-d & D₂O) δ 7.02 (s, 1H), 6.99 (s, 1H), 6.69 (s, 1H), 6.63 (s, 1H), 5.34 (s, 1H), 4.63-4.48 (m, 1H), 4.25-4.04 (m, 4H), 3.98- 3.80 (m, 1H), 3.72 (s, 3H), 3.67-3.59 (m, 1H), 3.59-3.49 (m, 1H), 3.45-3.35 (m, 1H), 2.89 (t, J = 6.4 Hz, 2H), 2.34 (s, 3H), 2.16 (s, 3H), 2.14 (s, 3H), 1.80-1.58 (m, 2H), 1.48 (t, J = 7.0 Hz, 3H).

### Example 56: synthesis of compound 59

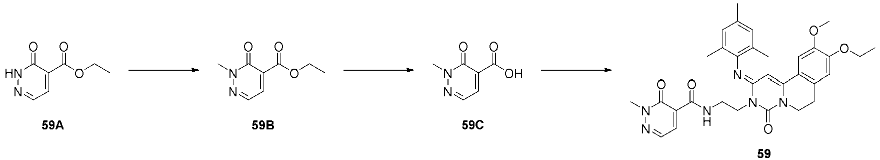

### Step 1, synthesis of compound 59B

Compound 59A (200 mg) was dissolved in N,N-dimethylformamide (5 mL), and added with potassium carbonate (247 mg) and methyl iodide (253 mg) in sequence. The reaction mixture was stirred at 25°C for 16 hours. After the reaction was completed, the reaction mixture was diluted with water (10 mL), and extracted with ethyl acetate (20 mL × 3). The organic phases were combined, washed with saturated saline (10 mL), dried over anhydrous sodium sulfate, filtered, and concentrated under reduced pressure. The obtained crude product was purified by Flash reversed-phase chromatography, and compound 59B (80 mg) was yielded. MS m/z(ESI): 182.7[M+1] ⁺.

### Step 2, synthesis of compound 59C

Compound 59B (200 mg) was dissolved in tetrahydrofuran/water (1/1, 10 mL), and added with lithium hydroxide (53 mg), and the reaction mixture was stirred at 45°C for 2 hours. After the reaction was completed, the mixture was concentrated under reduced pressure, the residue was diluted with water, and the pH was adjusted to 2 with 1 M dilute hydrochloric acid. The precipitated solid was filtered, washed with water and lyophilized, and compound 59C (80 mg) was yielded. MS m/z(ESI): 155.1[M+1] ⁺.

### Step 3, synthesis of compound 59

Compound 59C (18 mg), compound 2 (50 mg), HATU (106 mg), and diisopropylethylamine (43 mg) were dissolved in N,N-dimethylformamide (2 mL), and the mixture was stirred at 25°C for 16 hours. After the reaction was completed, the mixture was purified by Flash reversed-phase chromatography, and compound 59 (10.2 mg) was yielded. MS m/z(ESI): 585.2[M+1] ⁺.

¹H NMR (400 MHz, DMSO-d₆) δ 9.62 (t, J = 6.0 Hz, 1H), 8.13 (d, J = 4.2 Hz, 1H), 8.04 (d, J = 4.2 Hz, 1H), 6.94 (s, 1H), 6.84 (s, 2H), 6.67 (s, 1H), 5.32 (s, 1H), 4.36 (t, J = 6.2 Hz, 2H), 4.06 (q, J = 7.0 Hz, 2H), 3.88 (t, J = 6.0 Hz, 2H), 3.82-3.68 (m, 5H), 3.62 (s, 3H), 2.87 (t, J = 6.2 Hz, 2H), 2.21 (s, 3H), 1.94 (s, 6H), 1.33 (t, J = 7.0 Hz, 3H).

### Example 57: synthesis of compound 60

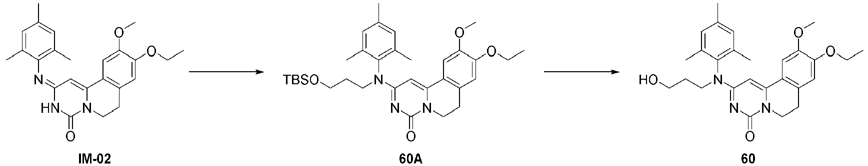

### Step 1, synthesis of compound 60A

IM-02 (1.0 g) was dissolved in toluene (20 mL), and added with 3-(tert-butyldimethylsilyloxy)propanol (0.71 g) and cyanomethylene tri-n-butylphosphorane. Under a nitrogen atmosphere, the mixture was heated to 120°C and stirred for 2 hours of reaction. After the reaction was completed, the mixture was concentrated under reduced pressure. The crude product was purified by Flash reversed-phase chromatography, and compound 60A (280 mg) was yielded. MS m/z(ESI): 578.3[M+1] ⁺.

### Step 2, synthesis of compound 60

Compound 60A (600 mg) was dissolved in dichloromethane (10 mL), and pyridine hydrofluoride solution (617 mg) was added dropwise. The reaction mixture was stirred at room temperature for 1 hour. After the reaction was completed, the mixture was quenched with saturated sodium bicarbonate (2 mL) and extracted with dichloromethane (15 mL × 3). The organic phases were combined, dried over anhydrous sodium sulfate, filtered, and concentrated under reduced pressure. The resulting residue was purified by Flash reversed-phase chromatography, and compound 60 (300 mg) was yielded. MS m/z(ESI): 464.2[M+1]⁺.

¹H NMR (400 MHz, Chloroform-d) δ 7.01 (s, 2H), 6.69 (s, 1H), 6.63 (s, 1H), 5.33 (s, 1H), 4.21-4.08 (m, 4H), 3.99 (t, J = 6.1 Hz, 2H), 3.80-3.73 (m, 2H), 3.71 (s, 3H), 2.89 (t, J = 6.5 Hz, 2H), 2.34 (s, 3H), 2.16 (s, 6H), 1.80 (p, J = 5.7 Hz, 2H), 1.48 (t, J = 7.0 Hz, 3H).

### Example 58: synthesis of compound 61

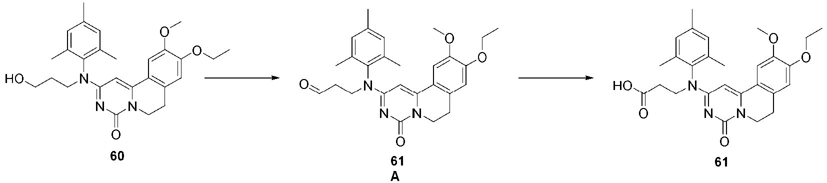

### Step 1, synthesis of compound 61A

Compound 60 (300 mg) was dissolved in DMSO (6 mL), and added with IBX (543.69 mg), and the mixture was stirred at 25°C for 5 hours. After the reaction was completed, water (0.5 mL) was added to quench the reaction. The reaction mixture was purified by Flash reversed-phase chromatography, and compound 61A (200 mg) was yielded. MS m/z(ESI): 462.1[M+1]⁺.

### Step 2, synthesis of compound 61

Compound 61A (200 mg) was dissolved in acetonitrile/tert-butanol/water (2/2/1, 10 mL), and added with sodium dihydrogen phosphate (182.0 mg) and sodium chlorite (117.5 mg) in sequence. The reaction mixture was stirred at 25°C for 1 hour. After the reaction was completed, the reaction was quenched with saturated sodium thiosulfate solution (0.5 mL). The resulting mixture was purified by Flash reversed-phase chromatography, and compound 61 (17 mg) was yielded. MS m/z(ESI): 478.1[M+1]⁺.

¹H NMR (400 MHz, Chloroform-d & D₂O) δ 7.01 (s, 2H), 6.69 (s, 1H), 6.64 (s, 1H), 5.38 (s, 1H), 4.21 - 4.03 (m, 6H), 3.72 (s, 3H), 2.97 (t, J = 7.4 Hz, 2H), 2.90 (t, J = 6.5 Hz, 2H), 2.34 (s, 3H), 2.16 (s, 6H), 1.48 (t, J = 7.0 Hz, 3H).

### Example 59: synthesis of compound 62

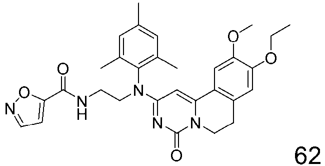

Compound 62 (10 mg) was yielded by referring to the synthesis method described in Example 48. MS m/z(ESI): 544.2[M+1]⁺.

¹H NMR (400 MHz, CDCl3) δ 9.48 (s, 1H), 8.33 (d, J = 1.2 Hz, 1H), 7.30 (d, J = 1.3 Hz, 1H), 7.02 (s, 2H), 6.69 (s, 1H), 6.64 (s, 1H), 5.37 (s, 1H), 4.23-4.07 (m, 6H), 3.76-3.67 (m, 5H), 2.92 (t, J = 6.3 Hz, 2H), 2.34 (s, 3H), 2.16 (s, 6H), 1.48 (t, J = 7.0 Hz, 3H).

### Example 60: synthesis of compound 63

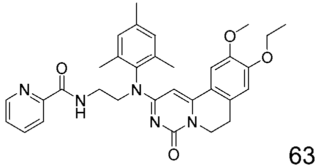

Compound 63 (10 mg) was yielded by referring to the synthesis method described in Example 48. MS m/z(ESI): 554.2[M+1]⁺.

¹H NMR (400 MHz, Chloroform-d) δ 9.02 (s, 1H), 8.60 (d, J = 4.5 Hz, 1H), 8.10 (d, J = 7.8 Hz, 1H), 7.83-7.73 (m, 1H), 7.43-7.33 (m, 1H), 6.98 (s, 2H), 6.68 (s, 1H), 6.65 (s, 1H), 5.38 (s, 1H), 4.21-4.08 (m, 6H), 3.79 (q, J = 6.5 Hz, 2H), 3.71 (s, 3H), 2.89 (t, J = 6.4 Hz, 2H), 2.32 (s, 3H), 2.17 (s, 6H), 1.47 (t, J = 7.0 Hz, 3H).

### Example 61: synthesis of compound 64

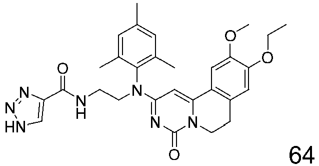

Compound 64 (24 mg) was yielded by referring to the synthesis method described in Example 48. MS m/z(ESI): 544.3[M+1]⁺.

¹H NMR (400 MHz, DMSO-d₆) δ 15.83-15.18 (m, 1H), 8.89-6.79 (m, 6H), 6.79-5.14 (m, 1H), 4.22-3.48 (m, 11H), 2.94-2.84 (m, 2H), 2.35-2.21 (m, 3H), 2.18 -1.91 (m, 6H), 1.44-1.27 (m, 3H).

### Example 62: synthesis of compound 65

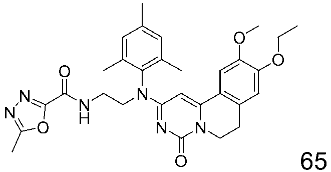

Compound 65 (10 mg) was yielded by referring to the synthesis method described in Example 48. MS m/z(ESI): 559.2[M+1]⁺.

¹H NMR (400 MHz, DMSO-d₆) δ 9.54-9.39 (m, 1H), 8.00-5.20 (m, 5H), 4.15-4.03 (m, 3H), 3.96-3.91 (m, 3H), 3.73-3.54 (m, 4H), 2.91-2.85 (m, 2H), 2.74 -2.56 (m, 3H), 2.29-2.26 (m, 3H), 2.11 (s, 3H), 2.05 (s, 3H), 1.39-1.31 (m, 3H).

### Example 63: synthesis of compound 66

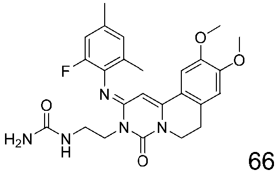

Compound 66 (30 mg) was yielded by referring to the synthesis method described in Example 11. MS m/z(ESI): 482.1[M+1]⁺.

¹H NMR (400 MHz, DMSO-d₆) δ 6.97 (s, 1H), 6.90-6.83 (m, 2H), 6.79 (s, 1H), 6.10 (t, J = 5.8 Hz, 1H), 5.52 (d, J = 1.9 Hz, 1H), 5.43 (s, 2H), 4.17 (t, J = 6.5 Hz, 2H), 3.93 (t, J = 6.1 Hz, 2H), 3.81 (s, 3H), 3.65 (s, 3H), 2.91 (t, J = 5.9 Hz, 2H), 2.26 (s, 3H), 2.08 (s, 3H).

### Example 64: synthesis of compound 67

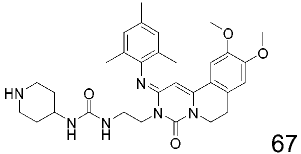

Compound 67 (25 mg) was yielded by referring to the synthesis method described in Example 31. MS m/z(ESI): 561.1[M+1]⁺.

¹H NMR (400 MHz, DMSO-d₆) δ 8.47 (s, 1H), 8.26 (s, 1H), 7.20-6.96 (m, 3H), 6.77 (s, 2H), 6.33 (s, 1H), 5.58 (s, 1H), 4.27 (s, 2H), 4.06 (s, 2H), 3.85 (s, 3H), 3.66-3.55 (m, 5H), 3.47-3.39 (m, 2H), 3.23-3.16 (m, 2H), 3.03-2.88 (m, 4H), 2.32 (s, 3H), 2.20 (s, 6H), 1.90-1.81 (m, 2H), 1.52-1.40 (m, 2H).

### Example 65: synthesis of compound 68

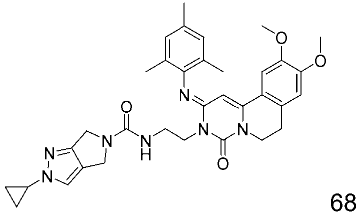

Compound 68 (30 mg) was yielded by referring to the synthesis method described in Example 36. MS m/z (ESI): 610.3 [M+1]⁺.

¹H NMR (400 MHz, DMSO-d₆) δ 7.59 (s, 1H), 6.96 (s, 1H), 6.85 (s, 2H), 6.67 (s, 1H), 6.49 (t, J = 5.5 Hz, 1H), 5.32 (s, 1H), 4.27 (s, 6H), 3.92-3.82 (m, 2H), 3.80 (s, 3H), 3.70-3.64 (m, 1H), 3.62 (s, 3H), 3.48 (q, J = 6.4 Hz, 2H), 2.88 (t, J = 5.8 Hz, 2H), 2.22 (s, 3H), 1.97 (s, 6H), 1.05-0.98 (m, 2H), 0.97-0.89 (m, 2H).

### Example 66: synthesis of compound 69

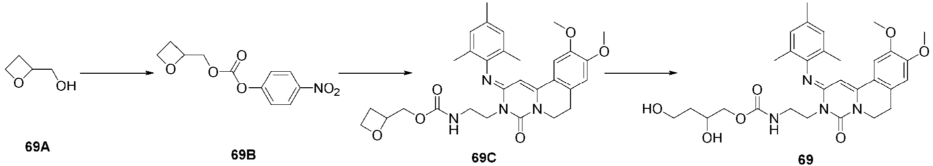

### Step 1, synthesis of compound 69B

Compound 69A (0.48 g) and triethylamine (0.66 g) were dissolved in dichloromethane (15 mL). Then, at room temperature, a solution of p-nitrophenyl chloroformate (1.0 g) in dichloromethane (25 mL) was slowly added dropwise. The reaction mixture was reacted at room temperature for 16 hours. After the reaction was completed, the reaction mixture was concentrated under reduced pressure. The crude product was purified by silica gel column chromatography (petroleum ether/ethyl acetate = 4/1), and compound 69B (0.8 g) was yielded.

¹H NMR (400 MHz, CDCl₃) δ 8.38-8.22 (m, 2H), 7.46-7.34 (m, 2H), 5.18 - 5.01 (m, 1H), 4.79-4.68 (m, 1H), 4.68-4.57 (m, 1H), 4.53-4.37 (m, 2H), 2.89 - 2.72 (m, 1H), 2.72-2.55 (m, 1H).

### Step 2, synthesis of compound 69C

Compound 69B (50 mg) was added to tetrahydrofuran (5 mL), and added with compound 2 (85.8 mg) and triethylamine (180 mg) in sequence. The reaction mixture was stirred at 25°C for 12 hours. After the reaction was completed, the reaction mixture was concentrated under reduced pressure. The crude product was purified by preparative thin-layer chromatography (petroleum ether/ethyl acetate = 4/1), and compound 69C (30 mg) was yielded. MS m/z (ESI): 549.2 [M+1]⁺.

### Step 3, synthesis of compound 69

Compound 69C (80 mg) was added to 0.5 M sulfuric acid (2 mL). The reaction mixture was stirred at 60°C for 24 hours. After the reaction was completed, the reaction mixture was directly purified by reversed-phase preparative chromatography, and compound 69 (15 mg) was yielded.

MS m/z (ESI): 567.2 [M+1]⁺.

¹H NMR (400 MHz, DMSO-d₆) δ 7.22 (t, J = 5.8 Hz, 1H), 6.96 (s, 1H), 6.85 (s, 2H), 6.66 (s, 1H), 5.32 (s, 1H), 4.61 (d, J = 5.3 Hz, 1H), 4.35 (t, J = 5.0 Hz, 1H), 4.24 (t, J = 6.3 Hz, 2H), 3.91 (t, J = 6.0 Hz, 2H), 3.85-3.77 (m, 5H), 3.71 (s, 1H), 3.62 (s, 3H), 3.49 (q, J = 6.0 Hz, 2H), 3.39 (q, J = 6.2 Hz, 2H), 2.90 (t, J = 6.0 Hz, 2H), 2.22 (s, 3H), 1.97 (s, 6H), 1.62-1.50 (m, 1H), 1.50-1.36 (m, 1H).

### Example 67: synthesis of compound 70

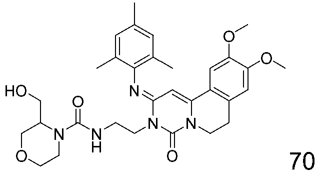

Compound 70 (20 mg) was yielded by referring to the synthesis method described in Example 36.

MS m/z (ESI): 578.3 [M+1]⁺.

¹H NMR (400 MHz, DMSO-d₆) δ 7.12 (s, 2H), 7.09 (s, 1H), 6.96 (s, 1H), 6.80 (s, 1H), 5.61 (s, 1H), 4.31 (s, 2H), 4.08 (t, J = 6.3 Hz, 2H), 3.86 (s, 3H), 3.80-3.74 (m, 1H), 3.70-3.58 (m, 5H), 3.57-3.23 (m, 7H), 3.03-2.89 (m, 3H), 2.32 (s, 3H), 2.22 (s, 3H), 2.21 (s, 3H).

### Example 68: synthesis of compound 71

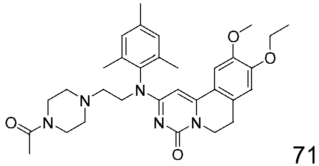

Compound 71 (3 mg) was yielded by referring to the synthesis method described in Example 32.

MS m/z (ESI): 560.2 [M+1]⁺.

¹H NMR (400 MHz, CDCl₃) δ 6.98 (s, 2H), 6.68 (s, 1H), 6.65 (s, 1H), 5.35 (s, 1H), 4.20-4.00 (m, 6H), 3.72 (s, 3H), 3.65-3.26 (m, 4H), 2.88 (t, J = 6.4 Hz, 2H), 2.86-2.34 (m, 6H), 2.33 (s, 3H), 2.17 (s, 6H), 2.07 (s, 3H), 1.47 (t, J = 7.0 Hz, 3H).

### Example 69: synthesis of compound 72 and compound 73

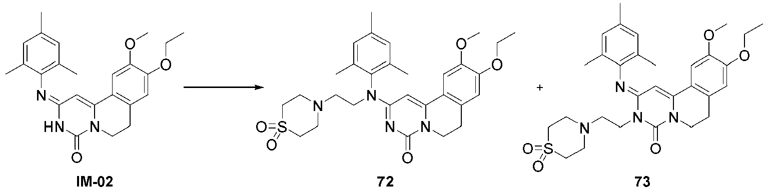

IM-02 (150 mg), 4-(2-hydroxyethyl)thiomorpholin-1,1-dioxide (80 mg), and cyanomethylene tri-n-butylphosphorane (268 mg) were dissolved in toluene (3 mL) in sequence. Under a nitrogen atmosphere, the reaction mixture was stirred and refluxed at 120°C for 2 hours. After the reaction was completed, the reaction mixture was concentrated to yield a crude product. The crude product was purified by reversed-phase chromatography (acetonitrile/0.1% trifluoroacetic acid), and compound 72 (70.4 mg) was yielded.

Repeatedly, materials were added according to the above procedure. The crude product was purified by reversed-phase chromatography (acetonitrile/0.1% aqueous ammonia solution), and compound 73 (40.8 mg) was yielded.

Compound 72: MS m/z (ESI): 567.2 [M+1]⁺.

¹H NMR (400 MHz, CDCl₃) δ 7.02 (s, 2H), 6.71 (s, 1H), 6.64 (s, 1H), 5.42 (s, 1H), 4.26-4.01 (m, 6H), 3.72 (s, 3H), 3.59-3.25 (m, 8H), 3.13 (t, J = 5.8 Hz, 2H), 2.95 (t, J = 6.2 Hz, 2H), 2.34 (s, 3H), 2.18 (s, 6H), 1.49 (t, J = 7.0 Hz, 3H).

Compound 73: MS m/z (ESI): 567.2 [M+1]⁺.

¹H NMR (400 MHz, DMSO-d₆) δ 6.94 (s, 1H), 6.85 (s, 2H), 6.66 (s, 1H), 5.31 (s, 1H), 4.26 (t, J = 6.9 Hz, 2H), 4.06 (q, J = 6.9 Hz, 2H), 3.91 (t, J = 6.1 Hz, 2H), 3.62 (s, 3H), 3.12-3.05 (m, 4H), 3.04-2.98 (m, 4H), 2.92-2.81 (m, 4H), 2.21 (s, 3H), 1.96 (s, 6H), 1.33 (t, J = 6.9 Hz, 3H).

### Example 70: synthesis of compound 74

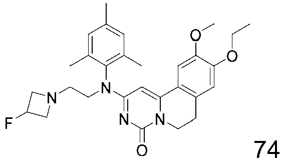

Compound 74 (13.5 mg) was yielded by referring to the synthesis method described in Example 32. MS m/z(ESI): 507.2[M+1]⁺.

### Example 71: synthesis of compound 75

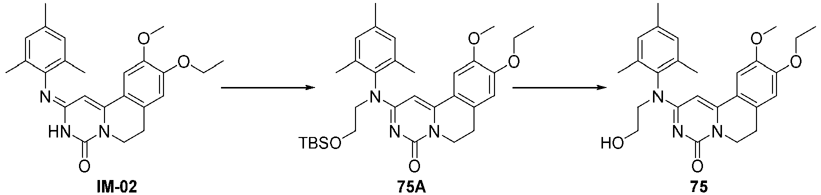

### Step 1, synthesis of compound 75A

IM-02 (250 mg) was dissolved in toluene (5 mL), and then tert-butyl-(2-iodoethoxy)-dimethylsilane (163 mg) and cyanomethylene tri-n-butylphosphorane (446 mg) were added in sequence. Under a nitrogen atmosphere, the mixture was heated to 120°C and stirred for 2 hours. After the reaction was completed, the reaction mixture was concentrated under reduced pressure. The crude product was purified by reversed-phase chromatography, and compound 75A (190 mg) was yielded. MS m/z (ESI): 564.2 [M+1]⁺.

### Step 2, synthesis of compound 75

Compound 75A (200 mg) was dissolved in dichloromethane (3 mL), and a solution of pyridine hydrofluoride (0.75 mL) was added dropwise. The reaction mixture was stirred at room temperature for 2 hours. After the reaction was completed, the mixture was diluted with dichloromethane, and saturated sodium bicarbonate solution was added dropwise to quench the reaction. The organic phase was collected, dried over anhydrous sodium sulfate, and concentrated under reduced pressure. The crude product was purified by reversed-phase chromatography, and compound 75 (97 mg) was yielded. MS m/z (ESI): 450.2 [M+1]⁺.

¹H NMR (400 MHz, Chloroform-d) δ 7.27 (d, J = 1.5 Hz, 1H), 7.00 (s, 2H), 6.72-6.63 (m, 2H), 5.36 (s, 1H), 4.19-4.09 (m, 4H), 4.05-3.98 (m, 2H), 3.93-3.85 (m, 2H), 3.72 (s, 3H), 2.89 (t, J = 6.3 Hz, 2H), 2.34 (s, 3H), 2.20 (s, 6H), 1.48 (t, J = 7.0 Hz, 3H).

### Example 72: synthesis of compound 76

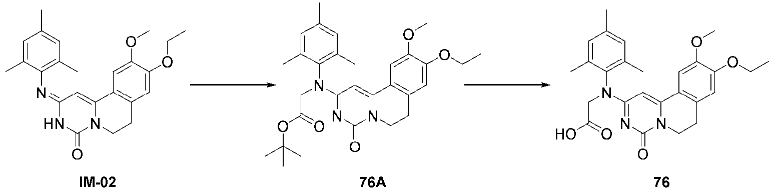

### Step 1, synthesis of compound 76A

IM-02 (1.5 g) was dissolved in N,N-dimethylformamide (20 mL), and then tert-butyl bromoacetate (5.05 g), potassium carbonate (4.6 g), and sodium iodide (3.33 g) were sequentially added. Under a nitrogen atmosphere, the mixture was stirred at 80°C for 16 hours. After the reaction was completed, the reaction mixture was concentrated under reduced pressure to yield a crude product. The crude product was purified by reversed-phase chromatography, and compound 76A (300 mg) was yielded. MS m/z (ESI): 520.5 [M+1]⁺.

### Step 2, synthesis of compound 76

Compound 76A (250 mg) was dissolved in dichloromethane (3 mL), and trifluoroacetic acid (1 mL) was added dropwise. The reaction mixture was stirred at room temperature for 4 hours. After the reaction was completed, the reaction mixture was concentrated under reduced pressure to yield a crude product. The crude product was purified by reversed-phase chromatography, and compound 76 (134 mg) was yielded. MS m/z (ESI): 464.3 [M+1]⁺.

¹H NMR (400 MHz, DMSO-d₆) δ 7.07-6.92 (m, 3H), 6.66-6.45 (m, 1H), 5.36 (s, 1H), 4.59-4.23 (m, 2H), 4.15-4.03 (m, 2H), 3.94 (t, J = 6.3 Hz, 2H), 3.88-3.55 (m, 3H), 2.96-2.82 (m, 2H), 2.35-2.24 (m, 3H), 2.22-2.08 (m, 6H), 1.41-1.27 (m, 3H).

### Example 73: synthesis of compound 77

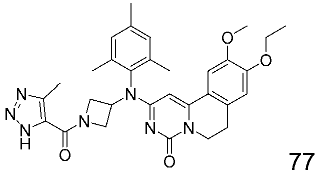

Compound 77 (70 mg) was yielded by referring to the synthesis method described in Example 45.

MS m/z (ESI): 570.3 [M+1]⁺.

¹H NMR (400 MHz, DMSO-d₆) δ 7.78 (s, 1H), 7.26 (s, 1H), 7.12 (d, J = 3.6 Hz, 2H), 7.04 (s, 1H), 5.93 (s, 1H), 4.84 (d, J = 8.1 Hz, 1H), 4.60-4.52 (m, 1H), 4.50-4.42 (m, 1H), 4.21-4.07 (m, 4H), 3.75 (s, 3H), 3.62-3.52 (m, 1H), 3.41-3.33 (m, 1H), 3.01 (s, 2H), 2.33 (s, 3H), 2.29 (s, 3H), 2.28-2.24 (m, 6H), 1.36 (t, J = 6.9 Hz, 3H).

### Example 74: synthesis of compound 78 and compound 79

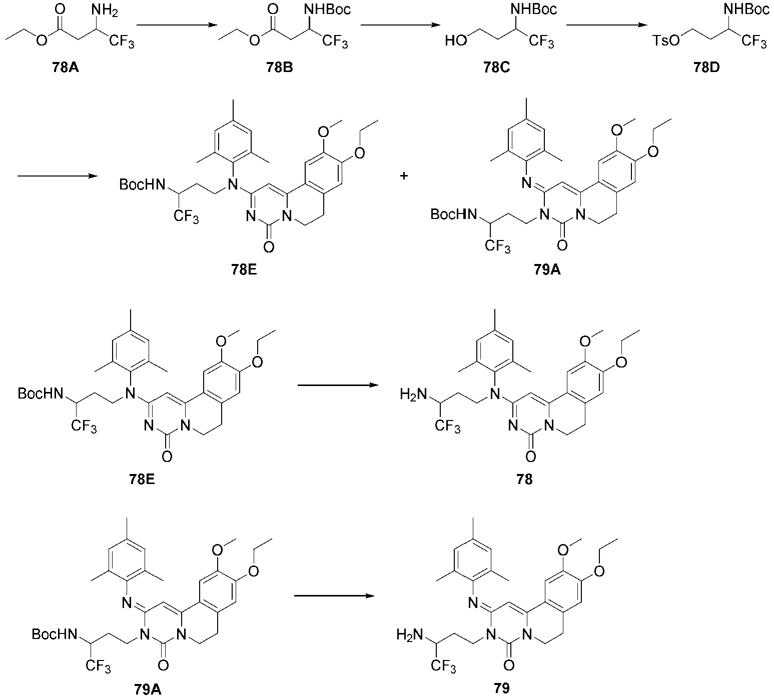

### Step 1, synthesis of compound 78B

Compound 78A (900 mg) and triethylamine (1076 mg) were added to a tetrahydrofuran (10 mL) solution, stirred for 5 minutes, and added with di-tert-butyl dicarbonate (2110 mg). The reaction mixture was stirred at 25°C for 16 hours. After the reaction was completed, the reaction mixture was added with water (10 mL), extracted with ethyl acetate (10 mL × 3). The organic phase was combined, washed with saturated saline (10 mL), dried over anhydrous sodium sulfate, filtered, and concentrated under reduced pressure. The resulting crude product was purified by silica gel column chromatography (petroleum ether/ethyl acetate = 4/1 to 3/2), and compound 78B (1400 mg) was yielded. MS m/z (ESI): 230.1 [M-56+1]⁺.

### Step 2, synthesis of compound 78C

Under a nitrogen atmosphere, a solution of lithium aluminum hydride in tetrahydrofuran (12 mL, 1 mol/L) was added to a three-necked flask. Compound 78B (1.4 g) was dissolved in tetrahydrofuran (15 mL) and added dropwise to the three-necked flask at room temperature. After the addition was completed, the reaction mixture was heated to 25°C and stirred for 3 hours. After the reaction was completed, water (1 mL), 15% sodium hydroxide aqueous solution (1 mL), and water (3 mL) were added in sequence to quench the reaction. Anhydrous sodium sulfate was then added, and the mixture was stirred for 20 minutes. The mixture was filtered, and the filtrate was collected. The filter cake was stirred for 20 minutes in a mixed solution of dichloromethane/methanol (10/1), and filtered again. The filtrates were combined and concentrated under reduced pressure. The resulting crude product was purified by silica gel column chromatography (dichloromethane/methanol = 93/7), and compound 78C (1.0 g) was yielded. MS m/z (ESI): 188.1 [M-56+1]⁺.

### Step 3, synthesis of compound 78D

Compound 78C (1.0 g) and p-toluenesulfonyl chloride (1.3 g) were dissolved in dichloromethane (10 mL), and triethylamine (1.0 g) was added. The reaction mixture was stirred at 25°C for 2 hours. After the reaction was completed, the reaction mixture was concentrated and purified by silica gel column chromatography (dichloromethane/methanol = 20/1), and compound 78D (800 mg) was yielded. MS m/z (ESI): 342.1 [M-56+1]⁺.

### Step 4, synthesis of compound 78E and compound 79A

Compound 78D (800 mg), IM-02 (1221 mg), potassium carbonate (1387 mg), and sodium iodide (903 mg) were added to 2-butanone (20 mL). The reaction mixture was stirred at 85°C for 72 hours. After the reaction was completed, water was added to quench the reaction, and the reaction mixture was extracted with ethyl acetate. The organic phase was dried over anhydrous sodium sulfate and concentrated. The crude product was purified by silica gel column chromatography (dichloromethane/methanol = 20/1), and compound 78E (500 mg) and compound 79A (500 mg) were yielded. Compound 78E: MS m/z (ESI): 631.2 [M+1]⁺. Compound 79A: MS m/z (ESI): 631.3 [M+1]⁺.

### Step 5, synthesis of compound 78

Compound 78E (100 mg) was added to a mixture of trifluoroacetic acid/dichloromethane (1/2, 5 mL). The reaction mixture was stirred at 25°C for 1 hour. After the reaction was completed, the mixture was concentrated under reduced pressure. The crude product was purified by reversed-phase chromatography, and compound 78 (10 mg) was yielded. MS m/z (ESI): 531.2 [M+1]⁺.

¹H NMR (400 MHz, CDCl₃) δ 7.05 (s, 1H), 7.03 (s, 1H), 6.70 (s, 1H), 6.63 (s, 1H), 5.43 (s, 1H), 4.61-4.38 (m, 1H), 4.23-3.99 (m, 5H), 3.72 (s, 4H), 2.92 (s, 2H), 2.47-2.29 (m, 4H), 2.28-2.07 (m, 7H), 1.48 (t, J = 6.9 Hz, 3H).

### Step 6, synthesis of compound 79

Compound 79A (20 mg) was added to a mixture of trifluoroacetic acid/dichloromethane (1/2, 5 mL). The reaction mixture was stirred at 25°C for 1 hour. After the reaction was completed, the mixture was concentrated under reduced pressure. The crude product was purified by reversed-phase chromatography, and compound 79 (5 mg) was yielded. MS m/z (ESI): 531.3 [M+1]⁺.

¹H NMR (400 MHz, DMSO-d₆) δ 7.03 (s, 3H), 6.74 (s, 1H), 5.48 (s, 1H), 4.54-4.18 (m, 4H), 4.17-3.93 (m, 5H), 3.64 (s, 3H), 2.96 (t, J = 6.2 Hz, 2H), 2.29 (s, 3H), 2.10 (s, 6H), 1.34 (t, J = 6.9 Hz, 3H).

### Example 75: synthesis of compound 80

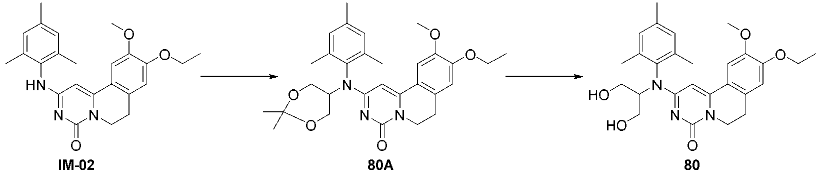

### Step 1, synthesis of compound 80A

IM-02 (300 mg) was added to toluene (10 mL), and added with 2,2-dimethyl-1,3-dioxan-5-ol (147 mg) and cyanomethylene tri-n-butylphosphorane (536 mg). Under a nitrogen atmosphere, the reaction mixture was stirred at 120°C for 2 hours. After the reaction was completed, the mixture was concentrated under reduced pressure. The crude product was purified by silica gel column chromatography (dichloromethane/methanol = 20/1), and compound 80A (100 mg) was yielded. MS m/z (ESI): 520.2 [M+1]⁺.

### Step 2, synthesis of compound 80

Compound 80A (50 mg) was added to acetic acid (5 mL). The reaction mixture was stirred at 25°C for 48 hours. After the reaction was completed, the mixture was concentrated under reduced pressure. The crude product was purified by reversed-phase chromatography, and compound 80 (10 mg) was yielded. MS m/z (ESI): 480.3 [M+1]⁺.

¹H NMR (400 MHz, Chloroform-d) δ 7.00 (s, 2H), 6.69 (s, 1H), 6.59 (s, 1H), 5.30 (s, 1H), 4.36-4.27 (m, 2H), 4.17-4.10 (m, 4H), 4.08-4.02 (m, 2H), 3.70 (s, 3H), 3.68-3.64 (m, 1H), 2.90 (t, J = 6.4 Hz, 2H), 2.33 (s, 3H), 2.18 (s, 6H), 1.48 (t, J = 7.0 Hz, 3H).

### Example 76: synthesis of compound 81

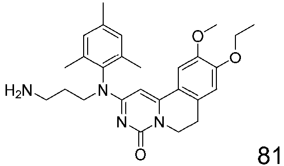

Compound 81 (30 mg) was yielded by referring to the synthesis method described in Example 29. MS m/z (ESI): 463.3 [M+1]⁺.

¹H NMR (400 MHz, CDCl₃) δ 8.97 (s, 3H), 7.03 (s, 2H), 6.70 (s, 1H), 6.63 (s, 1H), 5.42 (s, 1H), 4.14-4.12 (m, 4H), 4.02 (s, 2H), 3.72 (s, 3H), 3.26 (s, 2H), 2.92 (s, 2H), 2.35 (s, 3H), 2.23-2.07 (s, 8H), 1.48 (t, J = 6.5 Hz, 3H).

### Example 77: synthesis of compound 82

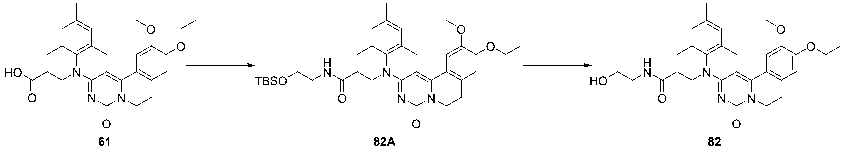

### Step 1, synthesis of compound 82A

Compound 61 (60 mg) was dissolved in N,N-dimethylformamide (2 mL), and added with HATU (72 mg) and diisopropylethylamine (41 mg) in sequence. The mixture was stirred for 15 minutes, added with (2-aminoethoxy)(tert-butyl)dimethylsilane (26 mg), and stirred for 3 hours. After the reaction was completed, water (1 mL) was added to quench the reaction. The reaction mixture was purified by reversed-phase chromatography, and compound 82A (40 mg) was yielded. MS m/z (ESI): 635.2 [M+1]⁺.

### Step 2, synthesis of compound 82

Compound 82A (45 mg) was dissolved in dichloromethane (3 mL), and pyridine hydrofluoride solution (105 mg) was added at 0°C. After the addition was completed, the reaction mixture was heated to room temperature and stirred for 1 hour. After the reaction was completed, saturated sodium bicarbonate aqueous solution (3 mL) was added to quench the reaction. The mixture was diluted with dichloromethane (20 mL) and subjected to phase separation, and the organic phase was concentrated to yield a crude product. The crude product was purified by reversed-phase chromatography, and compound 82 (15.2 mg) was yielded. MS m/z (ESI): 521.3 [M+1]⁺.

¹H NMR (400 MHz, Chloroform-d) δ 9.25 (s, 1H), 7.00 (s, 2H), 6.70 (s, 1H), 6.65 (s, 1H), 5.41 (s, 1H), 4.32-4.08 (m, 4H), 4.03 (t, J = 6.5 Hz, 2H), 3.91-3.81 (m, 2H), 3.72 (s, 3H), 3.48 (q, J = 4.7 Hz, 2H), 2.91 (t, J = 6.5 Hz, 2H), 2.77 (t, J = 6.4 Hz, 2H), 2.34 (s, 3H), 2.13 (s, 6H), 1.48 (t, J = 7.0 Hz, 3H).

### Example 78: synthesis of compound 83

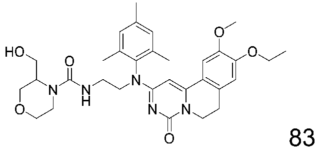

Compound 83 (15 mg) was yielded by referring to the synthesis method described in Example 46.

MS m/z (ESI): 592.2 [M+1]⁺.

¹H NMR (400 MHz, Chloroform-d) δ 7.01 (s, 2H), 6.70 (s, 1H), 6.63 (s, 1H), 5.40 (s, 1H), 4.29-4.03 (m, 8H), 3.93-3.83 (m, 3H), 3.79-3.65 (m, 5H), 3.65-3.57 (m, 1H), 3.53-3.42 (m, 3H), 3.13-3.00 (m, 1H), 2.95-2.85 (m, 2H), 2.34 (s, 3H), 2.16 (s, 3H), 2.14 (s, 3H), 1.48 (t, J = 7.0 Hz, 3H).

### Example 79: synthesis of compound 84

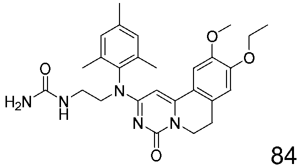

Compound 84 (10 mg) was yielded by referring to the synthesis method described in Example 43.

MS m/z (ESI): 492.2 [M+1]⁺.

¹H NMR (400 MHz, Chloroform-d) δ 6.99 (s, 2H), 6.69 (s, 1H), 6.65 (s, 1H), 5.36 (s, 1H), 4.18-4.08 (m, 4H), 3.92 (s, 2H), 3.72 (s, 3H), 3.53-3.43 (m, 2H), 2.90 (t, J = 6.3 Hz, 2H), 2.33 (s, 3H), 2.15 (s, 6H), 1.48 (t, J = 7.0 Hz, 3H).

### Example 80: synthesis of compound 85

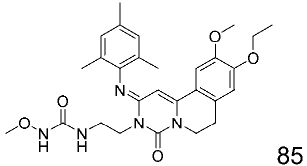

Compound 85 (61 mg) was yielded by referring to the synthesis method described in Example 7.

MS m/z (ESI): 522.2 [M+1]⁺.

¹H NMR (400 MHz, Chloroform-d) δ 7.81 (s, 1H), 6.99 (s, 2H), 6.73 (s, 1H), 6.61 (s, 1H), 5.55 (s, 1H), 4.67 (s, 2H), 4.25-4.12 (m, 4H), 3.85 (s, 2H), 3.72 (s, 3H), 3.68 (s, 3H), 3.01 (t, J = 6.4 Hz, 2H), 2.33 (s, 3H), 2.22 (s, 6H), 1.50 (t, J = 7.0 Hz, 3H).

### Example 81: synthesis of compound 86

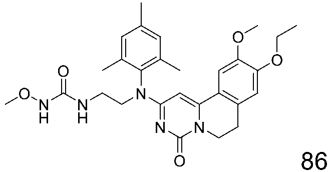

Compound 86 (15 mg) was yielded by referring to the synthesis method described in Example 7.

MS m/z (ESI): 522.2 [M+1]⁺.

¹H NMR (400 MHz, Chloroform-d) δ 6.99 (s, 2H), 6.68 (s, 1H), 6.64 (s, 1H), 5.36 (s, 1H), 4.18-4.06 (m, 6H), 3.73 (s, 3H), 3.71 (s, 3H), 3.58-3.51 (m, 2H), 2.89 (t, J = 6.4 Hz, 2H), 2.33 (s, 3H), 2.15 (s, 6H), 1.47 (t, J = 7.0 Hz, 3H).

### Example 82: synthesis of compound 87

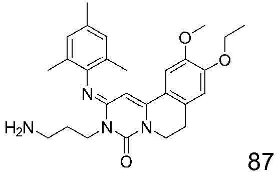

Compound 87 (30 mg) was yielded by referring to the synthesis method described in Example 15.

MS m/z(ESI): 463.3[M+1]⁺.

¹H NMR (400 MHz, DMSO-d₆) δ 7.96 (s, 3H), 7.31-7.02 (m, 4H), 6.75 (s, 1H), 5.53 (s, 1H), 4.27 (t, J = 6.9 Hz, 2H), 4.14-4.09 (m, 2H), 4.08-4.01 (m, 2H), 3.64 (s, 3H), 3.06-2.90 (m, 4H), 2.32 (s, 3H), 2.23-2.10 (m, 6H), 2.09-2.02 (m, 2H), 1.35 (t, J = 6.9 Hz, 3H).

### Experimental Example 1: activity assay 1 - inhibitory effect on PDE3A and PDE4B1 enzymes

1. Main reagents, consumables, and instruments for in vitro detection of inhibitory effect are shown in Table 1 and Table 2.

**Table 1 Main reagents and consumables for in vitro detection of inhibitory effect**

| Reagents (consumables) | Manufacturer |
|---|---|
| PDE3A | BPS |
| PDE4B1 | Sigma |
| cAMP (1mM) | Promega |
| Trequinsin hydrochloride (PDE3 inhibitor) | MCE |
| Rolipram (PDE4 inhibitor) | Sigma |
| AMP-Glo^{™} Assay Kit | Promega |
| Tris-HCl (pH8.0) | Invitrogen |
| MgCl₂ (1M) | Sigma |
| BSA | Sigma |
| Tween-20 | Sigma |
| 384-well low dead volume microplate | LABCYTE |
| Optiplate-384 assay plate | PerkinElmer |

**Table 2 Main instruments for in vitro detection of inhibitory effect**

| Instrument | Type | Manufacturer |
|---|---|---|
| Automated liquid handling workstation | Bravo G5263AA | Agilent |
| Centrifuge | Eppendorf Centrifuge 5910 Ri | Eppendorf |
| Multimode microplate reader | EnVision^{®} 2105 | PerkinElmer |

2. Preparation of experimental buffers
Reaction buffer (5×): 50 mM Tris-HCl (buffer), 50 mM MgCl₂, 0.5% BSA, 0.25% Tween-20, pH 7.4, stored at -20°C.
3. Experimental procedures
1) Test samples and reference substances were prepared to certain concentrations by DMSO, and the reaction buffer was diluted from 5× to 1× using ultrapure water.
2) Using Bravo, test samples and reference substances were serially diluted, and 1 µL of the diluted solution was transferred into an assay plate (Optiplate-384 assay plate) according to the pre-designed compound layout. The starting concentration of the reference substance serial dilution was the Low control, and 1% DMSO-reaction buffer served as the High control.
3) The enzyme was diluted to the desired concentration with 1× reaction buffer, and 4 µL of the diluted solution was transferred to the assay plate. After centrifugation, the 384-well low dead-volume microplate was sealed with sealing film and incubated in the incubator at 23°C for 15 minutes.
4) The substrate cAMP was diluted to the desired concentration using 1× reaction buffer, and 5 µL of the diluted solution was transferred to the assay plate. After centrifugation, the plate was sealed again and incubated in the incubator at 23°C for 60 minutes (PDE3A) or 10 minutes (PDE4B1).
5) Then, 5 µL of Reagent I from the AMP-Glo^{™} Assay Kit was added to the assay plate. After centrifugation and sealing, the plate was incubated in the incubator at 23°C for 60 minutes.
6) The detection solution was prepared according to the AMP-Glo^{™} Assay Kit instructions, and 10 µL of the detection solution was added to the assay plate. After centrifugation and sealing, the plate was incubated in the incubator at 23°C for 60 minutes.
7) The readout was recorded using a multimode microplate reader.

4. Result analysis
% Inhibition of each sample well was calculated according to the formula: % Inhibition=100%-(Sample value - Low control value) / (High control value - Low control value) ×100%.
IC₅₀ values were calculated by fitting the data using the "log(antagonist) vs. response --Variable slope" model in GraphPad Prism 5.0.

5. Experimental result is shown in Table 3:

**Table 3 Experimental result of in vitro PDE3 and PDE4 inhibitory effect**

| Compound | PDE3A (IC50, nM) | PDE4B1 (IC50, nM) | Compound | PDE3A (IC50, nM) | PDE4B1 (IC50, nM) |
|---|---|---|---|---|---|
| 1 | 0.11 | 298.4 | 2 | 4.39 | 5791 |
| 3 | 1.24 | 215.5 | 4 | 0.19 | 5831 |
| 5 | 0.10 | 974.4 | 6 | 0.026 | 121.2 |
| 7 | 0.85 | 51.3 | 8 | 0.86 | 377 |
| 9 | 5.66 | 113.9 | 10 | 3.33 | 49.7 |
| 11 | 117 | - | 12 | 7.45 | 337 |
| 13 | 44.59 | - | 14 | 0.82 | 9.3 |
| 15 | 214.3 | 6713 | 16 | 6.63 | - |
| 17 | 0.54 | 4998 | 18 | 0.088 | 213 |
| 19 | 0.53 | 62.1 | 20 | 24.95 | 251.4 |
| 21 | 1003 | 6914 | 22 | 3.30 | 727.6 |
| 23 | 0.0058 | 12.9 | 24 | 1007 | - |
| 25 | 4.47 | 381.1 | 26 | 0.34 | 418.8 |
| 27 | 2.16 | 918.3 | 28 | 0.46 | 478.0 |
| 29 | 4.78 | 423.3 | 30 | 3.23 | 26.3 |
| 31 | 0.57 | 193.2 | 32 | 0.21 | 237.5 |
| 33 | 4.78 | 3764 | 34 | 6.40 | - |
| 35 | 0.56 | 1277 | 36 | 0.90 | 529.7 |
| 37 | 60.13 | - | 38 | 1.61 | 1887 |
| 39 | 0.72 | 18.7 | 40 | 1.29 | 82.1 |
| 41 | 1.14 | 250.2 | 42 | 0.62 | 75.3 |
| 43 | 0.19 | 13.6 | 44 | 0.081 | 223.1 |
| 45 | 0.032 | 100 | 46 | 0.082 | 17.8 |
| 47 | 72.29 | - | 48 | 0.16 | 579 |
| 49 | 324.8 | - | 50 | 0.0050 | 67.4 |
| 51 | 89.55 | 1451 | 52 | 0.37 | 0.89 |
| 53 | 0.03 | 7.7 | 54 | 0.43 | 696.7 |
| 55 | 0.49 | 261.8 | 56 | 0.82 | 8882 |
| 57 | 25.91 | 997.5 | 58 | 0.012 | 8.1 |
| 59 | 0.76 | 27.6 | 60 | 0.0033 | 6.3 |
| 61 | 0.0061 | 11.1 | 62 | 0.055 | 2.6 |
| 63 | 0.042 | 1.2 | 64 | 0.019 | 8.4 |
| 65 | 0.036 | 10.8 | 66 | 0.64 | 268.4 |
| 67 | 5.49 | 3795 | 68 | 1.82 | 334 |
| 69 | 1.91 | 6754 | 70 | 1.24 | 2834 |
| 71 | 0.057 | 13.2 | 72 | 0.074 | 5.0 |
| 73 | 0.53 | 413.9 | 74 | 0.05 | 8.1 |
| 75 | 0.006 | 9.6 | 76 | 0.10 | 171.8 |
| 77 | 24.68 | 6731 | 78 | 0.027 | 8.9 |
| 79 | 0.33 | 2858 | 80 | 0.0028 | 7.4 |
| 81 | 0.33 | 64.4 | 82 | 0.088 | 14.2 |
| 83 | 0.043 | 37.3 | 84 | 0.008 | 7.5 |
| 85 | 0.31 | 40.5 | 86 | 0.034 | 7.9 |
| 87 | 3.02 | 359.2 | Ensifentrine | 0.17 | 102.3 |
| Trequinsin Hydrochlorid | 0.2272 | - | Rolipram | - | 40.17 |

### Experimental Example 2: activity assay 1- inhibitory effect on CYP (Cytochrome P450 Enzymes)

### 1. Experimental Procedures

1.1 Working solutions (100×) of sample compounds and standard inhibitors were prepared.

1.2 Microsomes were removed from a -80°C refrigerator, thawed on ice, labeled with the date, and stored in the refrigerator immediately after use.

1.3 20 µL of substrate solution was added to the corresponding wells, while 20 µL of PB (phosphate buffer) was added to the blank wells.

1.4 2 µL of sample compound solution and positive control working solution were added to the appropriate wells, while 2 µL of solvent was added to inhibitor-free wells and blank wells.

1.5 HLM (human liver microsome) working solution was prepared.

1.6 158 µL of HLM working solution was added to all wells of the plate.

1.7 The plate was pre-incubated in a 37°C water bath for approximately 10 minutes.

1.8 NADPH (nicotinamide adenosine denucleotide hydro-phosphoric acid, i.e. reduced form coenzyme II) cofactor solution was prepared.

1.9 20 µL of NADPH cofactor solution was added to all incubation wells.

1.10 The solution was mixed and the plate was placed in 37°C water bath to incubate CYP for 10 minutes.

1.11 At designated time points, the reaction was terminated by adding 400 µL of cold stopping solution.

1.12 Samples were centrifuged at 4000 rpm for 20 minutes to precipitate proteins.

1.13 200 µL of the supernatant was transferred into 100 µL of HPLC-grade water and vortexed for 10 minutes.

1.14 Samples were subjected to LC/MS/MS analysis.

### 2. Experimental data analysis

Concentration-inhibition percentage curves of the control compound and test compounds were plotted using XL Fit or SigmaPlot, followed by nonlinear regression analysis.

IC₅₀ values were determined using a 3 or 4 parameter logistic equation. When the inhibition percentage at the highest concentration (50 µM) was below 50%, the IC₅₀ value was reported as ">50 µM".

### 3. Experimental result is shown in the table below:

**Table 4 Experimental result of in vitro CYP inhibitory effect**

| Compound | IC₅₀ (µM) | | | | |
|---|---|---|---|---|---|
| | CYP1A2 | CYP2C9 | CYP2C19 | CYP2D6 | CYP3A-M |
| Ensifentrine (RPL554) | >50 | 3.52 | 23.2 | 41.5 | 8.6 |
| 60 | >50 | 3.87 | 42.1 | >50 | >50 |
| 61 | >50 | >50 | >50 | >50 | >50 |
| 75 | >50 | 8.31 | 30.6 | >50 | 33.2 |
| 80 | >50 | 10.1 | 31.5 | >50 | 43.5 |
| 82 | >50 | 9.35 | >50 | >50 | 42.8 |
| 84 | >50 | 4.52 | >50 | >50 | >50 |
| 86 | >50 | 3.13 | 29.1 | 29.7 | 1.63 |

The results in Table 4 indicated that, compared with Ensifentrine, certain compounds of the present disclosure exhibited no inhibitory effect on CYP (cytochrome enzymes).

The above examples are provided solely for the purpose of facilitating the understanding of the methods and core concepts of the present disclosure. It should be noted that, for those skilled in the art, various improvements and modifications may be made to the present disclosure without departing from its fundamental principles, and such improvements and modifications shall also fall within the scope of protection defined by the claims of the present disclosure.

## Claims

1. A compound having a structure represented by formula (I), or a deuterated compound, stereoisomer, or pharmaceutically acceptable salt thereof: 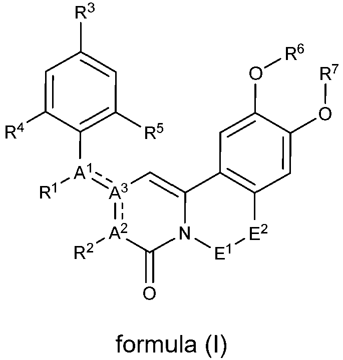
wherein, A¹ and A² are nitrogen atoms, A³ is a carbon atom;
in the case A¹---A³ is a single bond and A³---A² is a double bond, R² is absent;
in the case A¹---A³ is a double bond and A³---A² is a single bond, R' is absent;
R¹, R², R⁶ and R⁷ are each independently selected from the group consisting of hydrogen, substituted or unsubstituted linear or branched C₁-C₆ alkyl, substituted or unsubstituted C₂-C₆ alkenyl, substituted or unsubstituted C₂-C₆ alkynyl, and CₙH₂ₙR⁸,
n is an integer selected from 0 to 6;
R⁸ is one or more selected from the group consisting of substituted or unsubstituted 3-10 membered cycloalkyl, substituted or unsubstituted 4-10 membered heterocycloalkyl, substituted or unsubstituted 6-10 membered aryl, substituted or unsubstituted 5-10 membered heteroaryl, -OR^{a}, -OC(O)R^{a}, -OC(O)NR^{a}R^{b}, -C(O)R^{a}, -C(O)OR^{a}, -C(O)NR^{a}R^{b}, -NR^{a}R^{b}, -NR^{a}C(O)R^{b}, -NR^{a}C(O)OR^{b}, -NR^{a}C(O)NR^{b}CₙH₂ₙR^{c}, -SR^{a}, -S(O)R^{a}, -S(O)NR^{a}R^{b}, -S(O)₂R^{a}, -S(O)₂NR^{a}R^{b}, -NR^{a}S(O)₂R^{b}, -CN, -NH₂ and -CF₃;
R³, R⁴ and R⁵ are independently one or more selected from the group consisting of hydrogen, halogen, and substituted or unsubstituted linear or branched C₁-C₆ alkyl;
R^{a}, R^{b} and R^{c} are independently one or more selected from the group consisting of hydrogen, deuterium, oxo, thio, halogen, amino, methylimino, methoxyimino, methanesulfonyl, hydroxy, cyano, nitro, substituted or unsubstituted linear or branched C₁-C₆ alkyl, substituted or unsubstituted C₁-C₄ alkoxy, substituted or unsubstituted C₂-C₆ alkenyl, substituted or unsubstituted C₂-C₆ alkynyl, substituted or unsubstituted 3-10 membered cycloalkyl, substituted or unsubstituted 4-10 membered heterocycloalkyl, substituted or unsubstituted 6-10 membered aryl, and substituted or unsubstituted 5-10 membered heteroaryl;
E¹ is selected from -(CH₂)ₘ-, wherein m is 1, 2, or 3;
E² is selected from the group consisting of -O-, -NH-, -S-, 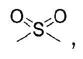 , and -CR⁹R¹⁰-;
R⁹ and R¹⁰ are independently one or more selected from the group consisting of hydrogen, deuterium, and substituted or unsubstituted linear or branched C₁-C₆ alkyl; or
R⁶ and R⁷ together with the adjacent oxygen atom and carbon atom of phenyl form a ring;
wherein the ring is a substituted or unsubstituted 5 or 6 membered monocyclic heterocyclyl;
optionally, the 5 or 6 membered monocyclic heterocyclyl is further fused or spiro-fused with aryl, heteroaryl, or heterocycloalkyl to form a fused ring group, a spiro ring group, or a bridged ring group.

2. The compound according to claim 1, wherein the substituted 3-10 membered cycloalkyl, substituted 4-10 membered heterocycloalkyl, substituted 6-10 membered aryl, or substituted 5-10 membered heteroaryl is substituted by a substituent selected from the group consisting of deuterium, oxo, thio, halogen, hydroxy, cyano, nitro, substituted or unsubstituted linear or branched C₁-C₆ alkyl, substituted or unsubstituted C₃-C₁₀ cycloalkyl, substituted or unsubstituted C₂-C₆ alkenyl, substituted or unsubstituted C₂-C₆ alkynyl, CₙH₂ₙOR^{a}, -CₙH₂ₙOC(O)R^{a}, -CₙH₂ₙOC(O)NR^{a}R^{b}, -CₙH₂ₙC(O)R^{a}, -CₙH₂ₙC(O)OR^{a}, - CₙH₂ₙC(O)NR^{a}R^{b}, -CₙH₂ₙNR^{a}R^{b}, -CₙH₂ₙNR^{a}C(O)R^{b}, -CₙH₂ₙNR^{a}C(O)OR^{b}, - CₙH₂ₙNR^{a}C(O)NR^{b}CₙH₂ₙR^{c}, -CₙH₂ₙSR^{a}, -CₙH₂ₙS(O)R^{a}, -CₙH₂ₙS(O)NR^{a}R^{b}, -CₙH₂ₙS(O)₂R^{a}, -CₙH₂ₙS(O)₂NR^{a}R^{b}, -CₙH₂ₙNR^{a}S(O)₂R^{b}, -CₙH₂ₙCN, -CₙH₂ₙNH₂, -CₙH₂ₙCF₃ and a combination thereof.

3. The compound according to claim 1, wherein the substituted linear or branched C₁-C₆ alkyl, or substituted C₁-C₄ alkoxy is substituted by a substituent selected from the group consisting of deuterium, oxo, thio, halogen, hydroxy, cyano, nitro, CₙH₂ₙOR^{a}, - CₙH₂ₙOC(O)R^{a}, -CₙH₂ₙOC(O)NR^{a}R^{b}, -CₙH₂ₙC(O)R^{a}, -CₙH₂ₙC(O)OR^{a}, -CₙH₂ₙC(O)NR^{a}R^{b}, -CₙH₂ₙNR^{a}R^{b}, -CₙH₂ₙNR^{a}C(O)R^{b}, -CₙH₂ₙNR^{a}C(O)OR^{b}, -CₙH₂ₙNR^{a}C(O)NR^{b}CₙH₂ₙR^{c}, - CₙH₂ₙSR^{a}, -CₙH₂ₙS(O)R^{a}, -CₙH₂ₙS(O)NR^{a}R^{b}, -CₙH₂ₙS(O)₂R^{a}, -CₙH₂ₙS(O)₂NR^{a}R^{b}, - CₙH₂ₙNR^{a}S(O)₂R^{b}, -CₙH₂ₙCN, -CnH₂ₙNH₂, -CₙH₂ₙCF₃ and a combination thereof;
wherein the substituted C₂-C₆ alkenyl is substituted by a substituent selected from the group consisting of deuterium, oxo, thio, halogen, hydroxy, cyano, nitro, substituted or unsubstituted C₁-C₆ alkyl, substituted or unsubstituted C₂-C₆ alkynyl, substituted or unsubstituted C₃-C₁₀ cycloalkyl, CₙH₂ₙOR^{a}, -CₙH₂ₙOC(O)R^{a}, -CₙH₂ₙOC(O)NR^{a}R^{b}, - CₙH₂ₙC(O)R^{a}, -CₙH₂ₙC(O)OR^{a}, -CₙH₂ₙC(O)NR^{a}R^{b}, -CₙH₂ₙNR^{a}R^{b}, -CₙH₂ₙNR^{a}C(O)R^{b}, - CₙH₂ₙNR^{a}C(O)OR^{b}, -CₙH₂ₙNR^{a}C(O)NR^{b}CₙH₂ₙR^{c}, -CₙH₂ₙSR^{a}, -CₙH₂ₙS(O)R^{a}, - CₙH₂ₙS(O)NR^{a}R^{b}, -CₙH₂ₙS(O)₂R^{a}, -CₙH₂ₙS(O)₂NR^{a}R^{b}, -CₙH₂ₙNR^{a}S(O)₂R^{b}, -CₙH₂ₙCN, - CₙH₂ₙNH₂, -CₙH₂ₙCF₃ and a combination thereof;
wherein the substituted C₂-C₆ alkynyl is substituted by a substituent selected from the group consisting of deuterium, oxo, thio, halogen, hydroxy, cyano, nitro, substituted or unsubstituted C₁-C₆ alkyl, substituted or unsubstituted C₂-C₆ alkenyl, substituted or unsubstituted C₃-C₁₀ cycloalkyl, CₙH₂ₙOR^{a}, -CₙH₂ₙOC(O)R^{a}, -CₙH₂ₙOC(O)NR^{a}R^{b}, - CₙH₂ₙC(O)R^{a}, -CₙH₂ₙC(O)OR^{a}, -CₙH₂ₙC(O)NR^{a}R^{b}, -CₙH₂ₙNR^{a}R^{b}, -CₙH₂ₙNR^{a}C(O)R^{b}, - CₙH₂ₙNR^{a}C(O)OR^{b}, -CₙH₂ₙNR^{a}C(O)NR^{b}CₙH₂ₙR^{c}, -CₙH₂ₙSR^{a}, -CₙH₂ₙS(O)R^{a}, - CₙH₂ₙS(O)NR^{a}R^{b}, -CₙH₂ₙS(O)₂R^{a}, -CₙH₂ₙS(O)₂NR^{a}R^{b}, -CₙH₂ₙNR^{a}S(O)₂R^{b}, -CₙH₂ₙCN, - CₙH₂ₙNH₂, -CₙH₂ₙCF₃ and a combination thereof.

4. The compound according to claim 1, wherein R⁶ and R⁷ in formula (I) together with the adjacent oxygen atom and carbon atom of phenyl form a 5 or 6 membered monocyclic heterocyclyl selected from the group consisting of 1,3-dioxolanyl and 1,4-dioxanyl.

5. The compound according to claim 1, wherein the 5 or 6 membered monocyclic heterocyclyl is fused or spiro-fused with aryl selected from the group consisting of substituted or unsubstituted phenyl and substituted or unsubstituted naphthyl;
wherein the 5 or 6 membered monocyclic heterocyclyl is fused or spiro-fused with heteroaryl selected from the group consisting of substituted or unsubstituted pyridinyl, indolyl, quinoxalinyl, quinolinyl, isoquinolinyl, benzothiophenyl, benzofuryl, benzopyranyl, furyl, pyrrolyl, thiazolyl, oxazolyl, isoxazolyl, triazolyl, tetrazolyl, pyrazolyl, imidazolyl, thiophenyl, oxadiazolyl, benzimidazolyl, benzothiazolyl, benzoxazolyl, and a combination thereof; or
wherein the 5 or 6 membered monocyclic heterocyclyl is fused or spiro-fused with heterocycloalkyl selected from the group consisting of substituted or unsubstituted oxetanyl, azetidinyl, azepanyl, diazepanyl, oxazepanyl, 8-aza-bicyclo[3.2.1]octyl, 8-oxa-3-aza-bicyclo[3.2.1]octyl, 9-aza-bicyclo[3.3.1]nonyl, 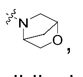 , pyrrolidinyl, 2-oxo-pyrrolidin-3-yl, tetrahydrofuranyl, tetrahydrothiophenyl, pyrazolidinyl, imidazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, homopiperazinyl, quinuclidinyl, dihydrofuranyl, imidazolinyl, tetrahydropyridinyl, dihydropyranyl and a combination thereof.

6. The compound according to claim 1, wherein the substituted 5 or 6 membered monocyclic heterocyclyl, substituted aryl, substituted heteroaryl, or substituted heterocycloalkyl is independently substituted by a substituent selected from the group consisting of substituted or unsubstituted linear or branched C₁-C₆ alkyl, substituted or unsubstituted C₁-C₄ alkoxy, halogen, hydroxy, nitro, cyano, amino and a combination thereof.

7. The compound according to claim 1, wherein the compound has a structure selected from the group consisting of: 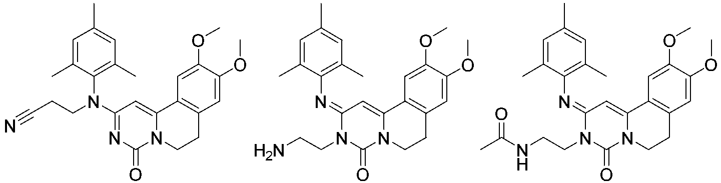 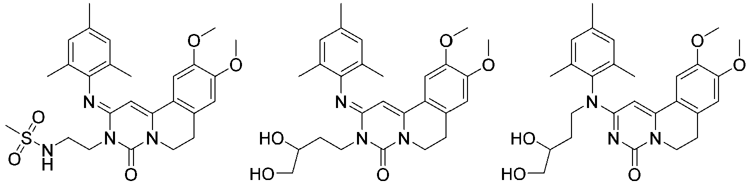 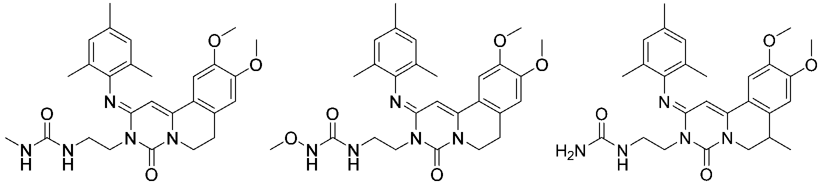 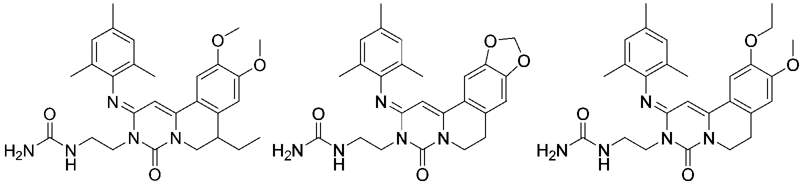 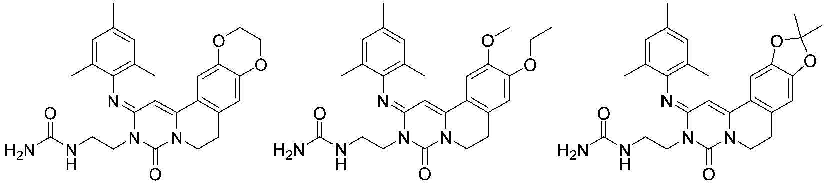 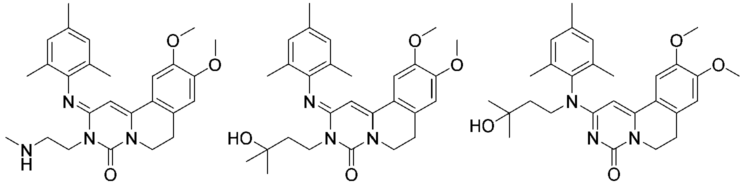 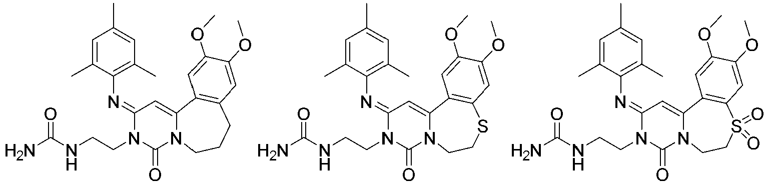 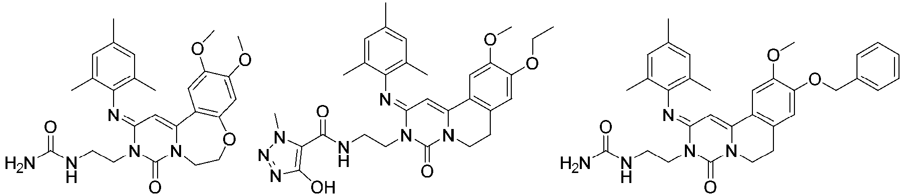 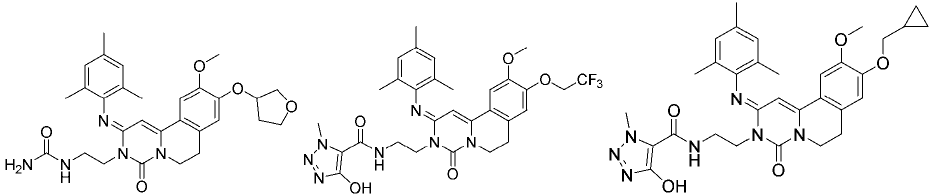 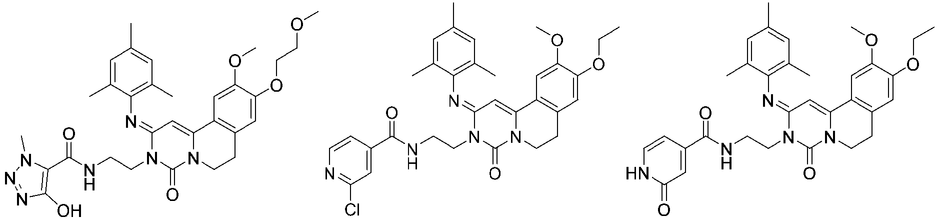 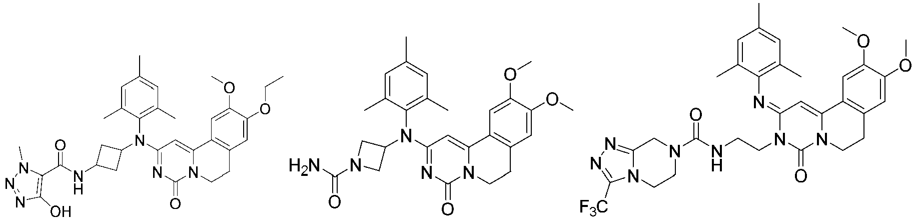 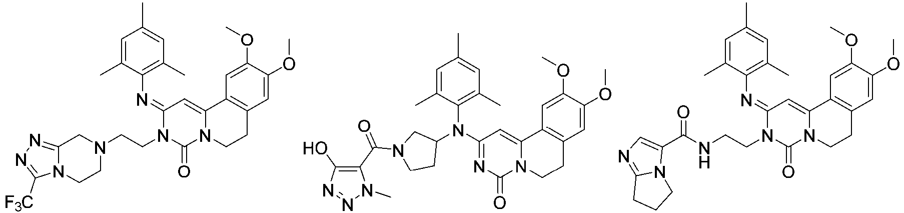 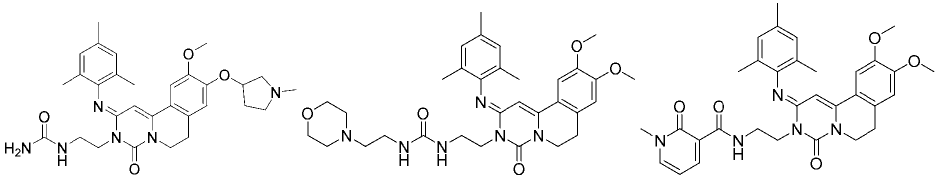 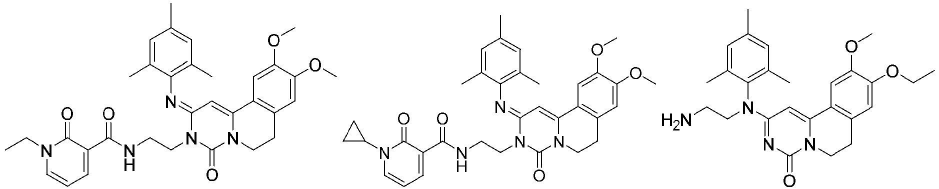 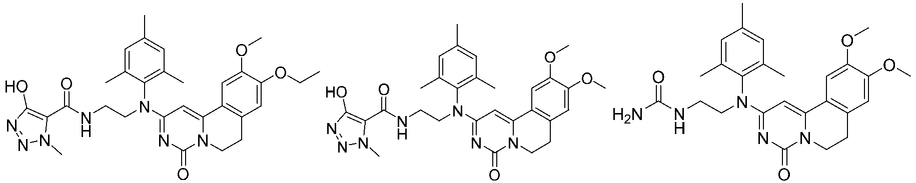 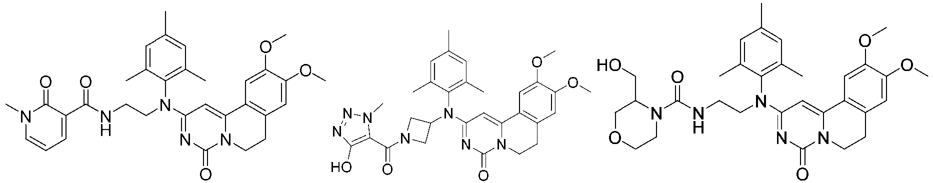 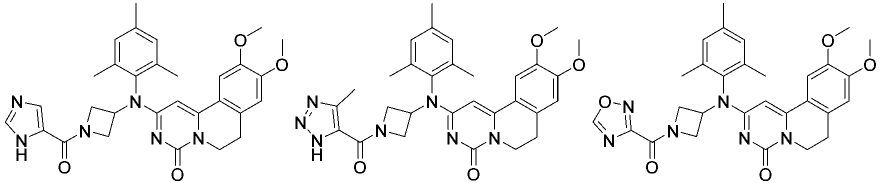 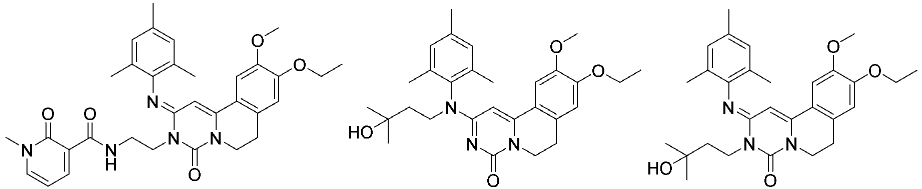 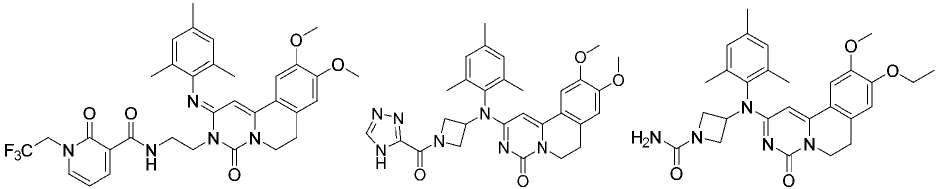 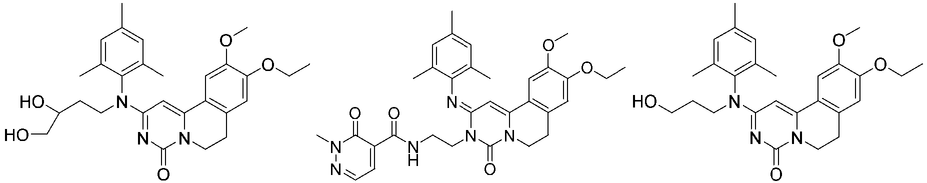 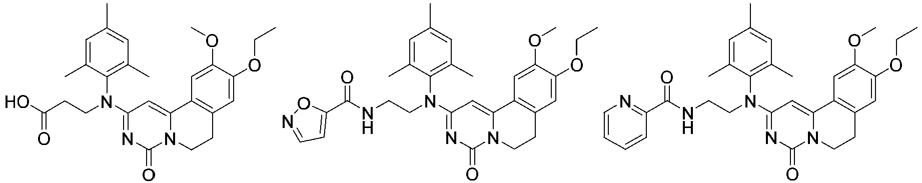 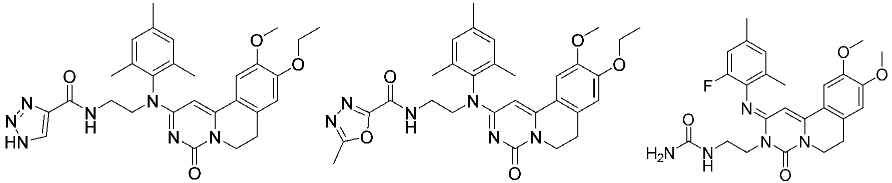 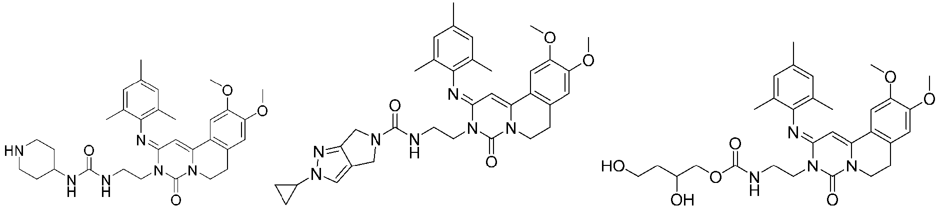 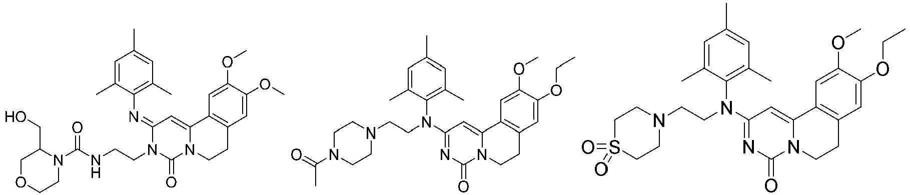 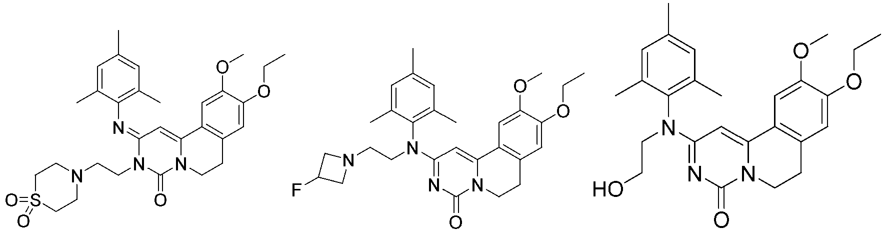 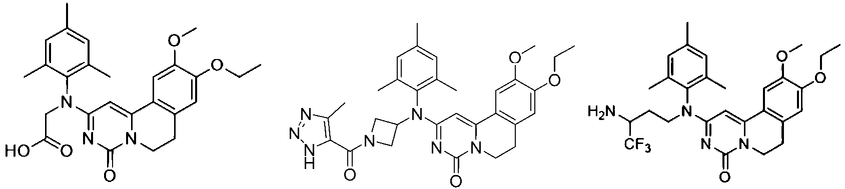 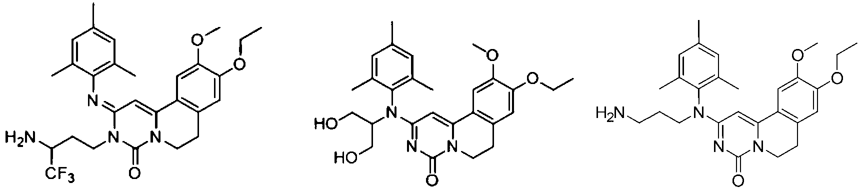 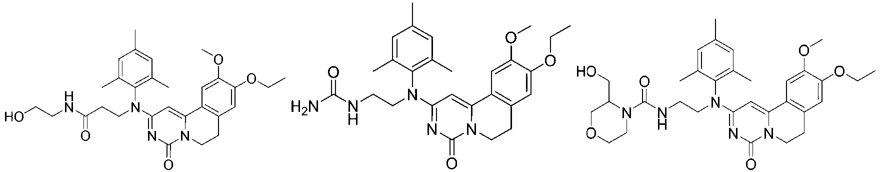 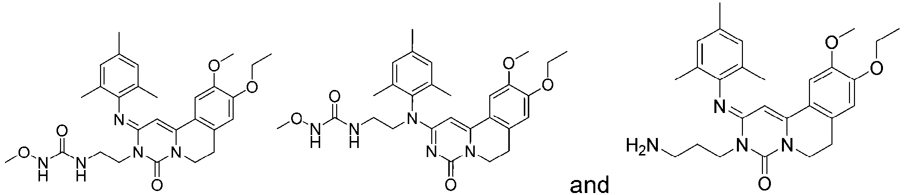 or a deuterated compound, stereoisomer, or pharmaceutically acceptable salt thereof.

8. A pharmaceutical composition comprising the compound according to any one of claims 1 to 7 and a pharmaceutically acceptable excipient.

9. Use of the compound according to any one of claims 1 to 7 or the pharmaceutical composition according to claim 8 in the manufacture of a medicament for treating a disease related to phosphodiesterase (PDE)3 and/or PDE4.

10. The use according to claim 9, wherein the disease related to PDE3 and/or PDE4 is one or more selected from the group consisting of inflammation, bronchiectasis, chronic obstructive pulmonary disease, and asthma.
